(19) Europäisches Patentamt
European Patent Office
Office européen des brevets

(11) EP 1 756 271 B1

(12) EUROPEAN PATENT SPECIFICATION

(45) Date of publication and mention of the grant of the patent:
**09.12.2015 Bulletin 2015/50**

(21) Application number: **05718476.4**

(22) Date of filing: **19.04.2005**

(51) Int Cl.:
***C12P 7/18*** *(2006.01)* ***C12P 17/02*** *(2006.01)*
***C12P 41/00*** *(2006.01)* *C12N 9/14 (2006.01)*
*C12N 1/19 (2006.01)*

(86) International application number:
**PCT/IB2005/001034**

(87) International publication number:
**WO 2005/100587 (27.10.2005 Gazette 2005/43)**

# (54) METHODS FOR OBTAINING OPTICALLY ACTIVE EPOXIDES AND VICINAL DIOLS FROM 2,2-DISUBSTITUTED EPOXIDES

VERFAHREN ZUR GEWINNUNG OPTISCH AKTIVER EPOXIDE UND VICINALER DIOLE AUS 2,2-DISUBSTITUIERTEN EPOXIDEN

METHODE PERMETTANT D'OBTENIR DES EPOXYDES ET DES DIOLS VICINAUX OPTIQUEMENT ACTIFS A PARTIR D'EPOXYDES 2,2-DISUBSTITUES

(84) Designated Contracting States:
**AT BE BG CH CY CZ DE DK EE ES FI FR GB GR HU IE IS IT LI LT LU MC NL PL PT RO SE SI SK TR**

(30) Priority: **19.04.2004 ZA 200402957**

(43) Date of publication of application:
**28.02.2007 Bulletin 2007/09**

(73) Proprietor: **CSIR**
**0184 Pretoria (ZA)**

(72) Inventors:
- **BOTES, Adriana, Leonora**
  **0002 Pretoria (ZA)**
- **LOTTER, Jeanette,**
  **18 The Cottages**
  **1644 Modderfontein (ZA)**

(74) Representative: **D Young & Co LLP**
**120 Holborn**
**London EC1N 2DY (GB)**

(56) References cited:
**EP-A- 1 291 436**

- **BOTES A L ET AL: "Enantioselectivities of yeast epoxide hydrolases for 1,2-epoxides" TETRAHEDRON ASYMMETRY 1999 UNITED KINGDOM, vol. 10, no. 17, 1999, pages 3327-3336, XP002348487 ISSN: 0957-4166**
- **VISSER H ET AL: "Construction and characterisation of a genetically engineered Escherichia coli strain for the epoxide hydrolase-catalysed kinetic resolution of epoxides" BIOCATALYSIS AND BIOTRANSFORMATION, vol. 21, no. 1, 2003, pages 33-40, XP008053606 ISSN: 1024-2422**
- **WEIJERS CAREL A G M ET AL: "Epoxide hydrolases from yeast and other sources: versatile tools in biocatalysis" JOURNAL OF MOLECULAR CATALYSIS. B, ENZYMATIC, ELSEVIER, AMSTERDAM,, NL, vol. 6, no. 3, 11 March 1998 (1998-03-11), pages 199-214, XP000946716 ISSN: 1381-1177**
- **KRENN W ET AL: "BACTERIAL EPOXIDE HYDROLASES OF OPPOSITE ENANTIOPREFERENCE" BIOTECHNOLOGY LETTERS, KEW, SURREY, GB, vol. 21, no. 8, 1999, pages 687-690, XP001056430 ISSN: 0141-5492**
- **STEINREIBER A ET AL: "Enantioselective hydrolysis of functionalized 2,2-disubstituted oxiranes with bacterial epoxide hydrolases" EUROPEAN JOURNAL OF ORGANIC CHEMISTRY, vol. 2000, 2000, pages 3703-3711, XP002348488 cited in the application**
- **VISSER H ET AL: "Cloning and characterization of an epoxide hydrolase-encoding gene from Rhodotorula glutinis" APPLIED MICROBIOLOGY AND BIOTECHNOLOGY, vol. 53, no. 4, April 2000 (2000-04), pages 415-419, XP002348541 ISSN: 0175-7598**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

(Cont. next page)

- LEE E Y ET AL: "Production of (S)-styrene oxide by recombinant Pichia pastoris containing epoxide hydrolase from Rhodotorula glutinis", ENZYME AND MICROBIAL TECHNOLOGY, STONEHAM, MA, US, vol. 35, no. 6-7, 1 December 2004 (2004-12-01), pages 624-631, XP004619962, ISSN: 0141-0229, DOI: 10.1016/J.ENZMICTEC.2004.08.016
- MADZAK C ET AL: "Heterologous Protein Expression and Secretion in the Non-conventional Yeast Yarrowia lipolytica: A Review", JOURNAL OF BIOTECHNOLOGY, ELSEVIER SCIENCE PUBLISHERS, AMSTERDAM, NL, vol. 109, no. 1-2, 1 April 2004 (2004-04-01), pages 63-81, XP002995176, ISSN: 0168-1656, DOI: 10.1016/J.JBIOTEC.2003.10.027

## Description

### TECHNICAL FIELD

THIS INVENTION relates to biocatalytic reactions, and more particularly to the use of enantiomer selective hydrolases to obtain optically active epoxides and vicinal diols.

### BACKGROUND

Optically active epoxides and vicinal diols are versatile fine chemical intermediates for use in the production of pharmaceuticals, agrochemicals, ferro-electric liquid crystals and flavours and fragrances. Epoxides are highly reactive electrophiles because of the strain inherent in the three-membered ring and the electronegativity of the oxygen. Epoxides react readily with various O-, N-, S-, and C-nucleophiles, acids, bases, reducing and oxidizing agents, allowing the production to bifunctional molecules. Vicinal diols, employed as their highly reactive cyclic sulfites and sulfates, act like epoxide-like synthons with a broad range of nucleophiles. The possibility of double nucleophilic displacement reactions with amidines and azide, allow access to dihydroimidazole derivatives, aziridines, diamines and diazides. Since enantiopure epoxides and vicinal diols can stereospecifically be interconverted, they can be regarded as synthetic equivalents.

Optically active 2,2-disubstituted epoxides (TDE) and 2-substituted-1,2-diols (DVD) have considerable synthetic potential, since these compounds constitute highly flexible molecular scaffolds which can be converted to valuable optically active tertiary alcohols, $\alpha$-methylamino acids and $\alpha$-hydroxy-$\alpha$-methyl carboxylic acids (Steinreiber *et al.* 2000).

Epoxide hydrolases (EC 3.3.2.3) are hydrolytic enzymes that convert epoxides to vicinal diols by ring-opening of the epoxide with water. Epoxide hydrolases are present in mammals, plants, insects and microorganisms.

Botes et al., Tetrahedron Asymmetry 10 (1999) 3327-3336, describes the enantioselective hydrolysis of epoxides using yeast strains belonging to the genera *Rhodotorula, Rhodosporidium and Trichosporon.*

Visser et al., Biocatalysis and Biotransformation, 2003, vol. 21(1), pp 33-40, describes a *Rhodotorula glutinis* epoxide hydrolase produced in the heterologous host *Escherichia coli*.

Weijers et al., Journal of Molecular Catalysis B: Enzymatic 6 (1999) 199-124, reviews epoxide hydrolase activity in yeasts.

### SUMMARY

The invention is based in part on the surprising discovery by the inventors that certain microorganisms express epoxide hydrolases with high enantioselectivity. The microorganisms of the invention selectively hydrolyse 2,2-disubstituted epoxides or selectively form 2-substituted-1,2-diols and their genomes encode polypeptides having highly enantioselective epoxide hydrolase activity.

More specifically, the invention provides a process for obtaining an optically active epoxide or an optically active vicinal diol, which process includes the steps of: providing an enantiomeric mixture of a 2,2-disubstituted epoxide (TDE); creating a reaction mixture by adding to the enantiomeric mixture a Yarrowia lipolytica cell comprising an exogenous nucleic acid encoding, and expressing, a heterologous polypeptide, or a functional fragment thereof, having enantioselective 2,2-disubstituted epoxide hydrolase activity, the polypeptide being a polypeptide encoded by a gene of a yeast cell; incubating the reaction mixture; and recovering from the reaction mixture: (a) an enantiopure, or a substantially enantiopure, 2,2-disubstituted vicinal diol (DVD); (b) an enantiopure, or a substantially enantiopure, 2,2-disubstituted epoxide; or (c) an enantiopure, or a substantially enantiopure, 2,2-disubstituted vicinal diol and an enantiopure, or a substantially enantiopure, 2,2-disubstituted epoxide.

The polypeptide can be encoded by an endogenous gene of the cell or the cell can be a recombinant cell, the polypeptide being encoded by a nucleic acid sequence with which the cell is transformed. The nucleic acid sequence can be an exogenous nucleic acid sequence, a heterologous nucleic acid sequence, or a homologous nucleic acid sequence. The polypeptide can be a full-length yeast epoxide hydrolase or a functional fragment of a full length yeast epoxide hydrolase.

The processes can be carried out at a pH from 5 to 10 and/or at a temperature of 0°C to 70°C. The concentration of the 2,2-disubstituted epoxide can be at least equal to the solubility of the 2,2-disubstituted epoxide in water.

The 2,2-disubstituted epoxide of the enantiomeric mixture can be a compound of the general formula (I) and the vicinal diol produced by the process is a compound of the general formula (II),

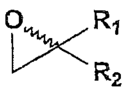

(I)

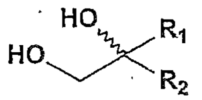
(II)

wherein:

$R_1$ and $R_2$ are, independently of each other, selected from the group consisting of a variably substituted straight-chain or branched alkyl group, a variably substituted straight-chain or branched alkenyl group, a variably substituted straight-chain or branched alkynyl group, a variably substituted cycloalkyl group as well as cycloalkenyl groups, a variably substituted aryl group, a variably substituted aryl alkyl group, a variably substituted heterocyclic group, a variably substituted straight-chain or branched alkoxy group, a variably substituted straight-chain or branched alkenyloxy group, a variably substituted aryloxy group, a variably substituted aryl alkyloxy group, a variably substituted alkylthio group, a variably substituted alkoxycarbonyl group, a variable substituted straight chain or branched alkylamino or alkenyl amino group, a variably substituted arylamino or arylalkylamino group, a variably substituted carbamoyl group, a variably substituted acyl group, and a functional group; or
wherein $R_1$ and $R_2$, together as a whole unit are a carbocycle with 5-7 atoms or a heterocycle with 5 to 7 carbon atoms.

Moreover, in the process the enantiomeric mixture can be a racemic mixture or a mixture of any ratio of amounts of the enantiomers. The process can include adding to the reaction mixture water and at least one water-immiscible solvent, including, for example, toluene, 1,1,2-trichlorotrifluoroethane, methyl *tert*-butyl ether, methyl isobutyl ketone, dibutyl-o-phtalate, aliphatic alcohols containing 6 to 9 carbon atoms or aliphatic hydrocarbons containing 6 to 16 carbon atoms. Alternatively, or in addition, the process can include adding to the reaction mixture water and at least one water-miscible organic solvent, for example, acetone, methanol, ethanol, propanol, isopropanol, acetonitrile, dimethylsulfoxide, *N*,*N*-dimethylformamide, or *N*-methylpyrrolidine. In addition, or alternatively, one or more surfactants, one or more cyclodextrins, or one or more phase-transfer catalysts can be added to the reaction mixtures. The process can include stopping the reaction when one enantiomer of the epoxide and/or vicinal diol is in excess compared to the other enantiomer of the epoxide and/or vicinal diol. Furthermore, the process can include recovering continuously during the reaction the optically active epoxide and/or the optically active vicinal diol produced by the reaction directly from the reaction mixture.

A method for producing a polypeptide, which process includes the steps of: providing a cell comprising a nucleic acid encoding and capable of expressing a polypeptide that has enantioselective 2,2-disubstituted epoxide hydrolase activity; culturing the cell; and recovering the polypeptide from the culture is disclosed herein. Recovering the polypeptide from the culture includes, for example, recovering it from the medium in which the cell was cultured or recovering it from the cell *per se*. The cell can be a yeast cell. The polypeptide can be encoded by an endogenous gene of the cell or the cell can be a recombinant cell, the polypeptide being encoded by a nucleic acid sequence with which the cell is transformed. The nucleic acid sequence can be an exogenous nucleic acid sequence, a heterologous nucleic acid sequence, or a homologous nucleic acid sequence. The polypeptide can be a full-length yeast epoxide hydrolase or a functional fragment of a full-length yeast epoxide hydrolase. The cell can be of any of the yeast genera, species, or strains disclosed herein or any recombinant cell disclosed herein.

There is also taught a crude or pure enzyme preparation which includes an isolated polypeptide having enantioselective 2,2-disubstituted epoxide hydrolase activity. The polypeptide can be one encoded by any of the yeast genera, species, or strains disclosed herein or one encoded by a recombinant cell.

In another aspect, the invention features a substantially pure culture of cells, a substantial number of which comprise a nucleic acid encoding, and are capable of expressing, a polypeptide having enantioselective 2,2-disubstituted epoxide hydrolase activity. The cells can be recombinant cells or cells of any of the yeast genera, species, or strains disclosed herein.

Another embodiment of the invention is an isolated cell, the cell comprising a nucleic acid encoding a polypeptide having enantioselective 2,2-disubstituted epoxide hydrolase activity, the cell being capable of expressing the polypeptide. The cell can be any of those disclosed herein.

Also disclosed is an isolated DNA that includes: (a) a nucleic acid sequence that encodes a polypeptide that has enantioselective 2,2 disubstituted epoxide hydrolase activity and that hybridizes under highly stringent conditions to the complement of a sequence that can be SEQ ID NO: 8, 9, 10, 11, 12, 13, or 14; or (b) the complement of the nucleic acid sequence. The nucleic acid sequence can encode a polypeptide that includes an amino acid sequence that can be SEQ ID NO: 1, 2, 3, 4, 5, 6, or 7. The nucleic acid sequence can be, for example, one of those with SEQ ID NOs: 8, 9, 10, 11, 12, 13, or 14.

Also taught herein is an isolated DNA that includes: (a) a nucleic acid sequence that is at least 55% identical to a sequence that can be SEQ ID NO: 8, 9, 10, 11, 12, 13, or 14; or (b) the complement of the nucleic acid sequence, the nucleic acid sequence encoding a polypeptide that has enantioselective 2,2-disubstituted epoxide hydrolase activity.

Taught herein is an isolated DNA that includes: (a) a nucleic acid sequence that encodes a polypeptide consisting of an amino acid sequence that is at least 55% identical to a sequence that can be SEQ ID NOs: 1, 2, 3, 4, 5, 6, or 7; or (b) the complement of the nucleic acid sequence, the polypeptide having enantioselective 2,2-disubstituted epoxide hydrolase activity.

Also included are vectors (e.g., those in which the coding sequence is operably linked to a transcriptional regulatory element) containing any of the above DNAs and cells (e.g., eukaryotic or prokaryotic cells) containing such vectors.

Also disclosed herein is an isolated polypeptide encoded by any of the above DNAs. The polypeptide can include an amino acid sequence that is at least 55% identical to SEQ ID NOs: 1, 2, 3, 4, 5, 6 or 7, the polypeptide having enantioselective 2,2-disubstituted epoxide hydrolase activity. The polypeptide can also include: (a) a sequence that can be SEQ ID NO: 1, 2, 3, 4, 5, 6, or 7, or a functional fragment of the sequence; or (b) the sequence of (a), but with no more than five conservative substitutions, the polypeptide having enantioselective 2,2-disubstituted epoxide hydrolase activity.

Further disclosed is an isolated antibody (e.g., a polyclonal or a monoclonal antibody) that binds to any of the above-described polypeptides.

The term "exogenous" as used herein with reference to nucleic acid and a particular host cell refers to any nucleic acid that does not occur in (and cannot be obtained from) that particular cell as found in nature. Thus, a non-naturally-occurring nucleic acid is considered to be exogenous to a host cell once introduced into the host cell. It is important to note that non-naturally-occurring nucleic acids can contain nucleic acid subsequences or fragments of nucleic acid sequences that are found in nature provided the nucleic acid as a whole does not exist in nature. For example, a nucleic acid molecule containing a genomic DNA sequence within an expression vector is non-naturally-occurring nucleic acid, and thus is exogenous to a host cell once introduced into the host cell, since that nucleic acid molecule as a whole (genomic DNA plus vector DNA) does not exist in nature. Thus, any vector, autonomously replicating plasmid, or virus (e.g., retrovirus, adenovirus, or herpes virus) that as a whole does not exist in nature is considered to be non-naturally-occurring nucleic acid. It follows that genomic DNA fragments produced by PCR or restriction endonuclease treatment as well as cDNAs are considered to be non-naturally-occurring nucleic acid since they exist as separate molecules not found in nature. It also follows that any nucleic acid containing a promoter sequence and polypeptide-encoding sequence (e.g., cDNA or genomic DNA) in an arrangement not found in nature is non-naturally-occurring nucleic acid. Nucleic acid that is naturally-occurring can be exogenous to a particular cell. For example, an entire chromosome isolated from a cell of yeast x is an exogenous nucleic acid with respect to a cell of yeast y once that chromosome is introduced into a cell of yeast y.

It will be clear from the above that "exogenous" nucleic acids can be "homologous" or "heterologous" nucleic acids. As used herein, "homologous" nucleic acids are those that are derived from a cell of the same species as the host cell and "heterologous" nucleic acids are those that are derived from a species other than that of the host cell.

In contrast, the term "endogenous" as used herein with reference to nucleic acids or genes and a particular cell refers to any nucleic acid or gene that does occur in (and can be obtained from) that particular cell as found in nature.

**The alkyl group** can be a straight chain or branched alkyl group with 1 to 12 carbon atoms. The alkyl group can be selected from the group consisting of: methyl-; ethyl-; propyl-; isopropyl-; butyl-; isobutyl-; s-butyl-; t-butyl-; pent-1-yl-; pent-2-yl-; pent-3-yl-; 2-methylbut-1-yl-; 3-methylbut-1-yl-; 2-methylbut-2-yl-; 3-methylbut-2-yl-; hex-1-yl-; hex-2-yl-; hex-3-yl-; 1-methylpent-1-yl-; 2-methylpent-1-yl-; 3-methylpent-1-yl-; 2-methylpent-2-yl-; 3-methylpent-2-yl-; 4-methylpent-2-yl-; 2-methylpent-3-yl-; 3-methylpent-3-yl-; 2-ethylbut-1-yl-; hept-1-yl-; hept-2-yl-; hept-3-yl-; hept-4-yl-; 1-methylhex-1-yl-; 2-methylhex-1-yl-; 3-methylhex-1-yl-; 4-methylhex-1-yl-; 5-methylhex-1-yl-; 2-methylhex-2-yl-; 3-methylhex-2-yl-; 4-methylhex-2-yl-; 5-methylhex-2-yl-; 2-methylhex-3-yl-; 3-methylhex-3-yl-; 4-methylhex-3-yl-; 5-methylhex-3-yl-; 2-methylhex-4-yl-; 1,1-dimethylpent-1-yl-; 1,2-dimethylpent-1-yl-; 1,3-dimethylpent-1-yl-; 1,4-dimethylpent-1-yl-; 2,2-dimethylpent-1-yl-; 2,3-dimethylpent-1-yl-; 2,4-dimethylpent-1-yl-; 2,5-dimethylpent-1-yl-; 3,3-dimethylpent-1-yl-; 3,4-dimethylpent-1-yl-; 3,5-dimethylpent-1-yl-; 4,4-dimethylpent-1-yl-; 4,5-dimethylpent-1-yl-; 5,5-dimethylpent-1-yl-; 2,2-dimethylpent-2-yl-; 2,3-dimethylpent-2-yl-; 2,4-dimethylpent-2-yl-; 3,3-dimethylpent-2-yl-; 3,4-dimethylpent-2-yl-; 2,2-dimethylpent-3-yl-; 2,3-dimethylpent-3-yl-; 2,4-dimethylpent-3-yl-; 2,2-dimethylpent-4-yl-; 2-ethylpent-1-yl-; 3-ethylpent-1-yl-; 1,1,2-trimethylbut-1-yl-; 1,2,2-trimethylbut-1-yl-; 1,2,3-trimethylbut-1-yl-; 2,2,3-trimethylbut-1-yl-; 2,3,3-trimethylbut-1-yl-; 2,3,3-but-2-yl-; 2-isopropylbut-1-yl-; 2-isopropylbut-2-yl-; oct-1-yl-; oct-2-yl-; oct-3-yl-; oct-4-yl-; 2-methylhept-1-yl-; 3-methylhept-1-yl-; 4-methylhept-1-yl-; 5-methylhept-1-yl-; 6-methylhept-1-yl-; 2-methylhept-2-yl-; 3-methylhept-2-yl-; 4-methylhept-2-yl-; 5-methylhept-2-yl-; 6-methylhept-2-yl-; 2-methylhept-3-yl-; 3-methylhept-3-yl-; 4-methylhept-3-yl-; 5-methylhept-3-yl-; 6-methylhept-3-yl-; 2-methylhept-4-yl-; 3-methylhept-4-yl-; 4-methylhept-4-yl-; 2,2-dimethylhex-1-yl-; 2,3-dimethylhex-1-yl-; 2,4-dimethylhex-1-yl-; 2,5-dimethylhex-1-yl-; 3,3-dimethylhex-1-yl-; 3,4-dimethylhex-1-yl-; 3,5-dimethylhex-1-yl-; 4,4-dimethylhex-1-yl-; 4,5-dimethylhex-1-yl-; 5,5-dimethylhex-1-yl-; 2,3-dimethylhex-2-yl-; 2,4-dimethylhex-2-yl-; 2,5-dimethylhex-2-yl-; 3,3-dimethylhex-2-yl-; 3,4-dimethylhex-2-yl-; 3,5-dimethylhex-2-yl-; 4,4-dimethylhex-2-yl-; 4,5-dimethylhex-2-yl-; 5,5-dimethylhex-2-yl-; 2,2-dimethylhex-3-yl-; 2,3-dimethylhex-3-yl-; 2,4-dimethylhex-3-yl-; 2,5-dimethylhex-3-yl-; 3,3-dimethylhex-3-yl-; 3,4-dimethylhex-3-yl-; 3,5-dimethylhex-3-yl-; 4,4-dimethylhex-3-yl-; 4,5-dimethylhex-3-yl-; 5,5-dimethylhex-3-yl-; 2,2,3-trimethylpent-1-yl-; 2,2,4-trimethylpent-1-yl-; 2,3,3-trimethylpent-1-yl-; 2,3,4-trimethylpent-1-yl-; 3,3,4-trimethylpent-1-yl-; 3,4,4-trimethylpent-1-yl-; 2,4,4-trimethylpent-1-yl-; 2,3,3-trimethylpent-2-yl-; 2,3,4-trimethylpent-2-yl-; 3,3,4-trimethylpent-2-yl-; 3,4,4-trimethylpent-2-yl-; 2,4,4-trimethylpent-2-yl-; 2,2,3-trimethylpent-3-yl-; 2-methyl-3-ethylpen-1-yl-; 3-ethyl-3-methylpent-1-yl-; 3-ethyl-4-methylpent-1-yl-; (3-methylhex-3-yl)methyl-; (4-methylhex-3-yl)methyl-; (5-methylhex-3-yl)methyl-; (2-methylhex-2-yl)methyl-; 2-methyl-3-ethylpent-2-yl-; 3-ethyl-3-methylpent-2-yl-; 3-ethyl-4-methylpent-2-yl-; 2-methyl-2-ethylpent-3-yl-; 2-methyl-3-ethylpent-3-yl-; 2,2,3,3-tetramethylbut-1 -yl-; 2-ethyl-3,3-dimethylbut-2-yl-; 2-isopropyl-3-methylbut-2-yl-; (3-ethylpent-3-yl)methyl-; (2,3-dimethylpent-3-yl)methyl-; (2,4-dimethylpent-3-yl)methyl-; non-1-yl-; non-2-yl-; non-3-yl-; non-4-yl-; non-5-yl-; 2-methyloct-1-yl; 3-methylocfi-1-yl-; 4-methyloct-1-yl-; 5-methyloct-1-yl-; 6-methyloct-1-yl-; 7-methyloct-1-yl-; 2-methyloct-2-yl; 3-methyloct-2-yl-; 4-methyloct-2-yl-; 5-methyloct-2-yl-; 6-methyloct-2-yl-; 7-methyloct-2-yl-; 2-methyloct-3-yl; 3-methyloct-3-yl-; 4-methyloct-3-yl-; 5-methyloct-3-yl-; 6-methyloct-3-yl-; 7-methyloct-3-yl-; 2-methyloct-4-yl; 3-methyloct-4-yl-; 4-methyloct-4-yl-; 5-methyloct-4-yl-; 6-methyloct-4-yl-; 7-methyloct-4-yl-; 2,2-dimethylhept-1-yl-; 2,3-dimethylhept-1-yl-; 2,4-dimethylhept-1-yl-; 2,5-dimethylhept-1-yl-; 2,6-dimethylhept-1-yl-; 3,3-dimethylhept-1-yi-; 3,4-dimethylhept-1-yl-; 3,5-dimethylhept-1-yl-; 3,6-dimethylhept-1-yl-; 4,4-dimethylhept-1-yl-; 4,5-dimethylhept-1-yl-; 4,6-dimethylhept-1-yl-; 5,5-dimethylhept-1-yl-; 5,6-dimethylhept-1-yl-; 6,6-dimethylhept-1-yl-; 2,3-dimethylhept-2-yl-; 2,4-dimethylhept-2-yl-; 2,5-dimethylhept-2-yl-; 2,6-dimethylhept-2-yl-; 3,3-dimethylhept-2-yl-; 3,4-dimethylhept-2-yl-; 3,5-dimethylhept-2-yl-; 3,6-dimethylhept-2-yl-; 4 ,4-dimethylhept-2 -yl-; 4,5-dimethylhept-2-yl-; 4,6-dimethylhept-2-yl-; 5,5-dimethylhept-2-yl-; 5,6-dimethylhept-2-yl-; 6,6-dimethylhept-2-yl-; 2,2-dimethylhept-3-yl-; 2,3-dimethylhept-3-yl-; 2,4-dimethylhept-3-yl-; 2,5-dimethylhept-3-yl-; 2,6-dimethylhept-3-yl-; 3,4-dimethylhept-3-yl-; 3,5-dimethylhept-3-yl-; 3,6-dimethylhept-3-yl-; 4,4-dimethylhept-3-yl-; 4,5-dimethylhept-3-yl-; 4,6-dimethylhept-3-yl-; 5,5-dimethylhept-3-yl-; 5,6-dimethylhept-3-yl-; 6,6-dimethylhept-3-yl-; 3-ethylhept-1-yl-; 3-ethylhept-1-yl-; 4-ethylhept-1-yl-; 3-ethylhept-2-yl-; 4-ethylhept-2-yl-; 5-ethylhept-2-yl-; 3-ethylhept-3-yl-; 4-ethylhept-3-yl-; 5-ethylhept-3-yl-; 3-ethylhept-4-yl-; 4-ethylhept-4-yl-; 2,2,3-trimethylhex-1-yl-; 2,2,4-trimethylhex-1-yl-; 2,2,5-trimethylhex-1-yl-; 2,3,3-trimethylhex-1-yl-; 2,3,4-trimethylhex-1-yl-; 2,3,5-trimethylhex-1-yl-; 2,4,4-trimethylhex-1-yl-; 2,4,5-trimethylhex-1-yl-; 2,5,5-trimethylhex-1-yl-; 3,3,4-trimethylhex-1-yl-; 3,3,5-trimethylhex-1-yl-; 4,4,5-trimethylhex-1-yl-; 4,5,5-trimethylhex-1-yl-; 2,3,3-trimethylhex-2-yl-; 2,3,4-trimethylhex-2-yl-; 2,3,5-trimethylhex-2-yl-; 2,4,4-trimethylhex-2-yl-; 2,4,5-trimethylhex-2-yl-; 2,5,5-trimethylhex-2-yl-; 3,3,4-trimethylhex-2-yl-; 3,3,5-trimethylhex-2-yl-; 3,4,4-trimethylhex-2-yl-; 3,4,5-trimethylhex-2-yl-; 3,5,5-trimethylhex-2-yl-; 4,4,5-trimethylhex-2-yl-; 4,5,5-trimethylhex-2-yl-; 2,2,3-trimethylhex-3-yl-; 2,2,4-trimethylhex-3-yl-; 2,2,5-trimethylhex-3-yl-; 2,3,4-trimethylhex-3-yl-; 2,3,5-trimethylhex-3-yl-; 2,4,4-trimethylhex-3-yl-; 2,4,5-trimethylhex-3-yl-; 2,5,5-trimethylhex-3-yl-; 4,4,5-trimethylhex-3-yl-; 4,5,5-trimethylhex-3-yl-; (2-methylhex-3-yl)methyl-; 3-ethyl-2-methylhex-1-yl-; 3-ethyl-3-methylhex-1-yl-; 3-ethyl-4-methylhex-1-yl-; 3-ethyl-5-methylhex-1-yl-; 4-ethyl-2-methylhex-1-yl-; 4-ethyl-3-methylhex-1-yl-; 4-ethyl-4-methylhex-1-yl-; 4-ethyl-5-methylhex-1-yl-; (2-methylhex-1-yl)methyl-; (3-methylhex-1-yl)methyl-; (4-methylhex-1-yl)methyl-; (5-methylhex-1-yl)methyl-; (6-methylhex-1-yl)methyl-; 3-isopropylhex-1-yl-; 4-ethyl-5-methylhex-1-yl-; 3-ethyl-3-methylhex-2-yl-; 3-ethyl-4-methylhex-2-yl-; 3-ethyl-5-methylhex-2-yl-; 4-ethyl-2-methylhex-2-yl-; 4-ethyl-3-methylhex-2-yl-; 4-ethyl-4-methylhex-2-yl-; 4-ethyl-5-methylhex-2-yl-; 3-isopropylhex-2-yl-; 4-ethyl-5-methylhex-2-yl-; 3-ethyl-2-methylhex-3-yl-; 3-ethyl-4-methylhex-3-yl-; 3-ethyl-5-methylhex-3-yl-; 4-ethyl-2-methylhex-3-yl-; 4-ethyl-3-methylhex-3-yl-; 4-ethyl-4-methylhex-3-yl-; 4-ethyl-5-methylhex-3-yl-; 4-isopropylhex-1-yl-; 2,2,3,3-tetramethylpent-1-yl-; 2,2,3,4-tetramethylpent-1-yl-; 2,2,4,4-tetramethylpent-1-yl-; 2,3,3,4-tetramethylpent-1-yl-; 2,3,4,4-tetramethyl pent- 1 -yl-; 2,3,4,4-tetramethylpent-1-yl-; 3,3,4,4-tetramethylpent-1-yl-; 2,3,3,4-tetramethylpent-2-yl-; 2,3,4,4-tetramethylpent-2-yl-; 2,3,4,4-tetramethylpent-2-yl-; 3,3,4,4-tetramethylpent-2-yl-; 2,2,3,4-tetramethylpent-3-yl-; 2,2,4,4-tetramethylpent-3-yl-; 2,3,4,4-tetramethylpent-3-yl-; 2,3,4,4-tetramethylpent-3-yl-; (3-ethylhex-3-yl)methyl-; (4-ethylhex-3-yl)methyl-; (5-methylhept-3-yl)methyl-; 2,4-dimethyl-3-ethylpent-1-yl-; 3,4-dimethyl-3-ethylpent-1-yl-; 4,4-dimethyl-3-ethylpent-1-yl-; 2-ethyl-2-methylhex-1-yl-; 3-ethyl-2-methylhex-1-yl-; 4-ethyl-2-methylhex-1-yl-; 2-ethyl-3-methylhex-1-yl-; 2-ethyl-4-methylhex-1-yl-; 3-ethyl-3-methylhex-1-yl-; 3-ethyl-4-methylhex-1-yl-; 3-ethyl-5-methylhex-1-yl-; 4-ethyl-3-methylhex-1-yl-; 4-ethyl-4-methylhex-1-yl-; 4-ethyl-5-methylhex-1-yl-; and the like from dec-1-yl-; dec-2-yl-; dec-3-yl-; dec-4-yl-; dec-5-yl-; dec-6-yl-; undec-1-yl-; undec-2-yl-; undec-3-yl-; undec-4-yl-; undec-5-yl-; undec-6-yl-; undec-7-yl-; dodec-1-yl; dodec-2-yl; dodec-3-yl; dodec-4-yl; dodec-5-yl; dodec-6-yl groups. Preferably, the alkyl group is a straight chain or branched alkyl group with 1 to 8 carbon atoms.

**The alkenyl group** can be a straight chain or branched alkenyl group having 2-12 carbon atoms. The alkenyl group can be selected from the group consisting of: vinyl-; allyl-; α-methallyl-; β-methallyl-; 1-propenyl-; isopropenyl-; 1-butenyl-; 2-butenyl-; 3-butenyl; 1-buten-2-yl-; 1-buten-3-yl-; 1-methyl-1-propenyl-; 2-methyl-1-propenyl-; 1-pentenyl-; 2-pentenyl-; 3-pentenyl-; 4-pentenyl-; 1-penten-2-yl-; 1-penten-3-yl-; 2-methyl-1-butenyl-; 1-hexenyl-; 2-hexenyl-; 3-hexenyl-; 4-hexenyl-; 5-hexenyl-; 1-heptenyl-; 2-heptenyl-; 3-heptenyl-; 4-heptenyl-; 5-heptenyl-; 6-heptenyl-; 1-octenyl-; 2-octenyl-; 3-octenyl-; 4-octenyl-; 5-octenyl-; 6-octenyl-; 7-octenyl-; 1-nonenyl-; 2-nonenyl-; 3-nonenyl-; 4-nonenyl-; 5-nonenyl-; 6-nonenyl-; 7-nonenyl-; 8-nonenyl-; 1-decenyl-; 2-decenyl-; 3-decenyl-; 4-decenyl-; 5-decenyl-; 6-decenyl-; 7-decenyl-; 8-decenyl-; 9-decenyl-; 1-undecenyl; 2-undecenyl; 3-undecenyl; 4-undecenyl; 5-undecenyl; 6-undecenyl; 7-undecenyl; 8-undecenyl; 9-undecenyl; 10-undecenyl; 1-dodecenyl; 2-dodecenyl; 3-dodecenyl; 4-dodecenyl; 5-dodecenyl; 6-dodecenyl; 7-dodecenyl; 8-dodecenyl; 9-dodecenyl; 10-dodecenyl; 11-dodecenyl groups; and related branched

isomers. Preferably, the group is a straight chain or branched alkenyl group with 2 to 8 carbon atoms.

**The alkynyl group** can a straight chain or branched alkynyl group having 2-12 carbon atoms. The alkynyl group can be selected from the group consisting of: ethynyl-; 1-propynyl-; 2-propynyl-; 1-butynyl-; 2-butynyl-; 3-butynyl-; 1-pentynyl-; 2-pentynyl-; 3-pentynyl-; 4-pentynyl-; 1-hexynyl-; . 2-hexynyl-; 3-hexynyl-; 4-hexynyl-; 5-hexynyl-; 1-heptynyl-; 2-heptynyl-; 3-heptynyl-; 4-heptynyl-; 5-heptynyl-; 6-heptynyl-; 1-octynyl-; 2-octynyl-; 3-octynyl-; 4-octynyl-; 5-octynyl-; 6-octynyl-; 7-octynyl-; 1-nonynyl-; 2-nonynyl-; 3-nonynyl-; 4-nonynyl-; 5-nonynyl-; 6-nonynyl-; 7-nonynyl-; 8-nonynyl-; 1-decynyl-; 2-decynyl-; 3-decynyl-; 4-decynyl-; 5-decynyl-; 6-decynyl-; 7-decynyl-; 8-decynyl-; 9-decynyl-; 1-undecynyl-; 2-undecynyl-; 3-undecynyl-; 4-undecynyl-; 5-undecynyl-; 6-undecynyl-; 7-undecynyl-; 8-undecynyl-; 9-undecynyl-; 10-undecynyl-; 1-dodecynyl-; 2-dodecynyl-; 3-dodecynyl-; 4-dodecynyl-; 5-dodecynyl-; 6-dodecynyl-; 7-dodecynyl-; 8-dodecynyl-; 9-dodecynyl-; 10-dodecynyl-; 11-dodecynyl- groups; and related branched isomers. Preferably, the alkynyl group is a straight chain or branched alkenyl group with 2 to 8 carbon atoms.

**The cycloalkyl group** can be cycloalkyl groups with 3 to 10 carbon atoms. The cycloalkyl group can be selected from the group consisting of: cyclopropyl-; cyclobutyl-; cyclopentyl-; cyclohexyl-; cycloheptyl-; and cyclooctyl- groups. These groups can be variably substituted at any position(s) around the ring. Preferably, the cycloalkyl group is a cycloalkyl group with 5 to 7 carbon atoms.

**The cycloalkenyl group** can be cycloalkenyl groups with 3 to 10 carbon atoms. The cycloalkenyl group can be selected from the group consisting of cyclobutenyl-; cyclopentenyl-; cyclohexenyl-; cycloheptenyl-; and cyclooctenyl- groups that can variably be substituted at any position(s) around the ring. Preferably, the cycloalkenyl group is a cycloalkenyl group with 5 to 7 carbon atoms.

**The aryl group can** be selected from the group consisting of phenyl; biphenyl; naphtyl; anthracenyl groups; and the like. Preferably, the aryl group is a phenyl group.

**The aryl alkyl group** can be a group with 7 to 18 carbons. The aryl alkyl group can be selected from the group consisting of: benzyl-; 1-methylbenzyl-; 2-phenylethyl-; 3-phenylpropyl-; 4-phenylbutyl-; 5-phenylpentyl-; 6-phenylhexyl-; 1-naphtylmethyl; and 2-(1-naphtyl)-ethyl groups; and the like. Preferably, the aryl alkyl group is an aryl alkyl group with 7 to 12 carbon atoms.

**The heterocyclic group** can include 5- to 10-membered heterocyclic groups containing nitrogen, oxygen, or sulfur. The heterocyclic ring can be fused with a cyclic or aromatic ring having 3 to 7 carbon atoms such as benzene; cyclopropyl; cyclobutane; cyclopentane; and cyclohexane ring systems. Preferably, the heterocyclic ring has 5 or 6 carbon atoms. The heterocyclic ring can be selected from the group consisting of: furyl-; dihydrofuranyl-; tetrahydrofuranyl-; dioxolanyl-; oxazolyl-; dihydrooxazolyl-; oxazolidinyl-; isoxazolyl-; dihydroisoxazolyl-; isoxazolidinyl-; oxathiolanyl-; thienyl-; tetrahydrothienyl-; dithiolanyl-; thiazolyl-; dihydrothiazolyl-; thiazolidinyl-; isothiazolyl-; dihydroisothiazolyl-; isothiazolidinyl-; pyrrolyl-; dihydropyrrolyl-; pyrrolidinyl-; pyrazolyl-; dihydropyrazolyl-; pyrazolidinyl-; imidazolyl-; dihydroimidazolyl-; imidazolidinyl-; triazolyl-; dihydrotriazolyl-; triazolidinyl-; tetrazolyl-; dihydrotetrazolyl-; tetrazolidinyl-; pyridyl-; dihydropyridyl-; piperidinyl-; morpholinyl-; dioxanyl-; oxathianyl-; trioxanyl-; thiomorpholinyl-; pyridazinyl-; dihydropyridazinyl-; tetrahydropyridazinyl-; hexahydropyridazinyl-; pyrimidinyl-; dihydropyrimadinyl-; tetrahydropyrimadinyl-; hexahydropyrimadinyl-; pyrazinyl-; piperazinyl-; pyranyl-; dihydropyranyl-; tetrahydropyranyl-; thiopyranyl-; dihydrothiopyranyl-; tetrahydrothiopyranyl-; dithianyl-; purinyl-; pyrimidinyl-; pyrrolizinyl-; pyrrolizidinyl; indolyl-; dihydroindolyl-; isoindolyl-; indolizinyl-; indolizidinyl-; quinolyl-; dihydroquinolyl-; tetrahydroquinolyl-; isoquinolyl-; dihydroquinolyl-; tetrahydroquinolyl-; quinolizinyl-; quinolizidinyl-; phenanthrolinyl-; chromenyl-; chromanyl-; isochromenyl-; isochromanyl-; benzofuranyl-; and carbazolyl- groups; and the like.

**The alkoxy group** can be a straight chain or branched alkoxy group having 2-12 carbon atoms such as methoxy; ethoxy; propyloxy; isopropyloxy; butyloxy; isobutyloxy; tert-butyloxy; pentyloxy; hexyloxy; heptyloxy; or octyloxy.

**The alkenyloxy group** can be a straight chain or branched alkenyloxy group having 2-12 carbon atoms. The alkenyloxy group can be selected from the group consisting of: ethynyloxy-; 1-propynyloxy-; 2-propynyloxy-; 1-butynyloxy-; 2-butynyloxy-; 3-butynyloxy-; 1-pentynyloxy-; 2-pentynyloxy-; 3-pentynyloxy-; 4-pentynyloxy-; 1-hexynyloxy-; 2-hexynyloxy-; 3-hexynyloxy-; 4-hexynyloxy-; 5-hexynyloxy-; 1-heptynyloxy-; 2-heptynyloxy-; 3-heptynyloxy-; 4-heptynyloxy-; 5-heptynyloxy-; 6-heptynyloxy-; 1-octynyloxy-; 2-octynyloxy-; 3-octynyloxy-; 4-octynyloxy-; 5-octynyloxy-; 6-octynyloxy-; 7-octynyloxy-; 1-nonynyloxy-; 2-nonynyloxy-; 3-nonynyl-oxy-; 4-nonynyloxy-; 5-nonynyloxy-; 6-nonynyloxy-; 7-nonynyloxy-; 8-nonynyloxy-; 1-decynyloxy-; 2-decynyloxy-; 3-decynyloxy-; 4-decynyloxy-; 5-decynyloxy-; 6-decynyloxy-; 7-decynyloxy-; 8-decynyloxy-; 9-decynyloxy-; 1-undecynyloxy-; 2-undecynyloxy-; 3-undecynyloxy-; 4-undecynyloxy-; 5-undecynyloxy-; 6-undecynyloxy-; 7-undecynyloxy-; 8-undecynyloxy-; 9-undecynyloxy-; 10-undecynyloxy-; 1-dodecynyloxy-; 2-dodecynyloxy-; 3-dodecynyloxy-; 4-dodecynyloxy-; 5-dodecynyloxy-; 6-dodecynyloxy-; 7-dodecynyloxy-; 8-dodecynyloxy-; 9-dodecynyloxy-; 10-dodecynyloxy-; and 11-dodecynyloxy- groups; and related branched isomers. Preferably, the alkenyloxy group is a straight chain or branched alkenyloxy groups with 2 to 8 carbon atoms.

**The aryloxy group** can be an aryloxy group, such as a phenoxy or naphtyloxy group (e.g.: phenoxy; 2-methylphenoxy; 3-methylphenoxy; 4-methylphenoxy; 2-allylphenoxy; 2-chlorophenoxy; 3-chlorophenoxy; 4-chlorophenoxy; 4-methoxyphenoxy; 2-allyloxyphenoxy; naphtyloxy; and the like). The group can optionally be substituted with an alkyl or alkenyl or alkoxy group having 1 to 4 carbon atoms or halogens.

**The aryl alkyloxy group** can be benzyloxy or 2-phenylethyloxy.

**The alkylamino group** can be a straight chain or branched alkylamino group having 2-12 carbon atoms such as: methylamino; ethylamino; propylamino; isopropylamino; butylamino; isobutylamino; tert-butylamino; pentylamino; hexylamino; heptylamino; or octylamino.

**The alkenyl amino group** can be a straight chain or branched alkenylamino group having 2-12 carbon atoms. The alkenyl amino group can be selected from the group consisting of: ethynylamino-; 1-propynylamino-; 2-propynylamino-; 1-butynylamino-; 2-butynylamino-; 3-butynylamino-; 1-pentynylamino-; 2-pentynylamino-; 3-pentynylamino-; 4-pentynylamino-; 1-hexynylamino-; 2-hexynylamino-; 3-hexynylamino-; 4-hexynylamino-; 5-hexynylamino-; 1-heptynylamino-; 2-heptynylamino-; 3-heptynylamino-; 4-heptynylamino-; 5-heptynylamino-; 6-heptynylamino-; 1-octynylamino-; 2-octynylamino-; 3-octynylamino-; 4-octynylamino-; 5-octynylamino-; 6-octynylamino-; 7-octynylamino-; 1-nonynylamino-; 2-nonynylamino-; 3-nonynyl-amino; 4-nonynylamino-; 5-nonynylamino-; 6-nonynylamino-; 7-nonynylamino-; 8-nonynylamino-; 1-decynylamino-; 2-decynylamino-; 3-decynylamino-; 4-decynylamino-; 5-decynylamino-; 6-decynylamino-; 7-decynylamino-; 8-decynylamino-; 9-decynylamino-; 1-undecynylamino-; 2-undecynylamino-; 3-undecynylamino-; 4-undecynylamino-; 5-undecynylamino-; 6-undecynylamino-; 7-undecynylamino-; 8-undecynylamino-; 9-undecynylamino-; 10-undecynylamino-; 1-dodecynylamino-; 2-dodecynylamino-; 3-dodecynylamino-; 4-dodecynylamino-; 5-dodecynylamino-; 6-dodecynylamino-; 7-dodecynylamino-; 8-dodecynylamino-; 9-dodecynylamino-; 10-dodecynylamino-; and 11-dodecynylamino- groups; and related branched isomers. Preferably, the alkenyl amino group is a straight chain or branched alkenylamino group with 2 to 8 carbon atoms.

**The arylamino group** can be an arylamino group such as a phenylamino or naphtylamino group, optionally substituted with an alkyl or alkenyl or alkoxy group having 1 to 4 carbon atoms, or halogens. The arylamino group can be selected from the group consisting of: phenylamino; 2-methylphenylamino; 3-methylphenylamino; 4- methylphenylamino; 2-allylphenylamino; 2-chlorophenylamino; 3-chlorophenylamini; 4-chlorophenylamino; 4-methoxyphenylamino; 2-allyloxyphenylamino; naphtylamino; and the like.

**The arylalkylamino group** can be benzylamino or 2-phenylethylamino.

**The alkylthio group** can be an alkylthio group having 1 to 8 carbon atoms. The alkylthio group can be selected from the group consisting of: methylthio; ethylthio; propylthio; butylthio; isobutylthio; and pentylthio.

**The alkenylthio group** can be a straight chain or branched alkenylthio group having 1 to 8 carbon atoms. The alkenylthio group can be selected from the group consisting of: ethynylthio-; 1-propynylthio-; 2-propynylthio-; 1-butynylthio-; 2-butynylthio-; 3-butynylthio-; 1-pentynylathio-; 2-pentynylthio-; 3-pentynylthio-; 4-pentynylthio-; 1-hexynylthio-; 2-hexynylthio-; 3-hexynylthio-; 4-hexynylthio-; 5-hexynylthio-; and the like.

**The arylrthio group** can be an alkenylthio group having 1 to 8 carbon atoms such as a phenylthio or naphtylthio group, which can optionally be substituted with an alkyl or alkenyl or alkoxy group having 1 to 4 carbon atoms; and also halogens, e.g.: phenylthio; 2-m ethyl ph enylthio; 3-methylphenylthio; 4- methylphenylthio; 2-allylphenylthio; 2-chlorophenylthio; 3-chlorophenylamini; 4-chlorophenylthio; 4-methoxyphenylthio; 2-allyloxyphenylthio; naphtylthio; and the like.

**The arylalkylthio group** can be an alkenylthio group having 1 to 8 carbon atoms such as a benzylthio- group or a 2-phenylethylthio-group.

**The alkoxycarbonyl group** can be: methoxycarbonyl; ethoxycarbonyl; or the like.

**The substituted or unsubstituted carbamoyl group** can be: carbamoyl; methylcarbamoyl; dimethylcarbamoyl; diethylcarbamoyl; or the like.

**The acyl group** can be an acyl group with 1 to 8 carbon atoms such as: formyl; acetyl; propionyl; or benzoyl groups; or the like.

The alkyl, alkenyl, alkynyl, cycloalkyl, aryl, aryl alkyl, heterocyclic, alkoxy, alkenyloxy, aryloxy, aryl alkyloxy, alkylamino, alkenylamino, arylamino, arylalkylamino, alkylthio, alkenylthio, arylthio, arylalkylthio, alkoxycarbonyl, substituted and unsubstituted carbamoyl and acyl groups mentioned above can optionally be substituted. Examples of substituents include: halogens (F; Cl; Br; I); hydroxyl groups; mercapto groups; carboxylates; nitro groups; cyano groups; substituted or unsubstituted amino groups (including amino, methylamino, dimethylamino, ethylamino, diethylamino, and various protected amines such as tert-butoxycarbonyl- and arylsulfonamido groups); alkoxy groups (having 1 to 8 carbon atoms such as methoxy, ethoxy, propyloxy, isopropyloxy, butyloxy, isobutyloxy, tert-butyloxy, pentyloxy, hexyloxy, heptyloxy or octyloxy); alkenyloxy groups (having 2 to 8 carbon atoms such as a vinyloxy; allyloxy; 3-butenyloxy or 5-hexenyloxy); aryloxy groups (such as a phenoxy or naphtyloxy group which can be optionally substituted with an alkyl or alkenyl or alkoxy group having 1 to 4 carbon atoms; and also halogens, e.g., phenoxy, 2-methylphenoxy, 3-methylphenoxy, 4-methylphenoxy, 2-allylphenoxy, 2-chlorophenoxy, 3-chlorophenoxy, 4-chlorophenoxy, 4-methoxyphenoxy, 2-allyloxyphenoxy, naphtyloxy, and the like); aryl alkyloxy groups (e.g., benzyloxy and 2-phenylethyloxy); alkylthio groups (having 1 to 8 carbon atoms such as methylthio, ethylthio, propylthio, butylthio, isobutylthio, pentylthio); alkoxycarbonyl groups (e.g. methoxycarbonyl, ethoxycarbonyl, and the like); substituted or unsubstituted carbamoyl group (e.g. carbamoyl, methylcarbamoyl, dimethylcarbamoyl, diethylcarbamoyl and the like); acyl groups (with 1 to 8 carbon atoms such as formyl, acetyl, propionyl, or benzoyl groups); and others.

The above-mentioned cycloalkyl, cycloalkenyl, aryl, aryl alkyl, heterocyclic, alkoxy, alkenyloxy, aryloxy, aryl

alkyloxy, alkylthio, and alkoxycarbonyl groups can also be substituted with alkyl groups having 1 to 5 carbon atoms, alkenyl groups with 2 to 5 carbon atoms, or haloalkyl groups with 1 to 5 carbon atoms in addition to the substituents specified above.

The number of substituents can be one or more than one. The substituents can be the same or different.

The $R_1$ and $R_2$ groups can be each, independently of each other, be a functional group. The functional group is selected from the group consisting of: halo, pseudohalo, hydroxyl, variably substituted mercapto, variably substituted sulfinyl, variably substituted sulfonyl, carboxylates, variably substituted amino, variably substituted amido, variably substituted ureido, variably substituted carbamoyl, and variably substituted urethano. Pseudohalo is nitro, cyano, azido, cyanato, isocyanato, or isothiocyanato.

$R_1$ and $R_2$ together can also be present as a saturated or unsaturated optionally substituted carbocycle or a heterocycle having 5 to 12 carbon atoms, such as: cyclopentane; cyclohexane; cyclohexene; cyclohexadiene; cycloheptane; cyclooctane; cyclononane; or cyclodecane.

As used herein, a "variably substituted" group is a group that is unsubstituted or is substituted with one or more, in another embodiment one to five, in another embodiment one, two or three, substituents.

"Polypeptide" and "protein" are used interchangeably and mean any peptide-linked chain of amino acids, regardless of length or post-translational modification. The invention also features yeast derived enantioselective 2,2-disubstituted epoxide hydrolase (YEDH) polypeptides with conservative substitutions. Conservative substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine.

The term "isolated" polypeptide or peptide fragment, as used herein, refers to a polypeptide or a peptide fragment which either has no naturally-occurring counterpart or has been separated or purified from components which naturally accompany it, e.g., microorganism cellular components such as yeast cell cellular components. Typically, the polypeptide or peptide fragment is considered "isolated" when it is at least 70%, by dry weight, free from the proteins and other naturally-occurring organic molecules with which it is naturally associated. Preferably, a preparation of a polypeptide (or peptide fragment thereof) of the invention is at least 80%, more preferably at least 90%, and most preferably at least 99%, by dry weight, the polypeptide (or the peptide fragment thereof), respectively, of the invention. Thus, for example, a preparation of polypeptide x is at least 80%, more preferably at least 90%, and most preferably at least 99%, by dry weight, polypeptide x. Since a polypeptide that is chemically synthesized is, by its nature, separated from the components that naturally accompany it, the synthetic polypeptide is "isolated."

An isolated polypeptide (or peptide fragment) can be obtained, for example, by: extraction from a natural source (e.g., from yeast cells); expression of a recombinant nucleic acid encoding the polypeptide; or chemical synthesis. A polypeptide that is produced in a cellular system different from the source from which it naturally originates is "isolated," because it will necessarily be free of components which naturally accompany it. The degree of isolation or purity can be measured by any appropriate method, e.g., column chromatography, polyacrylamide gel electrophoresis, or HPLC analysis.

An "isolated DNA" is either (1) a DNA that contains sequence not identical to that of any naturally occurring sequence, or (2), in the context of a DNA with a naturally-occurring sequence (e.g., a cDNA or genomic DNA), a DNA free of at least one of the genes that flank the gene containing the DNA of interest in the genome of the organism in which the gene containing the DNA of interest naturally occurs. The term therefore includes a recombinant DNA incorporated into a vector, into an autonomously replicating plasmid or virus, or into the genomic DNA of a prokaryote or eukaryote. The term also includes a separate molecule such as: a cDNA (e.g., SEQ ID NOs: 8, 9, 10, 11, 12, 13, or 14) where the corresponding genomic DNA has introns and therefore a different sequence; a genomic fragment that lacks at least one of the flanking genes; a fragment of cDNA or genomic DNA produced by polymerase chain reaction (PCR) and that lacks at least one of the flanking genes; a restriction fragment that lacks at least one of the flanking genes; a DNA encoding a non-naturally occurring protein such as a fusion protein, mutein, or fragment of a given protein; and a nucleic acid which is a degenerate variant of a cDNA or a naturally occurring nucleic acid. In addition, it includes a recombinant nucleotide sequence that is part of a hybrid gene, i.e., a gene encoding a non-naturally occurring fusion protein. Also included is a recombinant DNA that includes a portion of SEQ ID NOs: 8-14. It will be apparent from the foregoing that isolated DNA does not mean a DNA present among hundreds to millions of other DNA molecules within, for example, cDNA or genomic DNA libraries or genomic DNA restriction digests in, for example, a restriction digest reaction mixture or an electrophoretic gel slice.

As used herein, a "functional fragment" of a YEDH polypeptide is a fragment of the polypeptide that is shorter than the full-length polypeptide and has at least 20% (e.g., at least: 3.0%; 40%; 50%; 60%; 70%; 80%; 90%; 95%; 98%; 99%; 100%, or more) of the ability of the full-length polypeptide to enantioselectively hydrolyse a TDE of interest. Fragments of interest can be made by either recombinant, synthetic, or proteolytic digestive methods and tested for their ability to enantioselectively hydrolyse a TDE.

As used herein, "operably linked" means incorporated into a genetic construct so that an expression control

sequence effectively controls expression of a coding sequence of interest.

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention.

The materials, methods, and examples disclosed herein are illustrative only and not intended to be limiting.

Other features and advantages of the invention, e.g., TDE and DVD substantially enriched for one optical enantiomer, will be apparent from the following description, from the drawings and from the claims.

The details of one or more embodiments of the invention are set forth in the accompanying drawings and the description below. Other features, objects, and advantages of the invention will be apparent from the description and drawings, and from the claims.

## DESCRIPTION OF DRAWINGS

Fig. 1 is schematic depiction of the reactions catalyzed by YEDH using racemic mixtures of two exemplary 2,2-disubstituted epoxides (of general formula (I)) and resulting in optically active epoxides and vicinal diols.

Figs. 2A to 10B are line graphs showing the hydrolysis of (±)-2-methyl-1,2-epoxyheptane by the indicated yeast strains to produce optically active (S)-2-methyl-1,2-epoxyheptane and/or (R)-2-methyl-1,2-heptanediol. The A panel in each figure is a line graph showing the change in concentrations of the epoxide and diol enantiomers with time and the B panel in each figure is a line graph showing the enantiomeric excess of the epoxide and diol at different conversions.

Figs. 11A and 11B are line graphs showing the hydrolysis of (±)-2-methyl-1,2-epoxyheptane by the indicated yeast strain to produce optically active (R)-2-methyl-1,2-epoxyheptane and/or (S)-2-methyl-1,2-heptanediol. The A and B panels are as indicated for Figs. 2A to 10B.

Figs. 12A and 12B are line graphs showing the hydrolysis of (±)-2-methyl-3-phenyl-1,2-epoxypropane by the indicated yeast strain to produce optically active (S)-2-methyl-3-phenyl-1,2-epoxypropane and/or (R)-2-methyl-3-phenyl-1,2-propanediol. The A and B panels are as indicated for Figs. 2A to 10B.

Figs. 13A to 14B are line graphs showing the hydrolysis of (±)-2-methyl-3-phenyl-1,2-epoxypropane by the indicated yeast strains to produce optically active (R)-2-methyl-3-phenyl-1,2-epoxypropane and/or (S)-2-methyl-3-phenyl-1,2-propanediol. The A and B panels are as indicated for Figs. 2A to 10B.

Fig. 15 is a line graph showing the hydrolysis of (±)-2-methyl-3-phenyl-1,2-epoxypropane by the indicated yeast cultivated in a 10 l fermenter to produce optically active (S)- 2-methyl-3-phenyl-1,2-epoxypropane and/or (R)-2-methyl-3-phenyl-1,2-propanediol. The graph shows the change in concentrations of the epoxide and diol enantiomers with time.

Figs. 16, 17, and 18 are line graphs showing the hydrolysis of (±)-2-methyl-3-phenyl-1,2-epoxypropane by yeast host strains transformed with vectors expressing YEDH from selected wild type yeast strains to produce optically active (S)- 2-methyl-3-phenyl-1,2-epoxypropane and/or (R)-2-methyl-3-phenyl-1,2-propanediol. The graphs show the change in concentrations of the epoxide and diol enantiomers with time.

Figs. 19A and 19B are line graphs showing the hydrolysis of (±)-2-methyl-3-phenyl-1,2-epoxypropane using whole cells (Fig. 19 A) and a crude enzyme preparation (Fig. 19B) obtained from the host *Yarrowia lipolytica* expressing the epoxide hydrolase derived from *Rhodotorula araucariae* NCYC 3183 (YL 25 TsA). This experiment demonstrates that whole cells or enzyme preparations can be used to catalyze the reactions.

Fig. 20 is a restriction map of the pYLHmA (pINA1291) expression vector. The positions of the hp4d promoter and LIP2 terminator and of unique restriction sites available for the insertion of coding sequences are indicated.

Fig. 21 is a restriction map of the pYLTsA (pINA3313) expression vector. The positions of the TEF promoter and LIP2 terminator and of unique restriction sites available for the insertion of coding sequences are indicated.

Fig. 22 is a depiction of the amino acid sequence (SEQ ID NO:1) of a YEDH polypeptide encoded by cDNA derived from a *Rhodosporidium toruloides* strain (assigned accession no. NCYC 3181).

Fig. 23 is a depiction of the amino acid sequence (SEQ ID NO:2) of a YEDH polypeptide encoded by cDNA derived from a *Rhodosporidium toruloides* strain (assigned identification no. UOFS Y-0471).

Fig. 24 is a depiction of the amino acid sequence (SEQ ID NO:3) of a YEDH polypeptide encoded by cDNA derived from a *Rhodotorula araucariae* strain (assigned accession no. NCYC 3183).

Fig. 25 is a depiction of the amino acid sequence (SEQ ID NO:4) of a YEDH polypeptide encoded by cDNA derived from a *Cryptococcus curvatus* strain (assigned accession no. NCYC 3158).

Fig. 26 is a depiction of the amino acid sequence (SEQ ID NO:5) of a YEDH polypeptide encoded by cDNA derived from a *Rhodosporidium paludigenum* (assigned accession no. NCYC 3179).

Fig. 27 is a depiction of the amino acid sequence (SEQ ID NO:6) of a YEDH polypeptide encoded by cDNA derived from a *Debaromyces hansenii* (assigned accession no. NCYC 3167).

Fig. 28 is a depiction of the partial amino acid sequence (SEQ ID NO:7) of a YEDH polypeptide encoded by cDNA derived from a *Rhodotorula minuta var. minuta* (assigned accession no. UOFS Y-0835).

Fig. 29 is a depiction of the nucleotide sequence (SEQ ID NO:8) of a YEDH polypeptide-encoding cDNA derived from a *Rhodosporidium toruloides* strain (assigned accession no. NCYC 3181).

Fig. 30 is a depiction of the nucleotide sequence (SEQ ID NO:9) of a YEDH polypeptide-encoding cDNA derived from a *Rhodosporidium toruloides* strain (assigned identification no. UOFS Y-0471).

Fig. 31 is a depiction of the nucleotide sequence (SEQ ID NO:10) of a YEDH polypeptide-encoding cDNA derived from a *Rhodotorula araucariae* strain (assigned accession no. NCYC 3183).

Fig. 32 is a depiction of the nucleotide sequence (SEQ ID NO:11) of a YEDH polypeptide-encoding cDNA derived from a *Cryptococcus curvatus* strain (assigned accession no. NCYC 3158).

Fig. 33 is a depiction of the nucleotide sequence (SEQ ID NO:12) of a YEDH polypeptide-encoding cDNA derived from a *Rhodosporidium paludigenum* (assigned accession no. NCYC 3179).

Fig. 34 is a depiction of the nucleotide sequence (SEQ ID NO:13) of a YEDH polypeptide-encoding cDNA derived from a *Debaromyces hansenii* (assigned accession no. NCYC 3167).

Fig. 35 is a depiction of the partial nucleotide sequence (SEQ ID NO:14) of a YEDH polypeptide-encoding cDNA derived from a *Rhodotorula minuta var. minuta* (assigned accession no. UOFS Y-0835).

Fig. 36 is a table showing the homology at the amino acid level of the YEDH polypeptides with SEQ ID NOs: 1-6.

Fig. 37 is a table showing the homology at the nucleotide level of YEDH-encoding cDNA molecules with SEQ ID NOs: 8-13.

Fig. 38 is a depiction of the amino acid sequences of eight enantioselective epoxide hydrolases aligned for maximum homology. Also shown are consensus amino acids. The sequences labeled #1, #46, #25, Car054, and #692 correspond to SEQ ID NOs: 1-5 and the sequences labeled #23, Jen46-2 and # 777 correspond to enantioselective hydrolases catalyzing the hydrolysis of non-TDE epoxides. The consensus catalytic triad is composed of a nucleophile, an acid and a base, the positions of which are indicated by N, A and B, respectively. "HGXP" represents the region of the oxy-anion hole of the enzyme. "sxNxss" represents the genetic motif found in $\alpha/\beta$-hydrolase fold enzymes. (= homology; P = identity of 75-100%; P = identity of 50-75%;. = gap).

## DETAILED DESCRIPTION

Various aspects of the invention are described below.

Nucleic Acid Molecules

The YEDH nucleic acid molecules can be cDNA, genomic DNA, synthetic DNA, or RNA, and can be double-stranded or single-stranded (i.e., either a sense or an antisense strand). Segments of these molecules are also considered and can be produced by, for example, the polymerase chain reaction (PCR) or generated by treatment with one or more restriction endonucleases. A ribonucleic acid (RNA) molecule can be produced by *in vitro* transcription. Preferably, the nucleic acid molecules encode polypeptides that, regardless of length, are soluble under normal physiological conditions.

The nucleic acid molecules can contain naturally occurring sequences, or sequences that differ from those that occur naturally, but, due to the degeneracy of the genetic code, encode the same polypeptide (for example, one the polypeptides with SEQ ID NOS:1 - 7). In addition, these nucleic acid molecules are not limited to coding sequences, e.g., they can include some or all of the non-coding sequences that lie upstream or downstream from a coding sequence.

The nucleic acid molecules can be synthesized (for example, by phosphoramidite-based synthesis) or obtained from a biological cell, such as the cell of a eukaryote (e.g., a mammal such as human or a mouse or a yeast such as any of the genera, species, and strains of yeast disclosed herein) or a prokarote (e.g., a bacterium such as *Escherichia coli*). The nucleic acids can be those of a yeast such as any of the genera, species, and strains of yeast disclosed herein. Combinations or modifications of the nucleotides within these types of nucleic acids are also encompassed.

Techniques associated with detection or regulation of genes are well known to skilled artisans. Such techniques can be used, for example, to test for expression of a YEDH gene in a test cell (e.g. a yeast cell) of interest.

A YEDH family gene or protein can be identified based on its similarity to the relevant YEDH gene or protein, respectively. For example, the identification can be based on sequence identity. The invention features isolated nucleic acid molecules which are, or are at least 50% (e.g., at least: 55%; 60%; 65%; 75%; 85%; 95%; 98%; or 99%) identical to: (a) a nucleic acid molecule that encodes the polypeptide of SEQ ID NOs: 1-7; (b) the nucleotide sequence of SEQ ID NOs: 8-14; (c) a nucleic acid molecule which includes a segment of at least 15 (e.g., at least: 20; 25; 30; 35; 40; 50; 60; 80; 100; 125; 150; 175; 200; 250; 300; 350; 400; 500; 600; 700; 800; 900; 1,000; 1,100; 1,200; 1,220; 1,225; 1,228; 1,230; 1,231; 1,232; 1,233; 1,234; 1,235; or 1,236) nucleotides of SEQ ID NOs: 8-14; (d) a nucleic acid molecule encoding

any of the polypeptides or fragments thereof disclosed below; and (e) the complement of any of the above nucleic acid molecules. The complements of the above molecules can be full-length complements or segment complements containing a segment of at least 15 (e.g., at least: 20; 25; 30; 35; 40; 50; 60; 80; 100; 125; 150; 175; 200; 250; 300; 350; 400; 500; 600; 700; 800; 900; 1,000; 1,100; 1,200; 1,220; 1,225; 1,228; 1,230; 1,231; 1,232; 1,233; 1,234; 1,235; or 1,236) consecutive nucleotides complementary to any of the above nucleic acid molecules. Identity can be over the full-length of SEQ ID NOs: 8-14 or over one or more contiguous or non-contiguous segments.

The determination of percent identity between two sequences is accomplished using the mathematical algorithm of Karlin and Altschul, Proc. Natl, Acad. Sci, USA 90, 5873-5877, 1993. Such an algorithm is incorporated into the BLASTN and BLASTP programs of Altschul et al. (1990) J. Mol. Biol. 215,403-410. BLAST nucleotide searches are performed with the BLASTN program, score = 100, wordlength = 12, to obtain nucleotide sequences homologous to YEDH-encoding nucleic acids. BLAST protein searches are performed with the BLASTP program, score = 50, wordlength = 3, to obtain amino acid sequences homologous to the YEDH polypeptide. To obtain gapped alignments for comparative purposes, Gapped BLAST is utilized as described in Altschul et al. (1997) Nucleic Acids Res. 25, 3389-3402. When utilizing BLAST and Gapped BLAST programs, the default parameters of the respective programs (e.g., XBLAST and NBLAST) are used.

Hybridization can also be used as a measure of homology between two nucleic acid sequences. A YEDH-encoding nucleic acid sequence, or a portion thereof, can be used as a hybridization probe according to standard hybridization techniques. The hybridization of a YEDH probe to DNA or RNA from a test source (e.g., a mammalian cell) is an indication of the presence of YEDH DNA or RNA in the test source. Hybridization conditions are known to those skilled in the art and can be found in Current Protocols in Molecular Biology, John Wiley & Sons, N.Y., 6.3.1-6.3.6, 1991. Moderate hybridization conditions are defined as equivalent to hybridization in 2X sodium chloride/sodium citrate (SSC) at 30°C, followed by a wash in 1 X SSC, 0.1% SDS at 50°C. Highly stringent conditions are defined as equivalent to hybridization in 6X sodium chloride/sodium citrate (SSC) at 45°C, followed by a wash in 0.2 X SSC, 0.1% SDS at 65°C.

The invention also encompasses: (a) vectors (see below) that contain any of the foregoing YEDH coding sequences (including coding sequence segments) and/or their complements (that is, "antisense" sequences); (b) expression vectors that contain any of the foregoing YEDH coding sequences (including coding sequence segments) operably linked to one or more transcriptional and/or translational regulatory elements (TRE; examples of which are given below) necessary to direct expression of the coding sequences; (c) expression vectors encoding, in addition to a YEDH polypeptide (or a fragment thereof), a sequence unrelated to YEDH, such as a reporter, a marker, or a signal peptide fused to YEDH; and (d) genetically engineered host cells (see below) that contain any of the foregoing expression vectors and thereby express the nucleic acid molecules of the invention.

Recombinant nucleic acid molecules can contain a sequence encoding a YEDH polypeptide or a YEDH polypeptide having an heterologous signal sequence. The full length YEDH polypeptide, or a fragment thereof, can be fused to such heterologous signal sequences or to additional polypeptides, as described below. Similarly, the nucleic acid molecules of the invention can encode the mature form of a YEDH or a form that includes an exogenous polypeptide that facilitates secretion.

The TRE referred to above and further described below include but are not limited to inducible and non-inducible promoters, enhancers, operators and other elements that are known to those skilled in the art and that drive or otherwise regulate gene expression. Such regulatory elements include but are not limited to the cytomegalovirus hCMV immediate early gene, the early or late promoters of SV40 adenovirus, the *lac* system, the *trp* system, the *TAC* system, the *TRC* system, the major operator and promoter regions of phage A, the control regions of fd coat protein, the promoter for 3-phosphoglycerate kinase, the promoters of acid phosphatase, and the promoters of the yeast α-mating factors. Other useful TRE are listed in the examples below.

Similarly, the nucleic acid can form part of a hybrid gene encoding additional polypeptide sequences, for example, a sequence that functions as a marker or reporter. Examples of marker and reporter genes include β-lactamase, chloramphenicol acetyltransferase (CAT), adenosine deaminase (ADA), aminoglycoside phosphotransferase ($neo^r$, $G418^r$), dihydrofolate reductase (DHFR), hygromycin-B-phosphotransferase (HPH), thymidine kinase (TK), lacZ (encoding β-galactosidase), and xanthine guanine phosphoribosyltransferase (XGPRT), and green, blue, or yellow fluorescent protein. As with many of the standard procedures associated with the practice of the invention, skilled artisans will be aware of additional useful reagents, for example, additional sequences that can serve the function of a marker or reporter. Generally, the hybrid polypeptide will include a first portion and a second portion; the first portion being a YEDH polypeptide (or any of YEDH fragments described below) and the second portion being, for example, the reporter described above or an Ig heavy chain constant region or part of an Ig heavy chain constant region, e.g., the CH2 and CH3 domains of IgG2a heavy chain. Other hybrids could include an antigenic tag or a poly-His tag to facilitate purification.

The expression systems that can be used for purposes of the invention include, but are not limited to, microorganisms such as yeasts (e.g, any of the genera, species or strains listed herein) or bacteria (for example, *E. coli* and *B. subtilis*) transformed with recombinant bacteriophage DNA, plasmid DNA, or cosmid DNA expression vectors containing the nucleic acid molecules of the invention; yeast (for example, *Saccharomyces, Kluyveromyces, Hansenula,*

*Pichia, Yarrowia, Arxula, Candida,* and other genera, species, and strains listed herein) transformed with recombinant yeast expression vectors containing the nucleic acid molecule of the invention; insect cell systems infected with recombinant virus expression vectors (for example, baculovirus) containing the nucleic acid molecule of the invention; plant cell systems infected with recombinant virus expression vectors (for example, cauliflower mosaic virus (CaMV) or tobacco mosaic virus (TMV)) or transformed with recombinant plasmid expression vectors (for example, Ti plasmid) containing a YEDH nucleotide sequence; or mammalian cell systems (for example, COS, CHO, BHK, 293, VERO, HeLa, MDCK, WI38, and NIH 3T3 cells) harboring recombinant expression constructs containing promoters derived from the genome of mammalian cells (for example, the metallothionein promoter) or from mammalian viruses (for example, the adenovirus late promoter and the vaccinia virus 7.5K promoter). Also useful as host cells are primary or secondary cells obtained directly from a mammal and transfected with a plasmid vector or infected with a viral vector.

The invention includes wild-type and recombinant Yarrowia lipolytica cells containing any of the above YEDH genes, nucleic acid molecules, and genetic constructs. Other cells that can be used as host cells are listed herein. The cells are preferably isolated cells. As used herein, the term "isolated" as applied to a microorganism (e.g., a yeast cell) refers to a microorganism which either has no naturally-occurring counterpart (e.g., a recombinant microorganism such as a recombinant yeast) or has been extracted and/or purified from an environment in which it naturally occurs. Thus, an "isolated microorganism" does not include one residing in an environment in which it naturally occurs, for example, in the air, outer space, the ground, oceans, lakes, rivers, and streams and the like, ground at the bottom of oceans, lakes, rivers, and streams and the like, snow, ice on top of the ground or in/on oceans lakes, rivers, and streams and the like, man-made structures (e.g., buildings), or in natural hosts (e.g., plant, animal or microbial hosts) of the microorganism, unless the microorganism (or a progenitor of the microorganism) was previously extracted and or purified from an environment in which it naturally occurs and subsequently returned to such an environment or any other environment in which it can survive. An example of an isolated microorganism is one in a substantially pure culture of the microorganism.

Disclosed herein is a substantially pure culture of a microorganism (e.g., a microbial cell such as a yeast cell. As used herein, a "substantially pure culture" of a microorganism is a culture of that microorganism in which less than about 40% (i.e., less than about: 35%; 30%; 25%; 20%; 15%; 10%; 5%; 2%; 1%; 0.5%; 0.25%; 0.1%; 0.01%; 0.001%; 0.0001%; or even less) of the total number of viable microbial cells (bacterial, fungal (including yeast), mycoplasmal, or protozoan cells) in the culture are viable microbial cells other than the microorganism. The term "about" in this context means that the relevant percentage can be 15% percent of the specified percentage above or below the specified percentage. Thus, for example, about 20% can be 17% to 23%. Such a culture of microorganisms includes the microorganisms and a growth, storage, or transport medium. Media can be liquid, semi-solid (e.g., gelatinous media), or frozen. The culture includes the cells growing in the liquid or in/on the semi-solid medium or being stored or transported in a storage or transport medium, including a frozen storage or transport medium. The cultures are in a culture vessel or storage vessel or substrate (e.g., a culture dish, flask, or tube or a storage vial or tube).

The microbial cells of the invention can be stored, for example, as frozen cell suspensions, e.g. in buffer containing a cryoprotectant such as glycerol or sucrose, as lyophilized cells. Alternatively, they can be stored, for example, as dried cell preparations obtained, e.g., by fluidised bed drying or spray drying, or any other suitable drying method. Similarly the enzyme preparations can be frozen, lyophilised, or immobilized and stored under appropriate conditions to retain activity.

Polypeptides and Polypeptide Fragments

The YEDH polypeptides of the invention include all the YEDH polypeptides and fragments of YEDH polypeptides disclosed herein. They can be, for example, the polypeptides with SEQ ID NOs:1-7 and functional fragments of these polypeptides. The polypeptides embraced by the invention also include fusion proteins that contain either full-length or a functional fragment of it fused to unrelated amino acid sequence. The unrelated sequences can be additional functional domains or signal peptides.

Isolated polypeptides which are, or are at least 50% (e.g., at least: 55%; 60%; 65%; 75%; 85%; 95%; 98%; or 99%) identical to the polypeptides with SEQ ID Nos: 1-7. The identity can be over the full-length of the latter polypeptides or over one or more contiguous or non-contiguous segments are taught herein.

Fragments of YEDH polypeptides are segments of the full-length YEDH polypeptides that are shorter than full-length YEDH polypeptides. Fragments of YEDH polypeptides can contain 5-410 (e.g., 10, 20, 30, 40, 50, 60, 70, 80, 90, 100, 120, 150, 250, 300, 350, 400, 405, 406, 407, 408, or 409) amino acids of SEQ ID NOs:1-7. Fragments of YEDH can be functional fragments or antigenic fragments.

The polypeptides can be any of those described above but with not more 50 (e.g., not more than 50, 45, 40, 35, 30, 25, 20, 17, 14, 12, 10, nine, eight, seven, six, five, four, three, two, or one) conservative substitution(s). Such substitutions can be made by, for example, site-directed mutagenesis or random mutagenesis of appropriate YEDH coding sequences.

"Functional fragments" of a YEDH polypeptide (and, optionally, any of the above-described YEDH polypeptide

variants) have at least 20% (e.g., 30%, 40%, 50%, 60%, 70%, 80%, 90%, 95%, 98%, 99%, 100%, or more) of the ability of the full-length, wild-type YEDH polypeptide to enantioselectively hydrolyse a TDE of interest. One of skill in the art will be able to predict YEDH functional fragments using his or her own knowledge and information provided herein, e.g., the amino acids alignments in Fig. 38 showing highly conserved domains as well and residues required for function of epoxide hydrolase activity.

Fragments of interest can be made either by recombinant, synthetic, or proteolytic digestive methods and tested for their ability to enantioselectively hydrolyse a TDE.

Antigenic fragments of the polypeptides of the invention are fragments that can bind to an antibody. Methods of testing whether a fragment of interest can bind to an antibody are known in the art.

The polypeptides can be purified from natural sources (e.g., wild-type or recombinant yeast cells such as any of those described herein). Smaller peptides (e.g., those less than about 100 amino acids in length) can also be conveniently synthesized by standard chemical means. In addition, both polypeptides and peptides can be produced by standard *in vitro* recombinant DNA techniques and *in vivo* transgenesis, using nucleotide sequences encoding the appropriate polypeptides or peptides. Methods well-known to those skilled in the art can be used to construct expression vectors containing relevant coding sequences and appropriate transcriptional/translational control signals. See, for example, the techniques described in Sambrook et al., Molecular Cloning: A Laboratory Manual (2nd Ed.) [Cold Spring Harbor Laboratory, N.Y., 1989], and Ausubel et al., Current Protocols in Molecular Biology [Green Publishing Associates and Wiley Interscience, N.Y., 1989].

Polypeptides and fragments of the invention also include those described above, but modified by the addition, at the amino- and/or carboxyl-terminal ends, of a blocking agent to facilitate survival of the relevant polypeptide. This can be useful in those situations in which the peptide termini tend to be degraded by proteases. Such blocking agents can include, without limitation, additional related or unrelated peptide sequences that can be attached to the amino and/or carboxyl terminal residues of the peptide to be administered. This can be done either chemically during the synthesis of the peptide or by recombinant DNA technology by methods familiar to artisans of average skill.

Alternatively, blocking agents such as pyroglutamic acid or other molecules known in the art can be attached to the amino and/or carboxyl terminal residues, or the amino group at the amino terminus or carboxyl group at the carboxyl) terminus can be replaced with a different moiety. Likewise, the peptides can be covalently or noncovalently coupled to pharmaceutically acceptable "carrier" proteins prior to administration.

Also of interest are peptidomimetic compounds that are designed based upon the amino acid sequences of the functional peptide fragments. Peptidomimetic compounds are synthetic compounds having a three-dimensional conformation (i.e., a "peptide motif") that is substantially the same as the three-dimensional conformation of a selected peptide. The peptide motif provides the peptidomimetic compound with the ability to enantioselectively hydrolyse a TDE of interest in a manner qualitatively identical to that of the YEDH functional fragment from which the peptidomimetic was derived. Peptidomimetic compounds can have additional characteristics that enhance their therapeutic utility, such as increased cell permeability and prolonged biological half-life.

The peptidomimetics typically have a backbone that is partially or completely non-peptide, but with side groups that are identical to the side groups of the amino acid residues that occur in the peptide on which the peptidomimetic is based. Several types of chemical bonds, e.g., ester, thioester, thioamide, retroamide, reduced carbonyl, dimethylene and ketomethylene bonds, are known in the art to be generally useful substitutes for peptide bonds in the construction of protease-resistant peptidomimetics.

The invention also provides compositions and preparations containing one or more (e.g., two, three, four, five, six, seven, eight, nine, ten, 12, 15, 20, 25, or more) of the above-described polypeptides, polypeptide variants, and polypeptide fragments. The composition or preparation can be, for example a crude cell (e.g., yeast cell) extract or culture supernatant, a crude enzyme preparation, a highly purified enzyme preparation. The compositions and preparations can also contain one or more of a variety of carriers or stabilizers known in the art. Carriers and stabilizers are known in the art and include, for example: buffers, such as phosphate, citrate, and other non-organic acids; antioxidants such as ascorbic acid; low molecular weight (less than 10 residues) polypeptides; proteins such as serum albumin, gelatin or immunoglobulins; hydrophilic polymers such polyvinylpyrrolidone; amino acids such as glycine, glutamine, asparagine, arginine, or lysine; monosaccharides, disaccharides, and other carbohydrates including glucose, mannose, or dextrans; chelating agents such as ethylenediaminetetraacetic acid (EDTA); sugar alcohols such as mannitol, or sorbitol; salt-forming counterions such as sodium; and/or nonionic surfactants such as Tween, and Pluronics.

## Methods of Producing Optically Active Epoxides and Optimally Active Vicinal Diols

The invention provides methods for obtaining enantiopure, or substantially enantiopure, optically active TDE and DVD. Enantiopure optically active TDE or DVD preparations are preparations containing one enantiomer of the TDE or DVD and none of the other enantiomer of the TDE or DVD. "Substantially enantiopure" optically active TDE or DVD preparations are preparations containing at least 55% (e.g., at least: 60%; 70%; 80%; 85%; 90%; 95%; 97%; 98%; 99%;

99.5%; 99.8%; or 99.9%), relative to the total amount of both TDE or DVD enantiomers, of the particular enantiomer of the TDE or the DVD.

The method involves exposing a TDE enantiomeric mixture to a YEDH polypeptide, e.g., by culturing or incubating the mixtures with an isolated YEDH polypeptide or a cell (wild-type or recombinant) that expresses the polypeptide. This exposure can have a variety of outcomes that depend on variables such as, without limitation, the YEDH itself, the chemical nature of the TDE, and, to a lesser extent, the reaction conditions.

First, the YEDH polypeptide can catalyse the conversion of only one of the two TDE enantiomers in the mixture to its corresponding DVD enantiomer. Alternatively, it can catalyse the conversion of one of the two TDE enantiomers in the mixture to its corresponding DVD enantiomer at a much more rapid rate than the other TDE enantiomer to its corresponding DVD. YEDH with such selective activity are designated "enantioselective" and catalyse the "classical" kinetic resolution of a mixture of TDE enantiomers. Such a YEDH polypeptide can catalyse, for example, the conversion of a (R)-TDE (in the TDE enantiomeric mixture) to its corresponding (R)-DVD. During such a reaction, the concentration of the selected TDE enantiomer decreases, the concentration of the unselected TDE remains constant or decreases at a much slower rate (i.e., the concentration of the unselected TDE increases relative to the concentration of the selected TDE in the mixture); the DVD corresponding to the selected TDE is produced, and the DVD corresponding to the unselected TDE is not produced or is produced at a much lower rate than that the DVD corresponding to the selected TDE. Examples of such reactions include those shown in Figs. 3, 6, 13, and 19. These reactions are of course useful for the enrichment, and hence the production, of a desired TDE enantiomer and/or the production of a desired DVD enantiomer.

In addition, a YEDH polypeptide can catalyse, under certain defined reaction conditions, the conversion of both enantiomers of a TDE to a single DVD. Such YEDH hydrolyse each TDE enantiomer with opposite regioselectivity (i.e., the attack of water occurs at different carbons of the epoxide ring for each of the TDE enantiomers) and are termed "regiospecific" for each TDE enantiomer. Such YEDH are said to catalyse the "enantioconvergent" hydrolysis of a mixture of TDE enantiomers. The YEDH can also have significant selectivity for one enantiomer of the TDE. Where the YEDH has such TDE enantiomeric selectivity, the DVD enantiomer produced has the same chirality as the TDE for which the YEDH polypeptide is selective; for example, if the YEDH polypeptide is selective for an (S)-TDE enantiomer, the DVD enantiomer produced from both TDE enantiomers will be the (S)-DVD enantiomer. Examples of such reactions include those shown in Figs. 9 and 11. During these reactions, there is a decrease in the concentrations of both TDE enantiomers (with the concentration of one decreasing faster than the other if the YDEH polypeptide has TDE enantiomeric selectivity) and the production of one DVD enantiomer. Such reactions are particularly useful for the production of a desired DVD enantiomer and, where the YEDH is significantly TDE enantioselective, they can also be useful for the enrichment and hence the production of a particular TDE enantiomer. The degree of the latter enrichment can be enhanced by, for example, stopping a reaction at a time when the concentration of the selected TDE has significantly decreased and that of the unselected TDE enantiomer is still relatively high.

In yet other reaction types, the YEDH polypeptide can catalyse, for example: (a) the conversion of one TDE enantiomer its corresponding DVD enantiomer and the other TDE enantiomer to both DVD enantiomers; or (b) the conversion of both TDE enantiomers to both DVD enantiomers. Naturally, such reactions, if the YEDH polypeptides employed have no enantioselectivity, would not be useful for the production of a desired TDE enantiomer or a desired DVD enantiomer. On the other hand, where the YEDH polypeptide has significant selectivity for one TDE enantiomer, the reaction can be used for the production of: (a) the corresponding DVD enantiomer; and (b) the unselected TDE enantiomer. This can be done, as described above, by, e.g., stopping a reaction at a time, or at times, at which the concentration of the desired TDE enantiomer (relative to the total TDE concentration) and/or the concentration of the desired DVD enantiomer (relative to the total DVD concentration) are higher than those of the undesired TDE enantiomer and/or the undesired DVD enantiomer, respectively. In situations where a YEDH polypeptide catalyses the conversion of one TDE enantiomer to both DVD enantiomers, advantage can also be taken of the fact that generally the production of one DVD enantiomer (e.g., one with the chirality corresponding to that of a selected TDE) is favored over the enantiomer.

A YEDH polypeptide useful for the invention is one that hydrolyses one enantiomer of a TDE, and/or effects the production of one enantiomer of a DVD, with less than 80% (e.g., less than: 70%; 60%; 50%; 40%; 30%; 20%; 10%; 5%; 2.5%; 1%; 0.5%; 0.25%; 0,01%, or less), or even none, of the efficiency (as measured by reaction rate) with which it hydrolyses the other TDE enantiomer and/or effects the production of the other DVD enantiomer, respectively.

Useful concentrations of the TDE and conditions of incubation will vary from one YEDH polypeptide to another and from one TDE to another. Given the teachings of the working examples contained herein, one skilled in the art will know how to select working conditions for the production of a desired enantiomer of a desired DVD and/or TDE.

The method can be implemented by, for example, incubating (culturing) the TDE enantiomeric mixtures with a wild-type yeast cell or a recombinant cell (yeast or any other host species listed herein) containing a nucleic acid sequence (e.g. a gene, or a recombinant nucleic acid sequence) encoding a YEDH, a crude extract from such cells, a semi-purified preparation of aYEDH polypeptide, or, for example an isolated YEDH polypeptide. As used herein, "incubating and "culturing" include growing cells and maintaining them in a resting state.

The strain of the yeast cell can be selected from but are not limited to, the following exemplary genera: *Arxula, Brettanomyces, Bullera, Bulleromyces, Candida, Cryptococcus, Debaryomyces, Dekkera, Exophiala, Geotrichum, Hormonema, Issatchenkia, Kluyveromyces, Lipomyces, Mastigomyces, Myxozyma, Pichia, Rhodosporidium, Rhodotorula, Sporidiobolus, Sporobolomyces, Trichosporon, Wingea*, and *Yarrowia.*

Yeast strains innately capable of producing a polypeptide which converts or hydrolyses mixtures of TDE enantiomers to optically active (S)-TDE and/or (R)-DVD include the following exemplary yeast genera and species:

| Genus | Species |
|---|---|
| *Arxula* | *A. adeninivorans* |
| *Bullera* | *B. dendrophila* |
| *Cryptococcus* | *C. albidus*<br>*C. curvatus*<br>*C. macerans*<br>*C. hungaricus*<br>*C. podzolicus* |
| *Rhodosporidium* | *R. paludigenum*<br>*R. sphaerocarpum*<br>*R. toruloides* |
| *Rhodotorula* | *R. araucariae*<br>*R. aurantiaca*<br>*R. glutinis*<br>*R. species* (e.g. NCYC 3192, UOFS Y-2042) |
| *Sporidiobolus* | *S. salmonicolor* |
| *Sporobolomyces* | *S. roseus*<br>*S. sp. "holsaticus"*<br>*S. tsugae* |
| *Trichosporon* | *T. cutaneum var. cutaneum*<br>*T. jirovecii*<br>*T. moniliiforme*<br>*T. species* (e.g. NCYC 3210, NCYC 3211) |
| *Yarrowia* | *Y. lipolytica* |

Yeast species referred to in the chart above (and the one below) as "*species*" correspond to strains obtained from a culture collection, the species of which had not at the time of writing been identified.

Yeast strains innately capable of producing a polypeptide that converts or hydrolyses mixtures of epoxides to optically active (R)-epoxides and/or (S)-vicinal diols include the following exemplary genera and species:

| Genus | Species |
|---|---|
| *Arxula* | *A. terrestris* |
| *Bullera* | *B. dendrophila* |
| *Candida* | *C. magnoliae*<br>*C. parapsilosis*<br>*C. rugosa*<br>*C. tenuis*<br>*C.* sp. (new) nearest *C. sorbophila* |
| *Cryptococcus* | *C. amylolentus*<br>*C. curvatus*<br>*C. laurentii*<br>*C. luteolus* |

(continued)

| Genus | Species |
|---|---|
| | *C. macerans* |
| *Debaryomyces* | *D. hansenii* |
| *Lipomyces* | *L. species* |
| *Pichi* | *P. finlandica*<br>*P. guillermondii*<br>*P. haplophila* |
| *Rhodotorula* | *R. minuta*<br>*R. mucilaginosa* |
| *Sporobolomyces* | *S. roseus*<br>*S. tsugae* |
| *Trichosporon* | *T. cutaneum var cutaneum*<br>*T. ovoides* |
| *Wingea* | *W. robertsiae* |

The yeast strain can be, for example, one selected from Tables 2 and 3 (see below).

Cultivation in bioreactors of wild-type or recombinant yeast strains expressing the polypeptide or fragment thereof having TDE enantioselective epoxide hydrolase activity (with the purpose of preparing yeasts stocks or for the enantioselective preparative methods of the invention) can be carried out under conditions that provide useful biomass and/or enzyme titer yields. Cultivation can be by batch, fed-batch or continuous culture methods. Useful cultivation conditions are dependent on the yeast strain used. General procedures for establishing useful culturing conditions of yeasts, fungi and bacteria in bioreactors are known to those skilled in the art. The mixture of epoxides can be added directly to the culture. The concentration of the TDE enantiomeric mixture can be at least equal to the solubility of the TDE enantiomeric mixture in the aqueous phase of the reaction mixture. The starting amount of epoxide added to the reaction mixture is not critical. The epoxide can be metered out continuously or in batch mode to the reaction mixture. The relative proportions of (R)- and (S)-epoxide in the mixture of enantiomers of the epoxide shown by the general formula (I) is not critical but it is advantageous for commercial purpose to employ a racemic form of the epoxide shown by the general formula (I). The epoxide can be added in a racemic form or as a mixture of enantiomers in different ratios. The amount of the yeast cells, crude yeast cell extract, or partially purified or isolated polypeptide having TDE enantioselective activity added to the reaction depends on the kinetic parameters of the specific reaction and the amount of epoxide that is to be hydrolysed. In the case of product inhibition, it can be advantageous to remove the formed vicinal diol from the reaction mixture or to maintain the concentration of the vicinal diol at levels that allow reasonable reaction rates. Techniques used to enhance enzyme and biomass yields include the identification of useful (or optimal) carbon sources, nitrogen sources, cultivation time, dilution rates (in the case of continuous culture) and feed rates, carbon starvation, addition of trace elements and growth factors to the culture medium, and addition of inducers for example substrates or substrate analogs of the epoxide hydrolases during cultivation. In the case of recombinant hosts, the conditions under which the promoters function for transcription of the gene encoding the polypeptide with epoxide hydrolase activity are taken into account. At the end of fermentation (culture), biomass and culture medium can be separated by methods known to one skilled in the art, such as filtration or centrifugation.

The processes are generally performed under mild conditions. For example, the reaction can be carried out at a pH from 5 to 10, preferably from 6.5 to 9, and most preferably from 7 to 8.5. The temperature for hydrolysis can be from 0 to 70 °C, preferably from 0 to 50 °C, most preferably from 4 to 40 °C. It is also known that lowering of the temperature of the reaction can enhance enantioselectivity of an enzyme.

The reaction mixture can contain mixtures of water with at least one water-miscible solvents (e.g., organic water-miscible solvents). Preferably water-miscible solvents are added to the reaction mixture such that epoxide hydrolase activity remains measurable. Water-miscible solvents are preferably organic solvents and can be, for example, acetone, methanol, ethanol, propanol, isopropanol, acetonitrile, dimethylsulfoxide, *N,N*-dimethylformamide, *N*-methylpyrolidine, and the like.

The reaction mixture can also contain mixtures of water with at least one water-immiscible organic solvent. Examples of water-immiscible solvents that can be used include, for example, toluene, 1,1,2-trichlorotrifluoroethane, methyl *tert*-butyl ether, methyl isobutyl ketone, dibutyl-*o*-phtalate, aliphatic alcohols containing 6 to 9 carbon atoms (for example hexanol, octanol), aliphatic hydrocarbons containing 6 to 16 carbon atoms (for example cyclohexane, *n*-hexane,

*n* -octane, *n* -decane, *n* -dodecane, *n* -tetradecane and *n* - hexadecane or mixtures of the aforementioned hydrocarbons), and the like. Thus, the reaction mixture can include water with at least one water-immiscible organic solvent selected from the group consisting of toluene, 1,1,2-trichlorotrifluoroethane, methyl *tert*-butyl ether, methyl isobutyl ketone, dibutyl-o-phtalate, aliphatic alcohols containing 6 to 9 carbon atoms, and aliphatic hydrocarbons containing 6 to 16 carbon.

The reaction mixture can also contain surfactants (for example Tween 80), cyclodextrins or phase-transfer catalysts and the like that can increase, selectively or otherwise, the solubility of the epoxide enantiomers in the reaction mixture.

The reaction mixture can also contain a buffer. Buffers are known in the art and include, for example, phosphate buffers, citrate buffers, TRIS buffers, HEPES buffers, and the like.

The production of the YEDH polypeptides, including functional fragments, can be, for example, as recited above in the section on Polypeptides and Polypeptide Fragments. Thus they can made by production in a natural host cell, production in a recombinant host cell, or synthetic production. Recombinant production can be carried out in host cells of microbial origin. Preferred yeast host cells are selected from, but are not limited to, the genera *Saccharomyces, Kluyveromyces, Hansenula, Pichia, Yarrowia, Arxula*, and Candida. Preferred bacterial host cells include *Escherichia coli, Agrobacterium* species, *Bacillus* species and *Streptomyces* species. Preferred filamentous fungal host cells are selected from the group consisting of the genera *Aspergillus, Trichoderma* and *Fusarium.* The production of the polypeptide can be, e.g., intra- or extra- cellular production and can be by, e.g., secretion into the culture medium.

In the fermentation reactions, the polypeptides (including functional fragments) can be immobilized on a solid support or free in solution. Procedures for immobilization of the yeast or preparation thereof include, but are not limited to, adsorption; covalent attachment; cross-linked enzyme aggregates; cross-linked enzyme crystals; entrapment in hydrogels; and entrapment into reverse micelles.

The progress of the reaction can be monitored by standard procedures known to one skilled in the art, which include, for example, gas chromatography or high-pressure liquid chromatography on columns containing chiral stationary phases. The vicinal diol formed can be removed from the reaction mixture at one or more stages of the reaction.

The reaction can be stopped when one enantiomer of the epoxide and/or vicinal diol is found to be in excess compared to the other enantiomer of the epoxide and/or vicinal diol. Preferably, the reaction is stopped when one enantiomer of an epoxide of general formula (I) and/or vicinal diol of general formula (II) is found to be in an enantiomeric excess of at least 90%. In a more preferred embodiment of the invention, the reaction is stopped when one enantiomer of an epoxide of general formula (I) and/or vicinal of general formula (II) is found to be in an enantiomeric excess of at least 95%. The reaction can be stopped by the separation (for example centrifugation, membrane filtration, precipitation by solvents, and the like) of the yeast, or preparation thereof, and the reaction mixture or by inactivation (for example by heat treatment or addition of salts and/or organic solvents) of the yeast or polypeptide, or preparation thereof. Thus, the reaction can be stopped by, for example, the separation of the catalytic agent from the reactants and products in the mixture, or by ablation or inhibition of the catalytic activity, by techniques known to one skilled in the art.

The optically active epoxides and/or vicinal diols produced by the reaction can be recovered from the reaction mixture, directly or after removal of the yeast, or preparation thereof. Preferably, the process can include continuously recovering the optically active epoxide and/or vicinal diol produced by the reaction directly from the reaction mixture. Methods of removal of the optically active epoxide and/or vicinal diol produced by the reaction include, for example, extraction with an organic solvent (such as hexane, toluene, diethyl ether, petroleum ether, dichloromethane, chloroform, ethyl acetate and the like), vacuum concentration, crystallisation, distillation, membrane separation, column chromatography and the like.

Thus, the present invention provides an efficient process with economical advantages compared to other chemical and biological methods for the production, in high enantiomeric purity, of optically active epoxides of the general formula (I) and vicinal diols of the general formula (II) in the presence of a yeast strain having epoxide hydrolase activity or a polypeptide that is derived from a yeast strain and has such activity.

The following examples serve to illustrate, not limit, the invention.

## EXAMPLES

### Example 1. Materials and Methods

#### Determination of concentrations and enantiomeric excesses

Quantitative determinations of the compounds and determination of enantiomeric excesses in the reactions described in the Examples below were carried out by gas chromatography. Gas chromatography (GLC) was performed on a Hewlett-Packard 6890 gas chromatograph equipped with FID detector and using $H_2$ as carrier gas. Determination of the enantiomeric excesses for 2-methyl-1,2-epoxyheptane and 2-methyl-1,2-heptanediol was performed by GLC using a fused silica β-DEX 225 cyclodextrin capillary column (Supelco) (30 m length, 25 mm ID and 25 μm film thickness).

The initial temperature of 75°C was maintained for 5.6 minutes, increased at a rate of 8°C per minute to 115°C, and maintained at this temperature for 8.5 minutes. The retention times (min) were as follows: $R_t$ (R)-epoxide = 5.3, $R_t$ (S)-epoxide = 5.6, $R_t$ (S)-diol = 17.9., $R_t$ (R)-diol = 18.4. Determination of the enantiomeric excesses of 2-methyl-3-phenyl-1,2-epoxpropane and 2-methyl-3-phenyl-propanediol was performed by GLC using a fused silica β-DEX 110 cyclodextrin capillary column (Supelco) (30 m length, 25 mm ID and 25 μm film thickness). The initial temperature of 80°C was maintained for 22 minutes, increased at a rate of 4°C per minute to 160°C, and maintained at this temperature for 1 minute. The retention times (min) were as follows: $R_t$ (S)-epoxide = 31.9, $R_t$ (R)-epoxide = 32.1, $R_t$ (S)-diol = 47.7., $R_t$ (R)-diol = 48.0. Concentrations of epoxides and diols were derived from calibration curves obtained from extractions of the epoxide and diol from buffer in the absence of active cells.

**<u>Synthesis of *rac* 2,2-disubstituted epoxides and vicinal diol standards</u>**

*(a) Synthesis of* 2-methyl-1,2-epoxyheptane *and 2-methyl-1,2-heptanediol*

*rac*-2-methyl-1,2-epoxyheptane was synthesised by direct epoxidation of the corresponding alkene with 3-chloroperbenzoic acid in dry $CH_2Cl_2$.: Yield 50%, $^1$H-NMR ($CDCl_3$): δ =0.8 - 1.0 (t, 3H, J=7 Hz, ω-$CH_3$), 1.25 - 1.30 (s, 3H, -$CH_3$), 1.30 - 1.70 (m, 8H, 4 x $CH_2$), 2.57 - 2.65 (dd, 2H, J = 6.8 and 5.5 Hz, -$CH_2$O-). 2-Methyl-1,2-heptanediol was synthesized by acid-catalyzed hydrolysis of (±)2-methyl-1,2-epoxyheptane in THF-$H_2O$ (Pedragosa-Moreau *et al.*, 1996). The crude product was purified by silica-gel chromatography ($CHCl_3$/ETOAc 1:1).

*(b) Synthesis of 2-methyl-3-phenyl-1,2-epoxpropane and 2-methyl-3-phenyl-propanediol*

*(i) Synthesis of rac-2-methyl-3-phenyl-1,2-epoxpropane:* Methallylbenzene (115.86 g, 0.88 mol) was dissolved in methyl alcohol (460 $cm^3$) containing acetonitrile (82 $cm^3$, 1.58 mol) and anhydrous potassium carbonate (24.53 g, 0.18 mol) in a one litre round bottomed flask equipped with a magnetic stirrer bar, condenser and pressure-equalising dropping funnel at room temperature. Hydrogen peroxide (35% by mass, 102.2 $cm^3$, 1.05 mol) was then added in a steady stream over about twenty minutes, causing spontaneous boiling of the mixture within thirty minutes of addition commencing. The reaction was left to stir, gradually reaching room temperature over the course of an hour. GC analysis indicated that there was only 55% conversion at this stage. Further acetonitrile (80 $cm^3$, 1.53 mol) and anhydrous potassium carbonate (24.46 g, 0.18 mol) were added to the mixture, and another portion of hydrogen peroxide (35% by mass, 90 $cm^3$, 0.93 mol) was added as before. Conversion had improved to 70-80% typically by this stage. The mixture was placed in a separating funnel, diluted with 400 $cm^3$ of ethyl acetate and the organic component isolated. The aqueous phase was washed with a further 200 $cm^3$ portion of ethyl acetate. The combined organic phases were then concentrated to a yellow heterogeneous suspension, dissolved in 200 $cm^3$ of ethyl acetate, washed twice with 50 $cm^3$ saturated aqueous potassium carbonate, and the isolated organic phase dried with magnesium sulphate and concentrated to a clear yellow oil. Purification by distillation *in vacuo* afforded *rac*-2-methyl-3-phenyl-1,2-epoxypropane (92.88 g, 71%) as a pale yellow oil (bp 73-78 C / 6 mmHg, 95% purity by GC area %). H (200 MHz, CDCl3) 1.33 (3H, br s), 2.65 (1 H, d, *J* 5.0), 2.70 (1 H, dd, *J* 4.8 and 0.6), 2.86 (1 H, d, *J* 14.2), 2.95 (1 H, d, *J* 14.4) and 7.22-7.40 (5H, m).

*(ii) Synthesis of methallylbenzene*: An oven-dried apparatus consisting of a three-necked round bottomed flask equipped with a magnetic stirrer bar, two glass stoppers, a 200 $cm^3$ pressure-equalising dropping funnel and a double-sided spiral condenser was cooled to room temperature under nitrogen atmosphere. The flask was charged with magnesium turnings (15.49 g, 0.64 mol) and anhydrous tetrahydrofuran (75 $cm^3$), and cooled to 0 °C in and ice-water bath. Bromobenzene (8.50 $cm^3$, 80.72 mmol) was then carefully layered over the magnesium turnings, and the magnesium scratched with a glass rod to initiate the reaction. An orange patina formed on the scratched surface, along with the gradual evolution of gas bubbles. Stirring was carefully initiated and the cooling bath removed, although external cooling was often re-applied and removed to moderate the course of this exothermic reaction. After ten minutes, additional bromobenzene (58.57 $cm^3$, 80.72 mmol) in anhydrous tetrahydrofuran (225 $cm^3$) was added dropwise to the magnesium mixture over an hour with intermittent cooling. After a further hour, by which time the reaction was at room temperature, methallylchloride (59.90 $cm^3$, 0.61 mol) in anhydrous tetrahydrofuran (100 $cm^3$) was added dropwise to the solution over an hour. A delayed exotherm occurred about twenty minutes after addition began, that could result in reflux of the mixture if left unchecked. This was controlled with cooling to about 15 °C during addition. The reaction was allowed to reach room temperature over two hours, affording a dense white suspension. Addition of 200 $cm^3$ of distilled water and mixing for 30 minutes, followed by phase separation, isolation of the organic phase, drying over magnesium sulphate and concentration *in vacuo* gave a light brown oil. The

procedure was repeated concurrently in a second experiment, and the combined organic residues distilled. After a brief forerun, methallylbenzene (125.05 g, 78%) was isolated as a clear oil (bp 61-62 C / 10 mmHg, 95% purity by GC area %). H (200 MHz, CDCl3) 1.72-1.76 (3H, m), 3.39 (2H, br s), 4.78-4.83 (1 H, m), 4.84-4.90 (1 H, m) and 7.21-7.40 (5H, m).

*(iii) Synthesis of rac*-2-methyl-3-phenyl-1,2-propanediol: A solution of methallylbenzene (20.01 g, 0.15 mol) and *N*-methylmorpholine-*N*-oxide (23.30 g, 0.20 mol) in 7:3 (v/v) acetone:water solution (852 $cm^3$) in a three-necked round bottomed flask was treated with osmium tetroxide (14.1 mg, 55.4 mol), stoppered and stirred at room temperature for seven days. The reaction's progress was monitored by gas chromatography. After this time, all the acetone was stripped off *in vacuo*, and the aqueous phase was extracted with ethyl acetate (2 x 200 $cm^3$). The combined organic phases were dried with magnesium sulphate and concentrated to a brown oil. This was passed through a plug of silica with ethyl acetate as the mobile phase, affording a yellow oil. This was recrystallised from 2% (v/v) ethyl acetate:hexane to yield 2-methyl-3-phenyl-1,2-propanediol (13.53 g, 54%) as colourless needles. H (200 MHz, CDCl3) 1.17 (3H, s), 2.14 (2H, s), 2.80 (1H, d, *J* 13.2), 2.89 (1H, d, *J* 13.2), 3.45 (1 H, d, *J* 11.0), 3.53 (1 H, d, *J* 11.0) and 7.20-7.39 (5H, m).

**<u>Preparation of frozen yeast cells for screening</u>**

Yeast cells were grown at 30°C in 1 L. shake-flask cultures containing 200 ml yeast extract/malt extract (YM) mixture (3% yeast extract, 2% malt extract, 1% peptone w/v) supplemented with 1% glucose (w/v). At late stationary phase (48 - 72 h), the cells were harvested by centrifugation (10 000 g, 10 min, 4°C), washed with phosphate buffer (50 mM, pH7.5), pelleted by centrifugation, and frozen in phosphate buffer containing glycerol (20%) at -20°C as 20% (w/v) cell suspensions. The cells were stored for several months without significant loss of activity.

**<u>Yeast isolate (strain) screening</u>**

Epoxide (10 μl of a 1M stock solution in EtOH) was added to a final concentration of 20 mM to 500 μl cell suspension (20% w/v) in phosphate buffer (50 mM, pH 7.5). The reaction mixtures were incubated at 30°C for 2 hours or 5 hours. The reaction mixtures were extracted with EtOAc (300 μl) and centrifuged. Vicinal diol formation was evaluated by TLC (silica gel Merck 60 $F_{254}$). Compounds were visualized by spraying with vanillin/conc. $H_2SO_4$ (5g/l). Reaction mixtures that showed substantial diol formation were evaluated for asymmetric hydrolysis of the epoxide by chiral GLC analysis. Some reactions were repeated over longer or shorter times and with more dilate cell suspensions (10%w/v) in order to analyse the reactions at suitable conversions.

**<u>General procedure for the hydrolysis of 2,2-disubstituted epoxides</u>**

Frozen yeast cells were thawed, washed with phosphate buffer (50 mM, pH 7.5) and resuspended in buffer. Cell suspensions (10 ml, 20% or 50% w/v) were placed in 20 ml glass bottles with screw caps fitted with septa. The substrate (100 or 250 μl of a 2M (v/v) stock solution in ethanol) was added to final concentrations of 20 mM or 50 mM. The mixtures were agitated on a shaking water bath at 30°C. The course of the bioconversions of epoxides was followed by withdrawing samples (500 μl) at appropriate time intervals. Samples were extracted with 300 μl EtOAc. After centrifugation (3000 x g, 2 min), the organic layer was dried over anhydrous $MgSO_4$ and the products analyzed by chiral GLC.

**<u>Determination of the absolute configuration of residual 2,2-disubstituted epoxides and residual diols</u>**

**(a) 2-methyl-1,2-epoxyheptane *and 2-methyl-1,2-heptanediol***

Absolute configurations were deduced from reported elution orders of the epoxide and diol enantiomers on cyclodextrin columns (Mischitz *et al.*, 1995).

***(b) 2-methyl-3-phenyl-1,2-epoxpropane and 2-methyl-3-phenyl-propanediol***

Absolute configurations were deduced from reported elution orders of the epoxide and diol enantiomers on cyclodextrin columns and verified by comparison of the optical rotation of the residual epoxide formed during the biohydrolysis of 2-methyl-3-phenyl-1,2-epoxpropane by yeast strain *Cryptococcus hungaricus* UOFS Y-1346. (S)-2-methyl-3-phenyl-1,2-epoxpropane: $[\alpha]^{22}D$ + 5.0 (c 0.25, MeOH) (Orru *et al.*, 1998). Optical rotation was measured on a Perkin-Elmer 341 polarimeter at 589 nm (Na line) in a 1 dm cuvette.

**Cultivation of *Cryptococcus hungraricus* (UOFS Y-1346) in a 10L fed-batch bioreactor**

Cultivation was performed at 25 °C in 15 L Braun Biostat C bioreactors (working volume 10 L). An overpressure of 500 mbar was applied to the reactor. Dissolved oxygen was continuously monitored and maintained at 30% saturation by adjustment of the stirrer speed. Airflow rate was controlled at 5.5 $L.min^{-1}$ by use of a mass flow meter. pH was automatically maintained at 5.5 $\pm$ 0.05 by the addition of 25 % (w/v) $NH_4OH$. Excessive foam formation was avoided by the addition of antifoam (Pluriol P 2000).

A Fernbach shake flask inoculum (10 %, v/v) of *C. hungaricus* (UOFS Y-1346) was transferred into a 15 l Braun Biostat C bioreactor containing 6 l mixture with the following composition (per litre): citric acid, 2.5 g; yeast extract, 7 g; $(NH_4)_2SO_4$, 58 g; $KH_2PO_4$, 11.3 g; $MgSO_4.7H_2O$, 8.2 g; $CaCl_2.2H_2O$, 0.88 g; NaCl, 0.1 g; $H_3PO4$, 13.4; vitamin solution, 1.7 ml; trace element solution, 1 ml; antifoam (Pluriol P-2000), 0.5 ml; and glucose, 20 g. Glucose (60 % m/m) was fed to maintain a residual glucose concentration of 5 g/l after the batch phase. Sugar feed was stopped when the sugar uptake rate decreased. Cultivation was continued for 12 hours after the residual glucose concentration was zero. The biomass was harvested by centrifugation and frozen at -20 °C in phosphate buffer pH 7.5 containing 20 % glycerol until use.

**Yeast strains**

Yeast strains with screen numbers donated "AB" or "Car" or "Alf" or "Poh" were isolated from soil from specialised ecological niches that were selected based on the inventors' belief that selectivity for specific classes of epoxides in microorganisms can be determined by environmental factors such as terpene-rich environments or highly contaminated soil. "AB" and "Alf" strains were isolated from Cape Mountain fynbos, an ecological environment unique to South Africa, "Car" strains were isolated from soil under pine trees, and "Poh" strains from soil contaminated by high concentrations of cyanide. It seemed likely that microorganisms existing in these contaminated soils would have developed alternative respiratory mechanisms. Nearly all these new isolates displayed activity and selectivity for 2,2-disubstituted epoxides, while only a few of the more than 200 strains from the Yeast Culture Collection that were included in the screening, displayed activity and selectivity for 2,2-disubstituted epoxides. Strains belonging to the species *Cryptococcus curvatus, Cryptococcus podzolicus*, and *Cryptococcus laurentii* were all obtained from the environment (as described above) and corresponding strains were not either not available from the Yeast Culture Collection or did not display the desired selectivity. Furthermore, the strains from the culture collection that were found to be able to produce optically active epoxides and/or diols from 2,2-disubstituted epoxides belonged in most cases to the same species as those isolated from soil from the selected environments. All new isolates that produced optically active epoxides or vicinal diols during hydrolysis of 2,2-disubstituted epoxides were identified by known biochemical characterisation methods, and most of the isolates were also subjected to molecular identification by sequence analysis of the D1/D2 region of the large subunit rDNA. These new isolates were deposited at the Yeast Culture Collection of the University of the Orange Free State (UOFS) and assigned UOFS numbers. Some of the isolates were also deposited at the National Collection of Yeast Cultures (NCYC), Norwich, United Kingdom.

**Example II. Cloning and overexpression of wild type yeast epoxide hydrolases in *Yarrowia lipolytica* as production host under the control of different promoters**

**1. Vectors, Strains and Primers (Table 1)**

The following features are common to all the *E. coli*/*Y. lipolytica* auto-cloning integrative vectors used:

- LIP2 terminator
- Zeta regions
- Kanamycin resistance for *E. coli* selection
- mono-copy auto cloning vectors (pINA 1311, pINA 1313, pINA 3313) with a fully functional selection marker gene carry the fully functional *ura3d1* allele from the *URA3* selection marker gene
- multi-copy auto cloning vectors (pINA 1291, pINA 1293, pINA 3293) with a defective selection marker gene (copy number amplification) carry the defective *ura3d4* allele from the *URA3* selection marker gene

**Table 1.** Vectors, strains and primers

| | Description | | | Cloning sites | |
|---|---|---|---|---|---|
| Vectors | Promoter | Selection marker | Targeting sequence | Upstream/down stream | Reference/ Origin |

(continued)

| | Description | | | Cloning sites | |
|---|---|---|---|---|---|
| pINA1291 (PYLHmA) | hp4d | ura3d4 | none | *Pml*I (blunt) / *Bam*HI, *Kpn*I, *Avr*II | Nicaud *et al* (2002) |
| pYL3313 (1313) (pYLTsA) | TEF | ura3d1 | none | *Xmn*I (in pro) / *Bam*HI, *Kpn*I, *Avr*II | This study |
| **Host Strain** | **Description** | | | | **Reference/ Origin** |
| *Yarrowia lipolytica* Polh | *MATA, ura3-302, uxpr2-322, axp1-2* (deleted for both extracellular proteases and growth on sucrose) | | | | CLIB882 |
| **Primers** | **Sequence** | | | **Specifications** | |
| YL-fwd | 5'-GGA GTT CTT CGC CCA C-3' (SEQ ID NO:15) | | | amplification of expression casette between *Not*I sites | |
| YL-rev | 5'-GAT CCC CAC CGG AAT TG-3' (SEQ ID NO:16) | | | | |
| pINA-1 | 5'- CAT ACA ACC ACA CAC ATC CA-3' (SEQ ID NO:17) | | | PYLHmA fwd primer | |
| pINA-2 | 5'-TAA ATA GCT TAG ATA CCA CAG-3' (SEQ ID NO:18) | | | pYLTsA/pYLHmA rev primer | |
| Pina-3 | 5'-CTC TCT CTC CTT GTC AAC T-3' (SEQ ID NO:19) | | | pYLTsA fwd primer | |

**2. Transformants (multi-copy and single-copy)**

| YL Transformants | Gene origin |
|---|---|
| **TEF promoter** | **Vector: YL3313 (1313) (pYLTsA)** |
| YL 25 TsA | *Rhodotorula araucariae* (NCYC 3183) |
| YL 46 TsA | *Rhodosporidium toruloides* (UOFS Y-0471) |
| YL 692 TsA | *Rhodosporidium paludigenum* (NCYC 3179) |
| YL 1 TsA | *Rhodosporidium toruloides* (NCYC 3181) |
| YL Car 54 TsA | *Cryptococcus curvatus* (NCYC 3158) |
| **hp4d promoter** | Vector: pINA1291=(pYLHmA) |
| YL 25 HmA | *Rhodotorula araucariae* (NCYC 3183) |
| YL 46 HmA | *Rhodosporidium toruloides* (UOFS Y-0471) |
| YL 692 HmA | *Rhodosporidium paludigenum* (NCYC 3179) |

**3. Vector preparation**

pINA1291 (Fig. 20) was obtained from Dr Madzak of Labo de Génétique, INRA, CNRS. This vector was renamed pYLHmA (***Y**arrowia **L**ipolytica* expression vector, with **H**p4d promoter, **m**ulti-copy integration selection, **A**bsent secretion signal).

pKOV93 (Fig. 21) was prepared by the inventors. This vector was renamed pYLTsA (***Y**arrowia **L**ipolytica* expression vector, with **T**EF promoter, single-copy integration selection, **A**bsent secretion signal).

To prepare the vectors for ligation with an epoxide hydrolase coding sequence (or other insert to be expressed in *Y. lipolytica*), DNA was digested with *Bam*HI and *Avr*II.

**4. Insert preparation**

Total RNA was isolated from selected yeast strain cells and messenger RNA (mRNA) was purified from it. The mRNA was used as a template to synthesise complementary DNA (cDNA) using reverse transcriptase. The cDNA was then used as a template for Polymerase Chain Reaction (PCR) using appropriate primers. PCR primers were selected by repeated experimentation using multiple test primers for each yeast strain, the sequences of which were based on previously described epoxide hydrolase sequences from a variety of species. The nucleotide sequences of the forward and reverse primers used to generate cDNA coding sequences from mRNA from six different yeast strains with appropriate restriction enzyme recognition sites at their termini are shown below. Restriction enzyme recognition sequences are underlined and the relevant restriction enzymes are shown in parentheses.

| Yeast Strain | Forward primer | Reverse primer |
|---|---|---|
| *Rhodosporidium toruloides* NCYC 3181 #1<br>*Rhodosporidium toruloides* UOFS Y-0471 #46<br>*Cryptococcus curvatus* NCYC 3158 Car054 | GTGGATCCATGGCGACACACA (SEQ ID NO:20)<br>(*Bam*HI) | GACCTAGGCTACTTCTCCCA CA (SEQ ID NO:21)<br>(*Bln*I) |
| *Rhodotorula araucariae* NCYC 3183 #25 | GATTAATGATCAATGAGCGAGCA (SEQ ID NO:22) (*Bcl*I) | GACCTAGGTCACGACGACA G (SEQ ID NO:23)<br>(*Bln*I) |
| *Rhodosporidium paludigenum* NCYC 3179 #692 | GTGGATCCATGGCTGCCCA (SEQ ID NO :24)<br>(*Bam*HI) | GAGCTAGCTCAGGCCTGG (SEQ ID NO:25)<br>(*Nhe*I) |
| *Debaromyces hansenii NCYC 3167* (#113) | GTGGATCCATGATGCAAGG(SEQ ID NO:26)<br>(*Bam*HI) | GACCTAGGCTAAGGATATT (SEQ ID NO:27)<br>*(Bln*I) |

Each PCR reaction contained 200 μM dNTPs, 250 nM of each primer, 2 mM of $MgCl_2$, cDNA and 2.5 U of Taq polymerase in a 50 μl reaction volume. The PCR profile used was: 95°C for 5 minutes, followed by 30 cycles of: 95°C - 1 min, 50°C - 1 min, 72°C - 2 min, then a final extension of 72°C for 10 minutes. The PCR product were purified and digested with the restriction enzymes whose recognition sites are engineered at the end of the primers. The cDNA fragment was cloned into a vector and sequenced for confirmation.

Coding seqences to be inserted in either pYLHmA or pYLTsA were prepared with *Bam*HI and *Avr*II at their termini. The above PCR primers were designed with these restriction sites, unless the sites were also present in the gene to be inserted. If this occurred, appropriate compatible restriction enzymes were selected. PCR template DNA was either the insert cloned into a different vector, or cDNA synthesized from the original host organism. PCR reactions consisted of 200 μM dNTP's, 250 nM each primer, 1X Taq polymerase buffer, and 2.5 units Taq polymerase per 100 μl reaction. The amplification programme used was: 95°C for 5 minutes, followed by 30 cycles of 95°C for 1 minute, 50°C for 1 minute, followed by a final extension at 72°C for 2 minutes, followed by 72°C for 10 minutes.

PCR products were purified and digested with the relevant restriction enzymes. The digested DNA was subsequently repurified and was ready for ligation into the prepared vector.

**5. Preparation of pYLHmA or pYLTsA constructs**

Vector and insert were ligated at pmol end ratios of 3:1 - 10:1 (insert:vector), using commercial T4 DNA Ligase. Ligations were electroporated into any laboratory strain of *Escherichia coli*, using the Bio-Rad GenePulser, or equivalent electroporator. Transformants were selected on LM media (10 g/l yeast extract, 10 g/l tryptone, 5 g/l NaCl), supplemented with kanamycin (50 μg/ml). Transformants were selected based on restriction enzyme digests of purified plasmid DNA.

### 6. *Yarrowia lipolytica* transformation

#### 6.1.1: Preparation of DNA - method 1

Digestion of the piNA-series of plasmids with *Not*I resulted in the release of a bacterial DNA-free expression cassette, containing the ura3d4 (pYLHmA) or the ura3d1 (pYLTsA) marker gene and the promoter-gene-terminator.

Scaled-up quantities of each plasmid were isolated. *Not*I was used to restrict the plasmid DNA, and the digested DNA was run on an agarose gel. *Not*I digests resulted in generation of the bacterial fragment of the plasmid as a band at 2210 bp, and the expression cassette as a band of 2760 bp + size of insert (pYLHmA) or 2596 bp + size of insert (pYLTsA). The expression cassette fragments were excised from the gel and purified from the agarose. The purified fragment was used for transformation of *Y. lipolytica* Po1h.

#### 6.1.2. Preparation of DNA - method 2

Primers YL-Fwd and YL-Rev (Table 1) were used to amplify the expression cassette. PCR reactions consisted of 200 μM dNTP's, 250 pmol each primer, 1X Taq polymerase buffer and 2.5 units Taq polymerase per 100 μl reaction. The amplification programme used was 95°C for 5 minutes, 30 cycles of 95°C for 1 minute, 50°C for 1 minute, and 72°C for 3 ½ minutes, followed by a final extension at 72°C for 10 minutes. The PCR product was purified from the PCR reaction mix and used for transformation of *Y. lipolytica* Po1h.

#### 6.1.3. Preparation of carrier DNA

DNA from salmon testes was made up as a 10 mg/ml stock in TE (10 mM Tris-HCl, pH 8.0, 1 mM EDTA) and sonicated to result in fragments that ranged from approximately 15 kb to 100 bp, with most fragments in a range of 6 to 10 kb. The DNA was denatured by boiling. Aliquots were stored at -20°C.

#### 6.1.4. Transformation of *Yarrowia lipolytica* with pYLHmA or PYLTsA

An adaptation of the method of Xuan *et al* (1988) was used for the transformation of *Y. lipolytica* Po1h. The yeast was inoculated into 50 ml YPD (10 g/l yeast extract, 20 g/l peptone, 20 g/l glucose). The culture was incubated at 30°C, 220 rpm until cell densities of 8 x $10^7$ - 2 x $10^8$ cells/ml were reached. The entire culture was harvested, the pellet resuspended in 10 ml TE and reharvested. 1 ml TE + 0.1 M LiOAc was used to resuspend the pellet and the culture was incubated at 28°C in a ProBlot Jr (Labnet) hybridisation oven, set at 4 rpm (or similar incubator) for 1 hour. Transformation mixes were set up with 0.5 - 2 μg of transforming DNA + 5 μg of carrier DNA with 100 μl of treated cells.

Each mix was set up in a 1.5 ml microfuge tube, and incubated in a 28°C heating block for 30 minutes. 7 volumes of PEG reagent (40% PEG 4,000, 0.1 M LiOAc, 10 mM Tris, 1 mM EDTA, pH 7.5, filter-sterilised) were added to each, mixed carefully and incubated at 28°C for a further 1 hour. The tubes were transferred to a 37°C heating block for 15 minutes, and then the cells were pelleted by centrifugation for 1 minute at 13,000 rpm. The cell pellets were carefully resuspended in 100 μl $dH_2O$ The transformations were plated on *Y. lipolytica* selective plates (17 g/l Difco yeast nitrogen base without amino acids and without $(NH_4)_2SO_4$, 20 g/l glucose, 4 g/l $NH_4Cl$, 2 g/l casamino acids, 300 mg/l leucine) and incubated at 28°C.

Colonies which appeared on the selective plates after 3 - 7 days were transferred onto fresh plates and regrown.

#### 6.1.5. Confirmation of integration of pYLHmA or pYLTsA

Colonies that grew on the newly-streaked selective plates were inoculated into 5 ml of YPD medium and grown at 30°C, 200 rpm for 24-48 hours. A small-scale genomic DNA isolation was performed.

PCR was performed using this genomic DNA as template, with either pINA-1 and pINA-2 as primers (transformants with pYLHmA), or pINA-3 and pINA-2 (pYLTsA) (Table 1). Each PCR reaction contained 200 μM dNTPs, 250 nM of each primer, 2 mM of $MgCl_2$, genomic DNA and 2.5 U/50μl of Taq polymerase. The PCR profile was as described above in this example. These primer sets gave products the size of the inserted genes.

### Example III. Selection of yeasts able to produce optically active 2-ethyl-1,2-epoxyheptane and 2-methyl-2-heptanediol from (±)-2-methyl-1,2-epoxyheptane

Yeasts were cultivated, harvested and frozen as described above. 2,2-disubstituted epoxide 2-methyl-1,2-epoxyheptane was added and the screening was performed as described above. Strains with the highest activities as judged by TLC from diol formation were subjected to chiral GC analysis as described above. The strains with E-values

> 2 are depicted as Samples 1-114 (Table 2). E-values were calculated from the followig formulas:

$$E = \frac{\ln[(1-\xi)(1-ee_s)]}{\ln[(1-\xi)(1+ee_s)]} \quad \text{where} \quad \begin{array}{l}\xi = \text{degree of conversion}\\ ee_s = \text{substrate enantiomeric excess}\end{array} \qquad (1)$$

$$E = \frac{\ln[1-\xi(1+ee_p)]}{\ln[1-\xi(1-ee_p)]} \quad \text{where} \quad \begin{array}{l}\xi = \text{degree of conversion}\\ ee_p = \text{product enantiomeric excess}\end{array} \qquad (2)$$

$$E = \frac{\ln\left[\frac{ee_p(1-ee_s)}{(eep+ees)}\right]}{\ln\left[\frac{ee_p(1+ee_s)}{(eep+ees)}\right]} \quad \text{where} \quad \begin{array}{l}ee_s = \text{substrate enantiomeric excess}\\ ee_p = \text{product enantiomeric excess}\end{array} \qquad (3)$$

Enantiomeric excess (ee) (%) = {([R]-[S]) / ([R]+[S])} x 100%, where [R] and [S] represent the concentrations of the R and S enantiomers of the TDE substrate or the DVD product, depending on whether the ee of the substrate or the product is to be calculated.

Notes:

The conversion at appropriate time intervals and ee's of the substrate and product was used to determine E-values calculated from $ee_s$ and conversion (Equation 1), $ee_p$ and conversion, (Equation 2), and $ee_s$ and $ee_p$ (Equation 3) respectively for strains (114) that displayed the most promising enantioselectivities (Table 2). If the degree of conversion can be accurately determined, E-values calculated from all three equations should be the same, provided that the reaction is irreversible and regiospecific (Pedragosa- Moreau *et al.* 1996). In this case, both the epoxide and diol could be extracted very efficiently, and the degree of conversion could be determined with high accuracy. If the reaction is not regioselective, E-values are not applicable, since E-values calculated from $ee_s$ and conversion and from eep and conversion will differ substantially and highly erroneous results will be obtained. If each of the two antipodes of the epoxide is attacked following a different regioselectivity, the ee of the diol will be high, and E-values based on $ee_p$ and conversion will be higher than those obtained form $ee_s$ and conversion and from $ee_s$ and eep. Strains displaying this type of regioselectivity can then be used for the synthesis of diol in high enantiomeric purity, which is difficult to obtain in high yields from enantioselective but regiospecific reactions. On the other hand, if a catalyst is enantioselective but not regioselective, the ee of the remaining epoxide will be high while the ee of the diol formed will be low, and E-values based on $ee_s$ and conversion will be higher than those obtained from $ee_p$ and conversion and from $ee_s$ and eep. Strains displaying this type of regioselectivity can then be used for the synthesis of epoxide in high enantiomeric purity. Comparison of E-values calculated from the different equations can thus be used as a qualitative tool to analyse the enantioselectivity and regioselectivity of the reaction and to make appropriate choices of biocatalysts for the synthesis of the desired epoxide or diol enantiomers in high yields and optical purity.

**Table 2.** Yeast strains that hydrolyse 2-methyl-1,2-epoxyheptane enantioselectively (E > 2)[a]

| Samp no. | Screen no | Yeast species | Culture Collection Nr | Reaction | | Remaining Epoxide | | Formed Diol | | Selectivity | | |
|---|---|---|---|---|---|---|---|---|---|---|---|---|
| | | | | Time (min) | Conv (%) | E.e. (%) | Abs conf | E.e. (%) | Abs Conf | $E^b$ | $E^c$ | $E^d$ |
| 1 | 269 | *Arxula adeninivonans* | NCYC 3147 | 90 | 3.2 | 0.7 | S | 82.5 | R | 1.6 | 10.7 | 10.5 |
| 2 | 693 | *Arxula terrestris* | NCYC 3148 | 180 | 17.0 | 13.3 | R | 58.0 | S | 5.4 | 4.2 | 4.3 |
| 3 | 43 | *Bullera dendrophila* | NCYC 3152 | 180 | 37.5 | 46.7 | S | 82.6 | R | 12.7 | 17.1 | 16.6 |
| 4 | 669 | *Candida magnoliae* | NCYC 3154 | 180 | 17.9 | 11.7 | R | 43.6 | S | 3.7 | 2.8 | 2.9 |
| 5 | 677 | *Candida magnoliae* | UOFS Y-0799 | 180 | 28.8 | 17.4 | R | 37.1 | S | 3.0 | 2.5 | 2.6 |
| 6 | 751 | *Candida magnoliae* | UOFS Y-1040 | 60 | 18.6 | 14.4 | R | 41.3 | S | 5.0 | 2.6 | 2.8 |
| 7 | 678 | *Candida magnoliae* | UOFS Y-1297 | 180 | 47.4 | 28.2 | R | 31.6 | S | 2.5 | 2.5 | 2.5 |
| 8 | 230 | *Cryptococcus albidus* | NCYC 3156 | 180 | 14.7 | 9.3 | S | 68.7 | R | 3.7 | 6.0 | 5.9 |
| 9 | Jen 17 | *Cryptococcus albidus* | UOFS Y-0223 | 90 | 5.2 | 3.9 | S | 74.5 | R | 6.2 | 7.1 | 7.1 |
| 10 | Jen 03 | *Cyptococcus albidus* | UOFS Y-0821 | 90 | 4.7 | 2.9 | S | 30.5 | R | 4.0 | 1.9 | 1.9 |
| 11 | 375 | *Cryptococcus amylolentus* | NCYC 3157 | 90 | 31.9 | 43.5 | R | 91.8 | S | 42.0 | 35.8 | 36.0 |
| 12 | Car 054 | *Cryptococcus curvatus* | NCYC 3158 | 10 | 46.9 | 54.8 | S | 48.2 | S | 7.3 | 4.3 | 4.8 |
| 13 | 379 | *Cryptococcus hungaricus* | NCYC 3159 | 90 | 33.7 | 32.1 | S | 55.7 | R | 6.0 | 4.6 | 4.8 |
| 14 | Jen 12 | *Cryptococcus laurentii* | NCYC 3160 | 90 | 2.6 | 0.8 | R | 65.3 | S | 1.9 | 4.9 | 4.8 |
| 15 | AB 24 | *Cryptococcus laurentii* | NCYC 3161 | 180 | 71.6 | 48.0 | R | 26.4 | S | 2.2 | 3.1 | 2.6 |
| 16 | Alf 12 | *Cryptococcus laurentii* | UOFS Y-0509 | 180 | 34.9 | 22.0 | R | 37.3 | S | 0.0 | 2.6 | 2.7 |
| 17 | Alf 03 | *Cryptococcus laurentii* | UOFS Y-0514 | 180 | 56.8 | 38.3 | R | 33.7 | S | 2.6 | 3.0 | 2.9 |

(continued)

| Samp no. | Screen no | Yeast species | Culture Collection Nr | Reaction | | Remaining Epoxide | | Formed Diol | | Selectivity | | |
|---|---|---|---|---|---|---|---|---|---|---|---|---|
| | | | | Time (min) | Conv (%) | E.e. (%) | Abs conf | E.e. (%) | Abs Conf | E[b] | E[c] | E[d] |
| 18 | AB 25 | *Cryptococcus laurentii* | UOFS Y-1884 | 180 | 62.1 | 43.3 | R | 30.3 | S | 2.5 | 3.0 | 3.0 |
| 19 | AB 26 | *Cryptococcus laurentii* | UOFS Y-1885 | 180 | 69.0 | 51.9 | R | 27.2 | S | 2.5 | 3.0 | 2.8 |
| 20 | AB 27 | *Cryptococcus laurentii* | UOFS Y-1886 | 180 | 42.7 | 26.4. | R | 37.6 | S | 2.7 | 2.9 | 2.8 |
| 21 | AB 32 | *Cryptococcus laurentii* | UOFS Y-1887 | 180 | 59.7 | 41.9 | R | 30.9 | S | 2.6 | 2.9 | 2.8 |
| 22 | AB 33 | *Cryptococcus laurentii* | UOFS Y-1888 | 180 | 53.6 | 36.9 | R | 33.9 | S | 2.7 | 2.9 | 2.8 |
| 23 | AB 35 | *Cryptococcus laurentii* | UOFS Y-1892 | 180 | 44.8 | 35.2 | R | 46.4 | S | 3.5 | 3.9 | 3.8 |
| 24 | Jen 09 | *Cryptococcus laurentii var. laurentii* | UOFS Y-1349 | 90 | 1.2 | 0.5 | R | 50.6 | S | 2.4 | 3.1 | 3.1 |
| 25 | AB 58 | *Cryptococcus podzolicus* | NCYC 3164 | 180 | 38.7 | 45.4 | S | 94.4 | R | 9.4 | 64.2 | 54.7 |
| 26 | AB 53 | *Cryptococcus podzolicus* | NCYC 3165 | 180 | 10.1 | 6.7 | S | 84.4 | R | 4.3 | 12.9 | 12.6 |
| 27 | AB 40 | *Cryptococcus podzolicus* | UOFS Y-1881 | 180 | 27.7 | 33.4 | S | 93.9 | R | 20.3 | 45.3 | 44.1 |
| 28 | AB 49 | *Cryptococcus podzolicus* | UOFS Y-1882 | 180 | 43.0 | 52.2 | S | 94.3 | R | 9.1 | 72.2 | 57.0 |
| 29 | AB 50 | *Cryptococcus podzolicus* | UOFS Y-1883 | 180 | 50.0 | 85.5 | S | 92.7 | R | 34.8 | 88.5 | 72.1 |
| 30 | AB 28 | *Cryptococcus podzolicus* | UOFS Y-1889 | 180 | 34.7 | 49.7 | S | 93.7 | R | 49.2 | 50.6 | 50.5 |
| 31 | AB 34 | *Cryptococcus podzolicus* | UOFS Y-1890 | 180 | 42.6 | 66.7 | S | 90.1 | R | 37.6 | 38.4 | 38.4 |

(continued)

| Samp no. | Screen no | Yeast species | Culture Collection Nr | Reaction | | Remaining Epoxide | | Formed Diol | | Selectivity | | |
|---|---|---|---|---|---|---|---|---|---|---|---|---|
| | | | | Time (min) | Conv (%) | E.e. (%) | Abs conf | E.e. (%) | Abs Conf | $E^b$ | $E^c$ | $E^d$ |
| 32 | AB 36 | *Cryptococcus podzolicus* | UOFS Y-1893 | 180 | 42.2 | 58.4 | S | 93.7 | R | 16.1 | 62.6 | 55.1 |
| 33 | AB 52 | *Cryptococcus podzolicus* | UOFS Y-1895 | 180 | 39.3 | 41.4 | S | 94.4 | R | 6.8 | 64.7 | 52.0 |
| 34 | AB 37 | *Cryptococcus podzolicus* | UOFS Y-1896 | 180 | 52.0 | 70.2 | S | 89.7 | R | 9.6 | 79.1 | 38.6 |
| 35 | AB 29 | *Cryptococcus podzolicus* | UOFS Y-1897 | 180 | 38.9 | 57.3 | S | 94.0 | R | 34.6 | 59.9 | 58.2 |
| 36 | AB 41 | *Cryptococcus podzolicus* | UOFS Y-1899 | 180 | 25.3 | 27.3 | S | 93.5 | R | 12.1 | 40.7 | 39.0 |
| 37 | AB 30 | *Cryptococcus podzolicus* | UOFS Y-1904 | 180 | 43.2 | 70.9 | S | 93.9 | R | 61.9 | 68.1 | 67.7 |
| 38 | AB 45 | *Cryptococcus podzolicus* | UOFS Y-1906 | 180 | 42.6 | 45.9 | S | 92.6 | R | 6.6 | 53.6 | 40.9 |
| 39 | AB 46 | *Cryptococcus podzolicus* | UOFS Y-1907 | 180 | 27.8 | 22.5 | S | 90.4 | R | 4.7 | 27.8 | 24.6 |
| 40 | AB 47 | *Cryptococcus podzolicus* | UOFS Y-1908 | 180 | 23.2 | 19.5 | S | 89.9 | R | 5.6 | 24.4 | 22.6 |
| 41 | AB 48 . | *Cryptococcus podzolicus* | UOFS Y-1910 | 180 | 28.1 | 24.4 | S | 88.7 | R | 7.7 | 22.0 | 19.7 |
| 42 | AB 39 | *Cryptococcus podzolicus* | UOFS Y-1912 | 180 | 50.4 | 70.7 | S | 92.4 | R | 11.6 | 90.1 | 53.7 |
| 43 | AB 56 | *Cryptococcus podzolicus* | UOFS Y-1913 | 180 | 42.7 | 36.5 | S | 92.4 | R | 4.1 | 52.5 | 36.3 |
| 44 | AB 57 | *Cryptococcus podzolicus* | UOFS Y-1914 | 180 | 46.6 | 57.6 | S | 92.8 | R | 8.6 | 66.7 | 47.8 |
| 45 | 520 | *Pichia finlandica* | NCYC 3173 | 180 | 70.6 | 79.6 | R | 30.8 | S | 4.4 | 3.8 | 4.1 |

(continued)

| Samp no. | Screen no | Yeast species | Culture Collection Nr | Reaction | | Remaining Epoxide | | Formed Diol | | Selectivity | | |
|---|---|---|---|---|---|---|---|---|---|---|---|---|
| | | | | Time (min) | Conv (%) | E.e. (%) | Abs conf | E.e. (%) | Abs Conf | E[b] | E[c] | E[d] |
| 46 | 676 | *Pichia haplophila* | NCYC 3176 | 180 | 19.1 | 22.7 | R | 98.0 | S | 64.0 | 127.2 | 126. 6 |
| 47 | 673 | *Pichia haplophila* | NCYC 3177 | 60 | 37.2 | 40.7 | R | 53.7 | S | 7.9 | 4.5 | 4.9 |
| 48 | 28 | *Pichia haplophila* | UOFS Y-2136 | 60 | 29.9 | 31.1 | R | 56.3 | S | 8.7 | 4.5 | 4.8 |
| 49 | 692 | *Rhodosporidium paludigenum* | NCYC 3179 | 180 | 72.9 | 100.0 | S | 36.5 | R | 17.2 | 8.4 | 16.8 |
| 50 | Alf 01 | *Rhodosporidium toruloides* | NCYC 3181 | 10 | 98.0 | 100.0 | S | 23.7 | R | 4.1 | 6.5 | 11.3 |
| 51 | Car 118 | *Rhodosporidium toruloides* | NCYC 3182 | 20 | 23.5 | 27.6 | S | 63.3 | R | 23.7 | 5.4 | 5.8 |
| 52 | 671 | *Rhodosporidium toruloides* | UOFS Y-0472 | 180 | 52.9 | 33.2 | S | 31.4 | R | 2.5 | 2.6 | 2.6 |
| 53 | Alf 02 | *Rhodosporidium toruloides* | UOFS Y-0518 | 20 | 46.9 | 51.0 | S | 57.4 | R | 6.0 | 6.0 | 6.0 |
| 54 | Car 003 | *Rhodosporidium toruloides* | UOFS Y-2222 | 20 | 31.2 | 38.0 | S | 59.5 | R | 16.3 | 5.1 | 5.6 |
| 55 | Car 006 | *Rhodosporidium toruloides* | UOFS Y-2223 | 20 | 26.1 | 32.7 | S | 61.2 | R | 35.7 | 5.1 | 5.7 |
| 56 | Car 020 | *Rhodosporidium toruloides* | UOFS Y-2226 | 20 | 41.6 | 44.6 | S | 57.1 | R | 6.7 | 5.4 | 5.6 |
| 57 | Car 038 | *Rhodosporidium toruloides* | UOFS Y-2228 | 20 | 23.5 | 28.4 | S | 63.6 | R | 34.1 | 5.4 | 5.9 |
| 58 | Car 052 | *Rhodosporidium toruloides* | UOFS Y-2230 | 20 | 45.9 | 55.9 | S | 53.5 | R | 8.4 | 5.1 | 5.6 |
| 59 | Car 059 | *Rhodosporidium toruloides* | UOFS Y-2231 | 20 | 37.7 | 39.3 | S | 58.1 | R | 6.8 | 5.3 | 5.5 |
| 60 | Car 067 | *Rhodosporidium toruloides* | UOFS Y-2236 | 20 | 23.3 | 29.4 | S | 64.1 | R | 83.0 | 5.5 | 6.1 |

(continued)

| Samp no. | Screen no | Yeast species | Culture Collection Nr | Reaction | | Remaining Epoxide | | Formed Diol | | Selectivity | | |
|---|---|---|---|---|---|---|---|---|---|---|---|---|
| | | | | Time (min) | Conv (%) | E.e. (%) | Abs conf | E.e. (%) | Abs Conf | $E^b$ | $E^c$ | $E^d$ |
| 61 | Car 070 | *Rhodosporidium toruloides* | UOFS Y-2237 | 60 | 66.8 | 100.0 | S | 37.5 | R | 25.1 | 4.6 | 17.3 |
| 62 | Car 076 | *Rhodosporidium toruloides* | UOFS Y-2238 | 60 | 81.8 | 100.0 | S | 18.3 | R | 10.8 | 3.1 | 9.4 |
| 63 | Car 077 | *Rhodosporidium toruloides* | UOFS Y-2239 | 60 | 83.5 | 100.0 | S | 16.1 | R | 9.9 | 2.9 | 8.7 |
| 64 | Car 078 | *Rhodosporidium toruloides* | UOFS Y-2240 | 20 | 27.1 | 28.6 | S | 63.2 | R | 10.2 | 5.6 | 5.8 |
| 65 | Car 092 | *Rhodosporidium toruloides* | UOFS Y-2241 | 60 | 83.9 | 100.0 | S | 15.8 | R | 9.7 | 2.9 | 8.6 |
| 66 | Car 093 | *Rhodosporidium toruloides* | UOFS Y-2242 | 60 | 81.9 | 100.0 | S | 18.1 | R | 10.7 | 3.1 | 9.4 |
| 67 | Car 094 | *Rhodosporidium toruloides* | UOFS Y-2243 | 20 | 22.5 | 27.7 | S | 63.0 | R | 56.1 | 5.3 | 5.7 |
| 68 | Car 100 | *Rhodosporidium toruloides* | UOFS Y-2245 | 60 | 66.7 | 100.0 | S | 37.1 | R | 25.4 | 4.5 | 17.1 |
| 69 | Car 103 | *Rhodosporidium toruloides* | UOFS Y-2246 | 60 | 66.5 | 100.0 | S | 37.2 | R | 25.7 | 4.5 | 17.2 |
| 70 | Car 108 | *Rhodosporidium toruloides* | UOFS Y-2247 | 60 | 82.6 | 100.0 | S | 17.7 | R | 10.4 | 3.2 | 9.3 |
| 71 | Car 120 | *Rhodosporidium toruloides* | UOFS Y-2249 | 60 | 82.5 | 100.0 | S | 17.5 | R | 10.4 | 3.1 | 9.2 |
| 72 | Car 121 | *Rhodosporidium toruloides* | UOFS Y-2250 | 60 | 80.9 | 100.0 | S | 19.6 | R | 11.3 | 3.2 | 9.9 |
| 73 | Car 126 | *Rhodosporidium toruloides* | UOFS Y-2251 | 20 | 29.3 | 38.0 | S | 58.6 | R | 33.9 | 4.8 | 5.5 |

(continued)

| Samp no. | Screen no | Yeast species | Culture Collection Nr | Reaction | | Remaining Epoxide | | Formed Diol | | Selectivity | | |
|---|---|---|---|---|---|---|---|---|---|---|---|---|
| | | | | Time (min) | Conv (%) | E.e. (%) | Abs conf | E.e. (%) | Abs Conf | $E^b$ | $E^c$ | $E^d$ |
| 74 | Car 131 | *Rhodosporidium toruloides* | UOFS Y-2252 | 60 | 66.0 | 100.0 | S | 37.3 | R | 26.7 | 4.4 | 17.2 |
| 75 | Car 134 | *Rhodosporidium toruloides* | UOFS Y-2253 | 60 | 81.9 | 100.0 | S | 18.1 | R | 10.7 | 3.1 | 9.4 |
| 76 | Car 142 | *Rhodosporidium toruloides* | UOFS Y-2255 | 60 | 71.7 | 90.0 | S | 29.3 | R | 5.8 | 3.7 | 4.9 |
| 77 | Car 200 | *Rhodosporidium toruloides* | UOFS Y-2256 | 10 | 34.8 | 37.0 | S | 53.2 | R | 7.9 | 4.3 | 4.6 |
| 78 | Car 204 | *Rhodosporidium toruloides* | UOFS Y-2257 | 60 | 83.9 | 100.0 | S | 15.8 | R | 9.7 | 2.9 | 8.6 |
| 79 | Car 205 | *Rhodosporidium toruloides* | UOFS Y-2258 | 60 | 63.8 | 100.0 | S | 40.0 | R | 31.5 | 4.6 | 18.7 |
| 80 | Car 209 | *Rhodosporidium toruloides* | UOFS Y-2260 | 20 | 14.3 | 15.5 | S | 68.1 | R | 31.2 | 5.9 | 6.1 |
| 81 | Car 210 | *Rhodosporidium toruloides* | UOFS Y-2261 | 60 | 80.7 | 100.0 | S | 19.1 | R | 11.4 | 3.1 | 9.7 |
| 82 | 46 | *Rhodosporidium toruloides* | UOFS Y-0471 | 20 | 11.6 | 11.0 | S | 74.2 | R | 12.3 | 7.4 | 7.5 |
| 83 | 25 | *Rhodotorula araucaride* | NCYC 3183 | 180 | 65.2 | 57.5 | S | 33.5 | R | 3.2 | 3.6 | 3.4 |
| 84 | EP 230 | *Rhodotorula aurantiaca* | NCYC 3185 | 180 | 54.7 | 46.0 | S | 51.9 | R | 3.4 | 5.8 | 4.9 |
| 85 | 50 | *Rhodotorula glutinis* | NCYC 3186 | 20 | 62.2 | 87.5 | S | 46.1 | R | 8.9 | 5.9 | 7.2 |
| 86 | 680 | *Rhodotorula glutinis* | UOFS Y-0459 | 20 | 57.3 | 75.2 | S | 51.5 | R | 7.7 | 6.2 | 6.8 |
| 87 | 713 | *Rhodotorula glutinis* | UOFS Y-0489 | 60 | 62.5 | 100.0 | S | 37.7 | R | 35.3 | 4.0 | 17.5 |

(continued)

| Samp no. | Screen no | Yeast species | Culture Collection Nr | Reaction | | Remaining Epoxide | | Formed Diol | | Selectivity | | |
|---|---|---|---|---|---|---|---|---|---|---|---|---|
| | | | | Time (min) | Conv (%) | E.e. (%) | Abs conf | E.e. (%) | Abs Conf | $E^b$ | $E^c$ | $E^d$ |
| 88 | Alf 06 | *Rhodotorula glutinis* | UOFS Y-0513 | 20 | 22.8 | 28.4 | S | 64.0 | R | 63.9 | 5.5 | 6.0 |
| 89 | Car 022 | *Rhodotorula glutinis* | UOFS Y-2227 | 60 | 75.9 | 100.0 | S | 26.3 | R | 14.5 | 3.9 | 12.3 |
| 90 | Car 060 | *Rhodotorula glutinis* | UOFS Y-2232 | 20 | 33.9 | 38.6 | S | 59.3 | R | 10.2 | 5.2 | 5.6 |
| 91 | Car 061 | *Rhodotorula glutinis* | UOFS Y-2233 | 60 | 74.8 | 100.0 | S | 27.8 | R | 15.5 | 4.0 | 12.9 |
| 92 | Car 062 | *Rhodotorula glutinis* | UOFS Y-2234 | 20 | 27.3 | 31.3 | S | 61.7 | R | 14.8 | 5.3 | 5.7 |
| 93 | Car 066 | *Rhodotorula glutinis* | UOFS Y-2235 | 60 | 77.3 | 100.0 | S | 24.1 | R | 13.5 | 3.6 | 11.5 |
| 94 | Car 075 | *Rhodotorula glutinis* | UOFS Y-2265 | 20 | 42.8 | 45.3 | S | 57.0 | R | 6.3 | 5.5 | 5.6 |
| 95 | 714 | *Rhodotorula minuta* | NCYC 3187 | 20 | 33.3 | 24.0 | R | 37.9 | S | 3.6 | 2.7 | 2.8 |
| 96 | 712 | *Rhodotorula minuta* | UOFS Y-0835 | 60 | 46.9 | 52.7 | R | 48.4 | S | 6.6 | 4.3 | 4.7 |
| 97 | 686 | *Rhodotorula minuta var. minuta* | NCYC 3188 | 60 | 30.1 | 25.8 | R | 50.5 | S | 5.1 | 3.7 | 3.9 |
| 98 | 158 | *Rhodotorula sp. "minuta/mucilaginosa"* | NCYC 3194 | 180 | 40.1 | 21.5 | R | 31.5 | S | 2.4 | 2.3 | 2.3 |
| 99 | 690 | *Rhodotorula sp. neatest "minute"* | UOFS Y-0125 | 180 | 49.3 | 29.5 | R | 28.2 | S | 2.4 | 2.3 | 2.3 |
| 100 | 172 | *Rhodotorula sp.* | NCYC 3192 | 180 | 54.9 | 83.0 | S | 70.9 | R | 13.4 | 16.0 | 14.9 |
| 101 | 165 | *Rhodotorula sp.* | NCYC 3193 | 180 | 16.9 | 13.3 | S | 69.6 | R | 5.5 | 6.4 | 6.4 |
| 102 | 24 | *Rhodotorula sp.* | UOFS Y-2042 | 180 | 18.3 | 17.4 | S | 83.2 | R | 9.3 | 13.1 | 12.9 |

(continued)

| Samp no. | Screen no | Yeast species | Culture Collection Nr | Reaction | | Remaining Epoxide | | Formed Diol | | Selectivity | | |
|---|---|---|---|---|---|---|---|---|---|---|---|---|
| | | | | Time (min) | Conv (%) | E.e. (%) | Abs conf | E.e. (%) | Abs Conf | $E^b$ | $E^c$ | $E^d$ |
| 103 | 293 | *Sporidiobolus salmonicolor* | NCYC 3195 | 90 | 9.4 | 8.1 | S | 61.3 | R | 8.8 | 4.4 | 4.5 |
| 104 | 42 | *Sporidiobolus salmonicolor* | NCYC 3196 | 90 | 13.2 | 13.0 | S | 60.2 | R | 14.3 | 4.4 | 4.6 |
| 105 | 285 | *Sporobolomyces roseus* | NCYC 3197 | 90 | 44.0 | 62.0 | S | 52.4 | R | 15.9 | 4.7 | 5.9 |
| 106 | 696 | *Sporobolomyces sp. "holsaticus"* | NCYC 3198 | 180 | 46.3 | 39.1 | S | 45.2 | R | 3.8 | 3.8 | 3.8 |
| 107 | 283 | *Sporobolomyces tsugae* | NCYC 3199 | 90 | 8.3 | 6.5 | S | 18.7 | S | 6.4 | 1.5 | 1.6 |
| 108 | 20 | *Trichosporon cutaneum var cutaneum* | NCYC 3201 | 180 | 24.1 | 15.9 | R | 61.7 | S | 3.5 | 5.1 | 4.9 |
| 109 | 14 | *Trichosporon mucoides* | NCYC 3205 | 180 | 17.0 | 8.3 | R | 3.3 | S | 2.6 | 1.1 | 1.1 |
| 110 | 19 | *Trichosporon ovoides* | NCYC 3207 | 180 | 40.5 | 27.8 | R | 41.2 | S | 3.1 | 3.1 | 3.1 |
| 111 | 225 | *Trichosporon sp.* | NCYC 3211 | 180 | 36.6 | 21.1 | S | 37.4 | R | 2.6 | 2.7 | 2.7 |
| 112 | 39 | *Wingea robertsiae* | NCYC 3228 | 180 | 9.3 | 6.3 | R | 45.3 | S | 4.4 | 2.8 | 2.8 |
| 113 | 711 | *Yarrowia lipolytica* | NCYC 3229 | 180 | 8.6 | 7.5 | S | 73.5 | R | 9.8 | 7.0 | 7.1 |
| 114 | Jen 48 | *Yarrowia lipolytica* | UOFS Y-1700 | 90 | 6.5 | 5.1 | S | 98.1 | R | 7.1 | 109 | 107 |

[a] Reaction conditions: 20 mM epoxide, 20 % cells [(w/v), wet weight] in phosphate buffer (50 mM, pH 7.5)
[b] E calculated from equation 1
[c] E calculated from equation 2
[d] E calculated from equation 3

All the yeast strains corresponding to sample 1-114 (Table 2) are kept and maintained at the University of the Orange Free State (UOFS), Department of Microbial, Biochemical and Food Biotechnology, Faculty of Natural and Agricultural Sciences, P.O. Box 339, Bloemfontein 9300, South Africa (Tel +27 51 401 2396, Fax + 27 51 444 3219) and are readily identified by the yeast species and culture collection number as indicated. Representative examples of strains belonging to the different species have been deposited under the Budapest Treaty at National Collection of Yeast Cultures (NCYC), Institute of Food Research Norwich Research Park Colney, Norwich NR4 7UA, U.K. (Tel: +44-(0)1603-255274 Fax: +44-(0)1603-458414 Email: ncyc@bbsrc.ac.uk) and are readily identified by the yeast species and culture collection accession number as indicated. The samples deposited with the NCYC are taken from the same deposit maintained by the South African Council for Scientific and Industrial Research (CSIR) since prior to the filing date of this application. The deposits will be maintained without restriction in the NCYC depository for a period of 30 years, or 5 years after the most recent request, or for the effective life of the patent, whichever is longer, and will be replaced if the deposit becomes non-viable during that period. Samples of the yeast strains not deposited at NCYC will be made available upon request on the same basis and conditions of the Budapest Treaty.

**Example IV. Selection of yeasts that are able to produce optically active 2-methyl-3-phenyl-1,2-epoxypropane and 2-methyl-3-phenyl-1,2-propanediol from (±)-2-methyl-3-phenyl-1,2-epoxypropane**

Yeasts were cultivated, harvested and frozen as described above. The 2,2-disubstituted epoxide 2-methyl-3-phenyl-1,2-epoxypropane was added and the screening was performed as described above. Strains with the highest activities as judged by TLC from diol formation were subjected to chiral GC analysis as described above. The strains that were able to produce optically active 2-methyl-3-phenyl-1,2-epoxypropane and 2-methyl-3-phenyl-1,2-propanediol from the racemic epoxide are listed as samples 115-210 in Table 3.

**Table 3.** Yeast strains that hydrolyse 2-methyl-3-phenyl-1,2-epoxypropane enantioselectively

| Sample No | Screen no | Yeast species | Culture Collection no. | Epoxide ee | Diol ee | E[a] | Abs.conf. | |
|---|---|---|---|---|---|---|---|---|
| | | | | (%) | (%) | | Epox. | Diol |
| 115 | 43 | *Bullera dendrophila* | NCYC 3152 | 32.2 | 15.7 | 1.8 | R | S |
| 116 | Jen-26 | *Bullera dendrophila* | NCYC 3208 | 21.1 | 20.2 | 1.8 | R | S |
| 117 | 708 | *Candida rugosa* | NCYC 3155 | 3.4 | 26.1 | 1.8 | R | S |
| 118 | POH-29 | *Candida* sp. (new) (*C. sorbophila*) | NCYC 3217 | 6.5 | 78.5 | 8.9 | R | S |
| 119 | Jen-2 | *Cryptococcus albidus* | NCYC 3156 | 26.5 | 93.8 | 40.2 | S | R |
| 120 | Jen-17 | *Cryptococcus albidus* | UOFS Y-0223 | 21.5 | 58.6 | 4.7 | S | R |
| 121 | Jen-3 | *Cryptococcus albidus* | UOFS Y-0821 | 100 | 63.0 | 38.8 | S | R |
| 122 | Jen-23 | *Cryptococcus amylolentus* | NCYC 3157 | 42.2 | 65.8 | 7.3 | R | S |
| 123 | Car-14 | *Cryptococcus curvatus* | UOFS Y-2225 | 8.5 | 34.9 | 2.2 | R | S |
| 124 | Jen-15 | *Cryptococcus hungaricus* | NCYC 3159 | 99.9 | 17.2 | 7.1 | S | R |
| 125 | Jen-12 | *Cryptococcus laurentii* | NCYC 3160 | 5.5 | 23.7 | 1.7 | R | S |
| 126 | AB-24 | *Cyptococcus laurentii* | NCYC 3161 | 7.4 | 15.5 | 1.5 | R | S |
| 127 | Alf 03 | *Cryptococcus laurentii* | UOFS Y-0514 | 7.6 | 20.1 | 1.6 | R | S |
| 128 | AB-25 | *Cryptococcus laurentii* | UOFS Y-1884 | 7.3 | 14.7 | 1.4 | R | S |
| 129 | AB-26 | *Cryptococcus laurentii* | UOFS-1885 | 10 | 15.2 | 1.5 | R | S |
| 130 | AB-27 | *Cryptococcus laurentii* | UOFS Y-1886 | 8.2 | 17.5 | 1.5 | R | S |
| 131 | AB-32 | *Cryptococcus laurentii* | UOFS Y-1887 | 5 | 15.9 | 1.4 | R | S |

(continued)

| Sample No | Screen no | Yeast species | Culture Collection no. | Epoxide ee | Diol ee | E[a] | Abs.conf. | |
|---|---|---|---|---|---|---|---|---|
| | | | | (%) | (%) | | Epox. | Diol |
| 132 | AB-33 | *Cryptococcus laurentii* | UOFS Y-1888 | 6.9 | 16 | 1.5 | R | S |
| 133 | Jen-9 | *Cryptococcus laurentii var. laurentii* | UOFS Y-1349 | 1.3 | 28.4 | 1.8 | R | S |
| 134 | Jen-16 | *Cryptococcus luteolus* | NCYC 3162 | 0.1 | 27.4 | 1.8 | R | S |
| 135 | BV-2 | *Cryptococcus macerans* | NCYC 3213 | 9.2 | 70.2 | 6.2 | R | S |
| 136 | AB-58 | *Cryptococcus podzolicus* | NCYC 3164 | 4.7 | 88.2 | 16.7 | S | R |
| 137 | AB-54 | *Cryptococcus podzolicus* | UOFS Y-1900 | 6.8 | 2.8 | 1.1 | S | R |
| 138 | AB-43 | *Cryptococcus podzolicus* | UOFS Y-1902 | 6.5 | 0.8 | 1.1 | S | R |
| 139 | AB-46 | *Cryptococcus podzolicus* | UOFS Y-1907 | 10.1 | 94.1 | 36.4 | S | R |
| 140 | AB-56 | *Cryptococcus podzolicus* | UOFS Y-1913 | 11.1 | 92.1 | 27.2 | S | R |
| 141 | AB-57 | *Cryptococcus podzolicus* | UOFS Y-1914 | 12.8 | 96.9 | 73 | S | R |
| 142 | 113 | *Debaryomyces hansenii* | NCYC 3167 | 4.6 | 27.4 | 1.8 | R | S |
| 143 | 520 | *Pichia finlandica* | NCYC 3173 | 8.5 | 79.6 | 9.6 | R | S |
| 144 | 707 | *Pichia guillermondii* | NCYC 3174 | 4.9 | 28.3 | 1.9 | R | S |
| 145 | 702 | *Pichia guillermondii* | NCYC 3175 | 1.5 | 27.7 | 1.8 | R | S |
| 146 | 112 | *Pichia guillermondii* | UOFS Y-0053 | 6.2 | 17.3 | 1.5 | R | S |
| 147 | 706 | *Pichia guillermondii* | UOFS Y-0057 | 2.1 | 30 | 1.9 | R | S |
| 148 | Alf 01 | *Rhodosporidium toruloides* | NCYC 3181 | 0 | 36.9 | 2.2 | S | R |
| 149 | Car-118 | *Rhodosporidium toruloides* | NCYC 3182 | 5.5 | 29.3 | 1.9 | S | R |
| 150 | POH-28 | *Rhodosporidium toruloides* | NCYC 3215 | 9.1 | 15.2 | 1.5 | S | R |
| 151 | POH-20 | *Rhodosporidium toruloides* | NCYC 3216 | 5.2 | 13.5 | 1.4 | S | R |
| 152 | POH-38 | *Rhodosporidium toruloides* | NCYC 3219 | 14.6 | 11.4 | 1.4 | S | R |
| 153 | 46 | *Rhodosporidium toruloides* | UOFS Y-0471 | 1.0 | 17.6 | 1.4 | S | R |
| 154 | Alf 02 | *Rhodosporidium toruloides* | UOFS Y-0518 | 1.5 | 37.9 | 2.3 | S | R |
| 155 | Car-3 | *Rhodosporidium toruloides* | UOFS Y-2222 | 3.5 | 25 | 1.7 | S | R |
| 156 | Car-6 | *Rhodosporidium toruloides* | UOFS Y-2223 | 1.7 | 26.5 | 1.8 | S | R |
| 157 | Car-20 | *Rhodosporidium toruloides* | UOFS Y-2226 | 3.7 | 24.4 | 1.7 | S | R |
| 158 | Car-38 | *Rhodosporidium toruloides* | UOFS Y-2228 | 2.4 | 36.7 | 2.2 | S | R |
| 159 | Car-52 | *Rhodosporidium toruloides* | UOFS Y-2230 | 5.3 | 27.8 | 1.9 | S | R |
| 160 | Car-59 | *Rhodosporidium toruloides* | UOFS Y-2231 | 0.4 | 25 | 1.7 | S | R |

(continued)

| Sample No | Screen no | Yeast species | Culture Collection no. | Epoxide ee | Diol ee | E[a] | Abs.conf. | |
|---|---|---|---|---|---|---|---|---|
| | | | | (%) | (%) | | Epox. | Diol |
| 161 | Car-67 | *Rhodosporidium toruloides* | UOFS Y-2236 | 2.7 | 31.3 | 2.0 | S | R |
| 162 | Car-70 | *Rhodosporidium toruloides* | UOFS Y-2237 | 4.8 | 21.2 | 1.6 | S | R |
| 163 | Car-76 | *Rhodosporidium toruloides* | UOFS Y-2238 | 5.9 | 22.1 | 1.7 | S | R |
| 164 | Car-77 | *Rhodosporidium toruloides* | UOFS Y-2239 | 4.5 | 22.8 | 1.7 | S | R |
| 165 | Car-78 | *Rhodosporidium toruloides* | UOFS Y-2240 | 3.8 | 29.9 | 1.9 | S | R |
| 166 | Car-93 | *Rhodosporidium toruloides* | UOFS Y-2242 | 3.0 | 22.7 | 1.6 | S | R |
| 167 | Car-94 | *Rhodosporidium toruloides* | UOFS Y-2243 | 4.7 | 31.8 | 2.0 | S | R |
| 168 | Car-100 | *Rhodosporidium toruloides* | UOFS Y-2245 | 4.8 | 24.3 | 1.7 | S | R |
| 169 | Car-103 | *Rhodosporidium toruloides* | UOFS Y-2246 | 2.1 | 23.5 | 1.6 | S | R |
| 170 | Car-108 | *Rhodosporidium toruloides* | UOFS Y-2247 | 3.8 | 24.9 | 1.7 | S | R |
| 171 | Car-120 | *Rhodosporidium toruloides* | UOFS Y-2249 | 3.4 | 27.0 | 1.8 | S | R |
| 172 | Car-121 | *Rhodosporidium toruloides* | UOFS Y-2250 | 5.0 | 29.6 | 1.9 | S | R |
| 173 | Car-126 | *Rhodosporidium toruloides* | UOFS Y-2251 | 5.4 | 18.9 | 1.5 | S | R |
| 174 | Car-131 | *Rhodosporidium toruloides* | UOFS Y-2252 | 3.3 | 26.2 | 1.8 | S | R |
| 175 | Car-134 | *Rhodosporidium toruloides* | UOFS Y-2253 | 3.6 | 23.7 | 1.7 | S | R |
| 176 | Car-142 | *Rhodosporidium toruloides* | UOFS Y-2255 | 5.5 | 22.4 | 1.7 | S | R |
| 177 | Car-200 | *Rhodosporidium toruloides* | UOFS Y-2256 | 5.1 | 8.0 | 1.2 | S | R |
| 178 | Car-204 | *Rhodosporidium toruloides* | UOFS Y-2257 | 3.4 | 24.3 | 1.7 | S | R |
| 179 | Car-205A | *Rhodosporidium toruloides* | UOFS Y-2258 | 1.9 | 25.5 | 1.7 | S | R |
| 180 | Car-209 | *Rhodosporidium toruloides* | UOFS Y-2260 | 6.4 | 22.0 | 1.7 | S | R |
| 181 | Car-210 | *Rhodosporidium toruloides* | UOFS Y-2261 | 4.6 | 27.6 | 1.8 | S | R |
| 182 | POH-33 | *Rhodosporidium toruloides* | NCYC 3218 | 11.2 | 10.1 | 1.4 | S | R |
| 183 | EP-230 | *Rhodotorula aurantiaca* | NCYC 3185 | 2.4 | 12.2 | 1.3 | S | R |
| 184 | 50 | *Rhodotorula glutinis* | NCYC 3186 | 2.5 | 29.7 | 1.9 | S | R |
| 185 | 680 | *Rhodotorula glutinis* | UOFS Y-0459 | 2.6 | 90.3 | 20.1 | S | R |

(continued)

| Sample No | Screen no | Yeast species | Culture Collection no. | Epoxide ee | Diol ee | E[a] | Abs.conf. | |
|---|---|---|---|---|---|---|---|---|
| | | | | (%) | (%) | | Epox. | Diol |
| 186 | 713 | *Rhodotorula glutinis* | UOFS Y-0489 | 8.8 | 36.5 | 2.3 | S | R |
| 187 | Alf 6 | *Rhodotorula glutinis* | UOFS Y-0513 | 1.0 | 39.7 | 2.3 | S | R |
| 188 | 681 | *Rhodotorula glutinis* | UOFS Y-0653 | 1.6 | 39.3 | 2.3 | S | R |
| 189 | Car-22 | *Rhodotorula glutinis* | UOFS Y-2227 | 2.7 | 14.9 | 1.4 | S | R |
| 190 | Car-60 | *Rhodotorula glutinis* | UOFS Y-2232 | 1.7 | 55.2 | 3.5 | S | R |
| 191 | Car-61 | *Rhodotorula glutinis* | UOFS Y-2233 | 6.6 | 28.2 | 1.9 | S | R |
| 192 | Car-62 | *Rhodotorula glutinis* | UOFS Y-2234 | 4.2 | 27.3 | 1.8 | S | R |
| 193 | Car-66 | *Rhodotorula glutinis* | UOFS Y-2235 | 4.0 | 27.7 | 1.8 | S | R |
| 194 | Car-75 | *Rhodotorula glutinis* | UOFS Y-2265 | 7.6 | 29.7 | 2.0 | S | R |
| 195 | 714 | *Rhodotorula minuta* | NCYC 3187 | 99.2 | 14.7 | 5.1 | R | S |
| 196 | 686 | *Rhodotorula minuta var. minuta* | NCYC 3188 | 100 | 51 | 25.8 | R | S |
| 197 | 712 | *Rhodotorula minuta var. minuta* | UOFS Y-0835 | 100 | 3.3 | 4.2 | R | S |
| 198 | 158 | *Rhodotorula sp. minuta / mucilaginosa* | NCYC 3194 | 16.4 | 2.4 | 1.2 | R | S |
| 199 | 690 | *Rhodotorula sp. nearest minuta* | UOFS Y-0125 | 80.1 | 15.5 | 2.8 | R | S |
| 200 | 172 | *Rhodotorula sp.* | NCYC 3192 | 4.1 | 15.1 | 1.4 | S | R |
| 201 | 24 | *Rhodotorula sp.* | UOFS Y-2042 | 11.2 | 78.1 | 9.1 | S | R |
| 202 | Jen-32 | *Sporidiobolus salmonicolor* | NCYC 3195 | 5.4 | 76.1 | 7.8 | S | R |
| 203 | Jen-29 | *Sporobolomyces roseus* | NCYC 3197 | 3.3 | 17 | 1.5 | R | S |
| 204 | Jen-28 | *Sporobolomyces tsugae* | NCYC 3199 | 8.8 | 23.9 | 1.8 | R | S |
| 205 | 232 | *Trichosporon cutaneum var. cutaneum* | NCYC 3202 | 32.0 | 80.3 | 12.5 | S | R |
| 206 | 20 | *Trichosporon cutaneum var. cutaneum* | NCYC 3201 | 23.8 | 100 | 200 | R | S |
| 207 | 22 | *Trichosporon jirovecii* | NCYC 3204 | 11.0 | 83.2 | 12.2 | S | R |
| 208 | BV-4 | *Trichosporon moniliiforme* | NCYC 3214 | 5.8 | 76.5 | 8.0 | S | R |
| 209 | 19 | *Trichosporon ovoides* | NCYC 3207 | 20.8 | 38.8 | 2.8 | R | S |
| 210 | 231 | *Trichosporon sp.* | NCYC 3210 | 7.1 | 73.7 | 7.1 | S | R |

[a] E calculated from equation 3

All the yeast strains corresponding to samples 115-210 (Table 3) are kept and maintained at the University of the Orange Free State (UOFS), Department of Microbial, Biochemical and Food Biotechnology, Faculty of Natural and Agricultural Sciences, P.O. Box 339, Bloemfontein 9300, South Africa (Tel +27 51 401 2396, Fax + 27 51 444 3219) and are readily identified by the yeast species and culture collection number as indicated. Representative examples of

strains belonging to the different species have been deposited under the Budapest Treaty at National Collection of Yeast Cultures (NCYC), Institute of Food Research Norwich Research Park Colney, Norwich NR4 7UA, U.K. (Tel: +44-(0)1603-255274 Fax: +44-(0)1603-458414 Email: ncyc@bbsrc.ac.uk) and are readily identified by the yeast species and culture collection accession numbers as indicated. The samples deposited with the NCYC are taken from the same deposit maintained by the South African Council for Scientific and Industrial Research (CSIR) since prior to the filing date of this application. The deposits will be maintained without restriction in the NCYC depository for a period of 30 years, or 5 years after the most recent request, or for the effective life of the patent, whichever is longer, and will be replaced if the deposit becomes non-viable during that period. Samples of the yeast strains not deposited at NCYC will be made available upon request on the same basis and conditions of the Budapest Treaty.

Three discrepancies were noted in the enantiopreferences for the aliphatic and aromatic epoxides:

1. *Bullera dendrophila* (NCYC 3152) was screened for both substrates and diplayed variance in enantiopreference depending on the substitutent: Se/Rd for 2-methyl-1,2-epoxyheptane and Re/Sd for 2-methyl-3-phenyl-1,2-epoxypropane.
2. *Cryptococcus curvatus:* Car 54 yielded Se/Rd for 2-methyl-1,2-epoxyheptane while Car 14 gave Re/Sd for 2-methyl-3-phenyl-1,2-epoxypropane.
3. *Trichopsoron cutaneum var cutaneum* (NCYC 3201) yielded Re/Sd for both epoxides, while NCYC 3202 yielded Re/Sd for 2-methyl-1,2-epoxyheptane but Se/Rd for 2-methyl-3-phenyl-1,2-epoxypropane.

These discrepancies can be due to the presence of more than one epoxide hydrolase having different activities towards aliphatic and aromatic substrates in these specific strains or to the incorrect classification of the strains. One skilled in the art will know how to test for such discrepancies and and to proceed accordingly.

**<u>Example V. Testing of samples selected from Examples III and IV</u>**

Exemplary yeast strain samples selected from Table 2 and Table 3 were tested (in this Example as samples 211-223) for their ability to produce optically active epoxides and optically active vicinal diols from 2,2-disubstituted epoxides. For each sample, two graphs are provided. The first graph (graph A in each figure) shows the change in concentrations of the epoxide and diol enantiomers with time, while the second graph (graph B in each figure) shows the enantiomeric excess of the epoxide and diol at different conversions. The yield of the optically active epoxide or diol that can be obtained at the required enantiomeric purity can be seen from these graphs. The TDE that were used to illustrate the use of the different yeast strains to prepare optically active epoxides and vicinal diols from gem-2,2-disubstituted epoxides represented by the general formula (I) is shown in scheme I in (Fig. 1).

**(a) Samples 211-219. Hydrolysis of (±)-2-methyl-1,2-epoxyheptane by selected yeasts to produce optically active (S)-2-methyl-1,2-epoxyheptane (1a) and/or (R)-2-methyl-1,2-heptanediol (1b) (Figs. 2A-10B).**

Hydrolysis of (±)-2-methyl-1,2-epoxyheptane by yeast strains selected from Table 2 was performed as described in Example I to produce (S)-2-methyl-1,2-epoxyheptane and/or (R)-2-methyl-1,2-heptanediol.

**(b) Sample 220. Hydrolysis of (±)-2-methyl-1,2-epoxyheptane by selected yeasts to produce optically active (R)-2-methyl-1,2-epoxyheptane and/or (S)-2-methyl-1,2-heptanediol (Figs. 11A and B).**

Hydrolysis of (±)-2-methyl-1,2-epoxyheptane by yeast strains selected from Table 2 was performed as described in Example I to produce (R)-2-methyl-1,2-epoxyheptane and/or (S)-2-methyl-1,2-heptanediol.

**(c) Sample 221. Hydrolysis of (±)-2-methyl-3-phenyl-1,2-epoxypropane by selected yeasts to produce optically active (S)-2-methyl-3-phenyl-1,2-epoxypropane and/or (R)-2-methyl-3-phenyl-1,2-propanediol (Figs. 12A and 12B).**

Hydrolysis of (±)-2-methyl-3-phenyl-1,2-epoxypropane by a yeast strain selected from Table 3 was performed as described in Example I to produce (S)-2-methyl-3-phenyl-1,2-epoxypropane and/or (R)-2-methyl-3-phenyl-1,2-propanediol.

**(d) Samples 222 and 223. Hydrolysis of (±)-2-methyl-3-phenyl-1,2-epoxypropane by selected yeasts to produce optically active (R)-2-methyl-3-phenyl-1,2-epoxypropane and/or (S)-2-methyl-3-phenyl-1,2-propanediol (Figs. 13A to 14B)**

Hydrolysis of (±)-2-methyl-3-phenyl-1,2-epoxypropane by yeasts selected from Table 3 was performed as described in Example I to produce (R)-2-methyl-3-phenyl-1,2-epoxypropane and/or (S)-2-methyl-3-phenyl-1,2-propanediol.

**Example VI. Hydrolysis of (±)-2-methyl-3-phenyl-1,2-epoxypropane by a yeast cultivated in a 10 L fermenter to produce optically active (S)-2-methyl-3-phenyl-1,2-epoxypropane and/or (R)-2-methyl-3-phenyl-1,2-propanediol**

Figs. 15A to 15B show hydrolysis of (±)-2-methyl-3-phenyl-1,2-epoxypropane by a yeast (Sample 224) cultivated in a 10 L fermenter to produce optically active (S)- 2-methyl-3-phenyl-1,2-epoxypropane and/or (R)-2-methyl-3-phenyl-1,2-propanediol.

**Example VII. Hydrolysis of (±)-2-methyl-3-phenyl-1,2-epoxypropane by recombinant yeast expression hosts transformed with the epoxide hydrolase genes from different wild type yeast strains**

Figs. 16A to 18B show hydrolysis of (±)-2-methyl-3-phenyl-1,2-epoxypropane by recombinant yeasts (as samples 225-227) expressing epoxide hydrolases from selected wild type yeast strains to produce optically active (S)- 2-methyl-3-phenyl-1,2-epoxypropane and/or (R)-2-methyl-3-phenyl-1,2-propanediol.

**(a) Sample 225.** Hydrolysis of (±)-2-methyl-3-phenyl-1,2-epoxypropane by host *Yarrowia lipolytica* cells transformed with the epoxide hydrolase coding sequence from *Rhodotorula araucariae* (NCYC 3183) (Fig. 16). The host strain is Po1h, the vector used was the pINA 1297 zeta-based auto-cloning multicopy vector from which a "yeast cassette" can be purified and integrated non-homologously into the genome of Po1h. The expression of the epoxide hydrolase gene is driven by the hp4d promoter. The XPR2 secretion signal was replaced by the LIP2 secretion signal in the vector. (See Madzak et al., Journal of Biotechnology 109, 2004). Reaction conditions: 50 % (w/v) cell suspension, 50 mM (±)-2-methyl-3-phenyl-1,2-epoxypropane.

**(b) Sample 226.** Hydrolysis of (±)-2-methyl-3-phenyl-1,2-epoxypropane by host *Yarrowia lipolytica* cells transformed with the epoxide hydrolase coding sequence from *Rhodosporidium paludigenum* (NCYC 3179) (Fig. 17). The host strain is Po1h. The expression of the epoxide hydrolase gene is driven by the constitutive TEF promoter. (See Madzak et al., Journal of Biotechnology 109, 2004). Reaction conditions: 50% (w/v) cell suspension, 100 mM (±)-2-methyl-3-phenyl-1,2-epoxypropane.

**(c) Sample 227.** Hydrolysis of (±)-2-methyl-3-phenyl-1,2-epoxypropane by the host *Yarrowia lipolytica* transformed with the gene from *Rhodosporidium paludigenum UOFS Y-0482/NCYC 3179* (Fig. 18). The host strain is Po1h. The expression of the epoxide hydrolase gene is driven by the hp4d promoter. (See Madzak et al., Journal of Biotechnology 109, 2004). Reaction conditions: 10 % (w/v) cell suspension, 100 mM (±)-2-methyl-3-phenyl-1,2-epoxypropane.

**Example VIII. Hydrolysis of (±)-2-methyl-3-phenyl-1,2-epoxypropane by a crude enzyme preparation obtained from the host *Yarrowia lipolytica* expressing the epoxide hydrolase derived from *Rhodotorula araucariae* NCYC 3183 (YL 25 TsA)**

Figs. 19A and 19B show hydrolysis of (±)-2-methyl-3-phenyl-1,2-epoxypropane by the epoxide hydrolase derived from *Rhodotorula araucariae* NCYC 3183 (YL 25 TsA) expressed in *Yarrowia lipolytica*. Fig 19A shows hydrolysis by whole cells and Fig. 19B shows hydrolysis by a crude enzyme prepartion obtained from the cells

**Example IX. Amino acid sequences of enantioselective epoxide hydrolase polypeptide for the hydrolysis of (±)-2,2disubstituted epoxides to produce optically active epoxides and/or diols.**

The sequences with SEQ ID NOs: 1-5 (Figs. 22-26) are examples of amino acid sequences of enantioselective epoxide hydrolases for the hydrolysis of (±)-2,2 -disubstituted epoxides to produce optically active (S)-epoxides and/or (R)- diols. The cDNAs encoding the polypeptides were obtained as described in Example II from the indicated yeast strains. The sequences with SEQ ID NOs:6 and 7 (Figs. 27 and 28) are examples of amino acid sequences of enantioselective epoxide hydrolases for the hydrolysis of (±)-2,2-disubstituted epoxides to produce optically active (R)-epoxides and/or (S)- diols. The cDNAs encoding the polypeptides were obtained as described in Example II from the indicated yeast strains.

**Example X. Nucleotide sequences of enantioselective epoxide hydrolase genes for the hydrolysis of (±)-2,2-disubstituted epoxides to produce optically active epoxides and/or diols.**

The sequences with SEQ ID NOs: 8-12 (Figs. 29-33) are examples of cDNA sequences encoding enantioselective epoxide hydrolase genes for the hydrolysis of (±)-2,2-disubstituted epoxides to produce optically active (S)-epoxides and/or (R)- diols. The cDNAs encoding the polypeptides were obtained as described in Example II from the indicated yeast strains.

The sequences with SEQ ID NOs:13 and 14 (Fig. 34 and 35) are examples of cDNA sequences encoding enantioselective epoxide hydrolase genes for the hydrolysis of (±)-2,2-disubstituted epoxides to produce optically active (R)-epoxides and/or (S)- diols. The cDNAs encoding the polypeptides were obtained as described in Example II from the indicated yeast strains.

**Example XI. Homology at the amino acid level of enantioelective epoxide hydolases for the hydrolysis of (±)-2,2-disubstituted epoxides to produce optically active epoxides and/or diols.**

Fig. 36 shows the percent homology at the amino acid level of enantioselective epoxide hydrolaseses described in the Example IX. Alignments were done using DNAMAN Version 5.2.9 (Lynnon Biosoft) software using the multiple sequence alignment mode (Fast alignment/Quick alignment settings).

**Example XII. Homology at nucleotide level of enantioelective epoxide hydolase cDNA for the hydrolysis of (±)-2,2-disubstituted epoxides to produce optically active epoxides and/or diols.**

Fig. 37 shows the percent homology at the nucleotide level of cDNAs encoding the enantioselective epoxide hydrolases described in Example X. Alignments were done in DNAMAN Version 5.2.9 (Lynnon Biosoft) software using the multiple sequence alignment mode (Fast alignment/Quick alignment settings).

References cited

Madzak, C., Gaillardin, C., Beckerich, J-M. (2004). Heterologous protein expression and secretion in the non-conventional yeast Yarrowia lipolytica: a review. Journal of Biotechnology 109: 63-81.

Mischitz, M.; Kroutil, W.; Wandel, U.; Faber, K. Tetrahedron Asymmetry 1995, 6, 1261-1272.

Nicaud J-M, Madzak C, Van den Broek P, Gysler C, Duboc P, Niederberger P, Gaillardin C. (2002) Protein expression and secretion in the yeast Yarrowia lipolytica. FEMS Yeast Research 2, 371-279.

Orru, R.V.A., Mayer, S., Kroutil, W. and Faber, K. (1998) Chemoenzymatic deracemization of (±)-2,2-disubstituted oxiranes. Tetrahedron 54: 859-874

Pedragosa-Moreau, S., Archelas, A. and Furstoss, R. (1996) Microbiological Transformations 32. Use of epoxide hydrolase mediated biohydrolysis as a way to enantiopure epoxides and vicinal diols - Application to substituted styrene oxide derivatives. Tetrahedron 52:4593-4606.

Steinreiber, A., Osprian, I., Mayer, S.F., Orru, R.V. and Faber, K. (2000) Enantioselective hydrolysis of functionalized 2,2-disubstituted oxiranes with bacterial epoxide hydrolases. Eur. J. Org. Chem. 22: 3703-3711.

Xuan J-W, Fournier P, Gaillardin C. (1988) Cloning of the LYS5 gene encoding saccharopine dehydrogenase from the yeast Yarrowia lipolytica by target integration. Current Genetics 14, 15-21.

**Claims**

1. A process for obtaining an optically active epoxide or an optically active vicinal diol, which process includes the steps of:

   providing an enantiomeric mixture of a 2,2-disubstituted epoxide;
   creating a reaction mixture by adding to the enantiomeric mixture a *Yarrowia lipolytica* cell comprising an exogenous nucleic acid encoding, and capable of expressing, a heterologous polypeptide or a functional fragment thereof, having enantioselective 2,2-disubstituted epoxide hydrolase activity;
   incubating the reaction mixture; and
   recovering from the reaction mixture: (a) an enantiopure, or a substantially enantiopure, 2,2-disubstituted vicinal diol; (b) an enantiopure, or a substantially enantiopure, 2,2-disubstituted epoxide; or (c) an enantiopure, or a

substantially enantiopure, 2,2-disubstituted vicinal diol and an enantiopure, or a substantially enantiopure, 2,2-disubstituted epoxide.

2. The process of claim 1, wherein the polypeptide is a full-length yeast epoxide hydrolase.

3. The process of claim 1, wherein the polypeptide is a functional fragment of yeast epoxide hydrolase.

4. The process of any one of claims 1 to 3, wherein the process is carried out at a pH from 5 to 10.

5. The process of any one of claims 1 to 4, wherein the process is carried out at a temperature of 0°C to 70°C.

6. The process of any one of claims 1 to 5, wherein the concentration of the 2,2-disubstituted epoxide in the reaction matrix is at least equal to the soluble concentration of the 2,2-disubstituted epoxide in water.

7. The process of any one of claims 1 to 6, wherein the 2,2-disubstituted epoxide of the enantiomeric mixture is a compound of the general formula (I) and the vicinal diol produced by the process is a compound of the general formula (II),

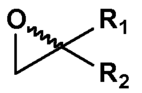

(I)

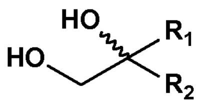

(II)

wherein:

$R_1$ and $R_2$ are, independently of each other, selected from the group consisting of a variably substituted straight-chain or branched alkyl group, a variably substituted straight-chain or branched alkenyl group, a variably substituted straight-chain or branched alkynyl group, a variably substituted cycloalkyl group as well as cycloalkenyl groups, a variably substituted aryl group, a variably substituted aryl alkyl group, a variably substituted heterocyclic group, a variably substituted straight-chain or branched alkoxy group, a variably substituted straight-chain or branched alkenyloxy group, a variably substituted aryloxy group, a variably substituted aryl alkyloxy group, a variably substituted alkylthio group, a variably substituted alkoxycarbonyl group, a variably substituted straight chain or branched alkylamino or alkenyl amino group, a variably substituted arylamino or arylalkylamino group, a variably substituted carbamoyl group, a variably substituted acyl group, and a functional group; or

wherein $R_1$ and $R_2$, together as a whole unit are a carbocycle with 5 to 7 atoms or a heterocycle with 5 to 7 carbon atoms.

8. The process of any of claims 1 to 7, wherein the enantiomeric mixture is a racemic mixture or a mixture of any ratio of amounts of the enantiomers.

9. The process of any of claims 1 to 8, which process includes adding to the reaction water and at least one water-immiscible solvent.

10. The process of claim 9, wherein the water-immiscible solvent is selected from the group consisting of toluene, 1,1,2-trichlorotrifluoroethane, methyl tert-butyl ether, methyl isobutyl ketone, dibutyl-o-phtalate, aliphatic alcohols containing 6 to 9 carbon atoms, and aliphatic hydrocarbons containing 6 to 16 carbon atoms.

11. The process of any of claims 1 to 8, which process includes adding to the reaction water and at least one water-miscible organic solvent.

12. The process of claim 11, wherein the water-miscible solvent is selected from the group consisting of acetone, methanol, ethanol, propanol, isopropanol, acetonitrile, dimethylsulfoxide, *N,N*-dimethylformamide, and *N*-methylpyrrolidine.

**13.** The process of any one of claims 1 to 12, which process includes adding to the reaction mixture at least one reagent selected from the group consisting of one or more surfactants, one or more cyclodextrins, and one or more phase-transfer catalysts.

**14.** The process of any one of claims 1 to 13, which process includes stopping the reaction when one enantiomer of the epoxide and/or vicinal diol is in excess compared to the other enantiomer of the epoxide and/or vicinal diol.

**15.** The process of any one of claims 1 to 14, which process includes recovering continuously during the reaction the optically active epoxide and/or the optically active vicinal diol produced by the reaction directly from the reaction mixture.

**Patentansprüche**

**1.** Verfahren zur Gewinnung eines optisch aktiven Epoxids oder eines optisch aktiven vicinalen Diols, wobei das Verfahren folgende Schritte enthält:

- Bereitstellen eines Enantiomerengemisches aus einem 2,2-disubstituierten Epoxid,
- Herstellen eines Reaktionsgemisches, indem zu dem Enantiomerengemisch eine *Yarrowia lipolytica*-Zelle gegeben wird, welche eine exogene Nukleinsäure enthält, die ein heterologes Polypeptid oder ein funktionelles Fragment davon kodiert und in der Lage ist, dieses zu enthalten, wobei dieses eine enantioselektive 2,2-disubstituierte Epoxidhydrolaseaktivität aufweist,
- Inkubieren des Reaktionsgemisches, und
- Gewinnung von (a) einem enantiomerenreinen oder eines im Wesentlichen enantiomerenreinen 2,2-disubstituierten vicinalen Diols, von (b) einem enantiomerenreinen oder einem im Wesentlichen enantiomerenreinen, 2,2-disubstituierten Epoxids, oder von (c) einem enantiomerenreinen oder einem im Wesentlichen enantiomerenreinen 2,2-disubstituierten vicinalen Diols und einem enantiomerenreinen oder einem im Wesentlichen enantiomerenreinen 2,2- disubstituierten Epoxids aus dem Reaktionsgemisch.

**2.** Verfahren nach Anspruch 1, wobei das Polypeptid eine volllängige Hefe-Epoxidhydrolase ist.

**3.** Verfahren nach Anspruch 1, wobei das Polypeptid ein funktionelles Fragment einer Hefe-Epoxidhydrolase ist.

**4.** Verfahren nach einem der Ansprüche 1 bis 3, wobei das Verfahren bei einem pH-Wert von 5 bis 10 durchgeführt wird.

**5.** Verfahren nach einem der Ansprüche 1 bis 4, wobei das Verfahren bei einer Temperatur von 0 °C bis 70 °C durchgeführt wird.

**6.** Verfahren nach einem der Ansprüche 1 bis 5, wobei die Konzentration des 2,2-disubstituierten Epoxids in der Reaktionsmatrix mindestens gleich der löslichen Konzentration des 2,2-disubstituierten Epoxids in Wasser ist.

**7.** Verfahren nach einem der Ansprüche 1 bis 6, wobei das 2,2-disubstituierte Epoxid in dem Enantiomerengemisch eine Verbindung der allgemeinen Formel (I) und das nach dem Verfahren hergestellte vicinale Diol eine Verbindung der allgemeinen Formel (II) ist

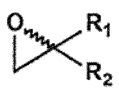 (I)

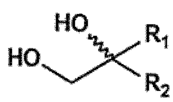 (II)

,

wobei $R_1$ und $R_2$ unabhängig voneinander ausgewählt sind aus einer Gruppe bestehend aus einer variabel substituierten geradkettigen oder verzweigten Alkylgruppe, einer variabel substituierten geradkettigen oder verzweigten

Alkenylgruppe, einer variabel substituierten geradkettigen oder verzweigten Alkinylgruppe, einer variabel substituierten Cycloalkylgruppe sowie Cycloalkenylgruppen, einer variabel substituierten Arylgruppe, einer variabel substituierten Arylalkylgruppe, einer variabel substituierten heterocyclischen Gruppe, einer variabel substituierten geradkettigen oder verzweigten Alkoxygruppe, einer variabel substituierten geradkettigen oder verzweigten Alkenyloxygruppe, einer variabel substituierten Aryloxy-Gruppe, einer variabel substituierten Arylalkyloxyruppe, einer variabel substituierten Alkylthiogruppe, einer variabel substituierten Alkoxycarbonylgruppe, einer variabel substituierten geradkettigen oder verzweigten Alkylamino- oder Alkenylaminogruppe, einer variabel substituierten Arylamino- oder Arylalkylaminogruppe, einer variabel substituierten Carbamoylgruppe, einer variabel substituierten Acylgruppe und einer funktionellen Gruppe, oder wobei $R_1$ und $R_2$ zusammen als eine Einheit einen Carbocyclus mit 5 bis 7 Atomen oder einen Heterocyclus mit 5 bis 7 Kohlenstoffatomen bilden.

**8.** Verfahren nach einem der Ansprüche 1 bis 7, wobei das Enantiomerengemisch ein racemisches Gemisch oder ein Gemisch mit beliebigem Mengenverhältnis der Enantiomere ist.

**9.** Verfahren nach einem der Ansprüche 1 bis 8, wobei das Verfahren die Zugabe von Wasser und von wenigstens einem nicht wassermischbaren Lösungsmittel zu dem Reaktionsgemisch beinhaltet.

**10.** Verfahren nach Anspruch 9, wobei das nicht wassermischbare Lösungsmittel ausgewählt ist aus einer Gruppe bestehend aus Toluol, 1,1,2-Trichlortrifluorethan, Methyl-*tert*-butylether, Methylisobutylketon, Dibutyl-o-phthalat, aliphatischen Alkoholen, die 6 bis 9 Kohlenstoffatome enthalten, und aliphatischen Kohlenwasserstoffen, die 6 bis 16 Kohlenstoffatome enthalten.

**11.** Verfahren nach einem der Ansprüche 1 bis 8, wobei das Verfahren die Zugabe von Wasser und von wenigstens einem wassermischbaren organischen Lösungsmittel zu dem Reaktionsgemisch beinhaltet.

**12.** Verfahren nach Anspruch 11, wobei das wassermischbare Lösungsmittel ausgewählt ist aus einer Gruppe bestehend aus Aceton, Methanol, Ethanol, Propanol, Isopropanol, Acetonitril, Dimethylsulfoxid, *N,N*-Dimethylformamid und *N*-Methylpyrrolidin.

**13.** Verfahren nach einem der Ansprüche 1 bis 12, wobei das Verfahren die Zugabe von wenigstens einem Reagenz zu dem Reaktionsgemisch beinhaltet, welches aus einer Gruppe bestehend aus einem oder mehreren Tensiden, einem oder mehreren Cyclodextrinen und einem oder mehreren Phasentransferkatalysatoren ausgewählt wird.

**14.** Verfahren nach einem der Ansprüche 1 bis 13, wobei das Verfahren ein Stoppen der Reaktion umfasst, wenn ein Enantiomer des Epoxids und/oder des vicinalen Diols verglichen mit dem anderen Enantiomer des Epoxids und/oder des vicinalen Diols im Überschuss vorliegt.

**15.** Verfahren nach einem der Ansprüche 1 bis 14, wobei das Verfahren ein kontinuierliches Abziehen des durch die Reaktion produzierten optisch aktiven Epoxids und/oder des optisch aktiven vicinalen Diols während der Reaktion direkt aus der Reaktionsmischung beinhaltet.

**Revendications**

**1.** Procédé d'obtention d'un époxyde optiquement actif ou d'un diol vicinal optiquement actif, lequel procédé inclut les étapes de :

fourniture d'un mélange énantiomérique d'un époxyde 2,2-disubstitué ;
création d'un mélange de réaction par addition au mélange énantiomérique d'une cellule de *Yarrowia lipolytica* comprenant un acide nucléique exogène codant, et capable d'exprimer, un polypeptide hétérologue ou un fragment fonctionnel de celui-ci, ayant une activité d'époxyde 2,2-disubstitué hydrolase énantiosélective ;
incubation du mélange de réaction ; et
récupération du mélange de réaction : (a) d'un diol vicinal 2,2-disubstitué énantiopur, ou sensiblement énantiopur ; (b) d'un époxyde 2,2-disubstitué énantiopur, ou sensiblement énantiopur ; ou (c) d'un diol vicinal 2,2-disubstitué énantiopur, ou sensiblement énantiopur et d'un époxyde 2,2-disubstitué énantiopur, ou sensiblement énantiopur.

**2.** Procédé selon la revendication 1, dans lequel le polypeptide est une époxyde hydrolase de levure pleine longueur.

**3.** Procédé selon la revendication 1, dans lequel le polypeptide est un fragment fonctionnel d'une époxyde hydrolase de levure.

**4.** Procédé selon l'une quelconque des revendications 1 à 3, dans lequel le procédé est réalisé à un pH de 5 à 10.

**5.** Procédé selon l'une quelconque des revendications 1 à 4, dans lequel le procédé est réalisé à une température de 0 °C à 70 °C.

**6.** Procédé selon l'une quelconque des revendications 1 à 5, dans lequel la concentration de l'époxyde 2,2-disubstitué dans la matrice de réaction est au moins égale à la concentration soluble de l'époxyde 2,2-disubstitué dans l'eau.

**7.** Procédé selon l'une quelconque des revendications 1 à 6, dans lequel l'époxyde 2,2-disubstitué du mélange énantiomérique est un composé de formule générale (I) et le diol vicinal produit par le procédé est un composé de formule générale (II),

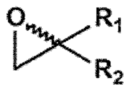

(I)

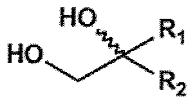

(II)

dans lesquels :

$R_1$ et $R_2$ sont, indépendamment l'un de l'autre, choisis dans le groupe consistant en un groupe alkyle à chaîne droite ou ramifié substitué de manière variable, un groupe alcényle à chaîne droite ou ramifié substitué de manière variable, un groupe alcynyle à chaîne droite ou ramifié substitué de manière variable, un groupe cycloalkyle substitué de manière variable ainsi que des groupes cycloalcényle, un groupe aryle substitué de manière variable, un groupe arylalkyle substitué de manière variable, un groupe hétérocyclique substitué de manière variable, un groupe alcoxy à chaîne droite ou ramifié substitué de manière variable, un groupe alcényloxy à chaîne droite ou ramifié substitué de manière variable, un groupe aryloxy substitué de manière variable, un groupe arylalkyloxy substitué de manière variable, un groupe alkylthio substitué de manière variable, un groupe alcoxycarbonyle substitué de manière variable, un groupe alkylamino ou alcénylamino à chaîne droite ou ramifié substitué de manière variable, un groupe arylamino ou arylalkylamino substitué de manière variable, un groupe carbamoyle substitué de manière variable, un groupe acyle substitué de manière variable, et un groupe fonctionnel ; ou

dans lesquels $R_1$ et $R_2$, réunis en une seule entité, sont un carbocycle de 5 à 7 atomes ou un hétérocycle de 5 à 7 atomes de carbone.

**8.** Procédé selon l'une quelconque des revendications 1 à 7, dans lequel le mélange énantiomérique est un mélange racémique ou un mélange de tout rapport de quantités des énantiomères.

**9.** Procédé selon l'une quelconque des revendications 1 à 8, lequel procédé inclut l'addition d'eau et d'au moins un solvant immiscible à l'eau à la réaction.

**10.** Procédé selon la revendication 9, dans lequel le solvant immiscible à l'eau est choisi dans le groupe consistant en le toluène, le 1,1,2-trichlorotrifluoroéthane, le méthyl*tert*-butyléther, la méthylisobutylcétone, l'*o*-phtalate de dibutyle, les alcools aliphatiques contenant 6 à 9 atomes de carbone, et les hydrocarbures aliphatiques contenant 6 à 16 atomes de carbone.

**11.** Procédé selon l'une quelconque des revendications 1 à 8, lequel procédé inclut l'addition d'eau et d'au moins un solvant miscible à l'eau à la réaction.

**12.** Procédé selon la revendication 11, dans lequel le solvant miscible à l'eau est choisi dans le groupe consistant en l'acétone, le méthanol, l'éthanol, le propanol, l'isopropanol, l'acétonitrile, le diméthylsulfoxyde, le *N,N*-diméthylformamide et la *N*-méthylpyrrolidine.

**13.** Procédé selon l'une quelconque des revendications 1 à 12, lequel procédé inclut l'addition au mélange de réaction d'au moins un réactif choisi dans le groupe consistant en un ou plusieurs tensioactifs, une ou plusieurs cyclodextrines, et un ou plusieurs catalyseurs de transfert de phase.

**14.** Procédé selon l'une quelconque des revendications 1 à 13, lequel procédé inclut l'arrêt de la réaction lorsqu'un énantiomère de l'époxyde et/ou du diol vicinal est en excès par rapport à l'autre énantiomère de l'époxyde et/ou du diol vicinal.

**15.** Procédé selon l'une quelconque des revendications 1 à 14, lequel procédé inclut la récupération en continu pendant la réaction de l'époxyde optiquement actif et/ou du diol vicinal optiquement actif produits par la réaction directement du mélange de réaction.

SCHEME 1

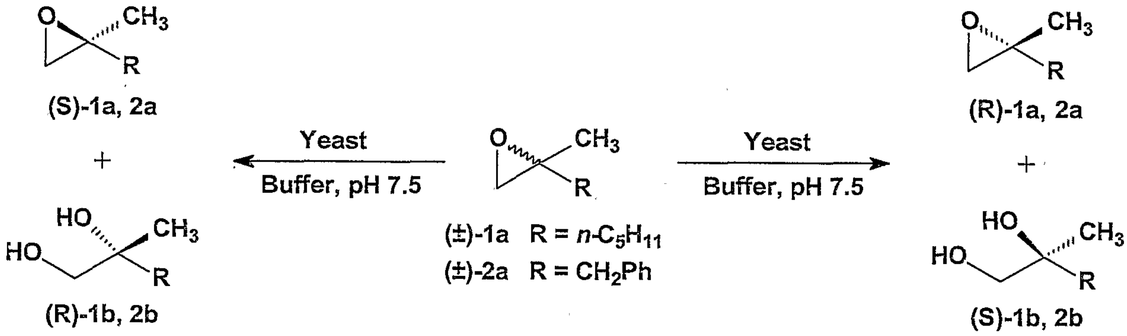


(S)-1a, 2a

+

(R)-1b, 2b

(±)-1a R = $n$-$C_5H_{11}$

(±)-2a R = $CH_2Ph$

(R)-1a, 2a

+

(S)-1b, 2b

FIG 1

Sample 211

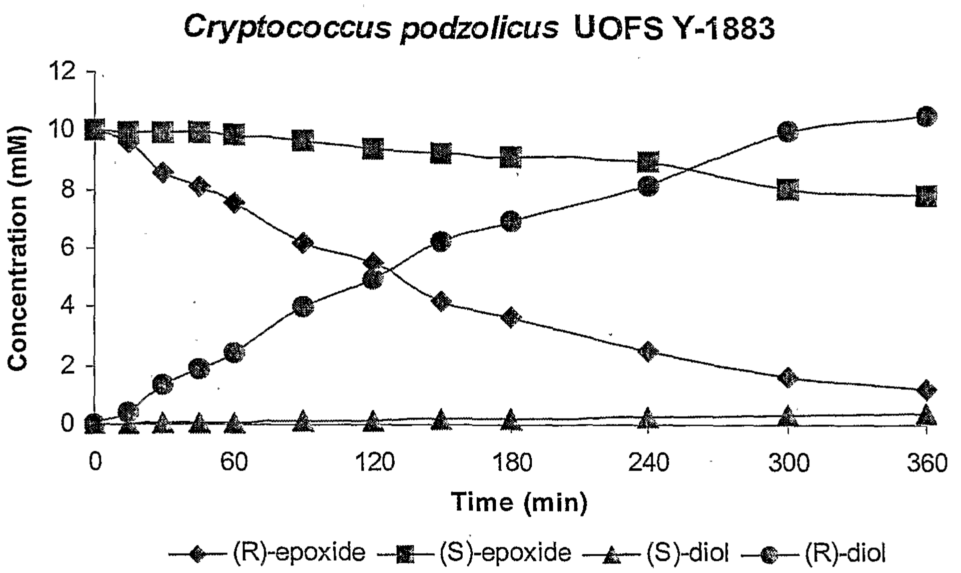


FIG 2A

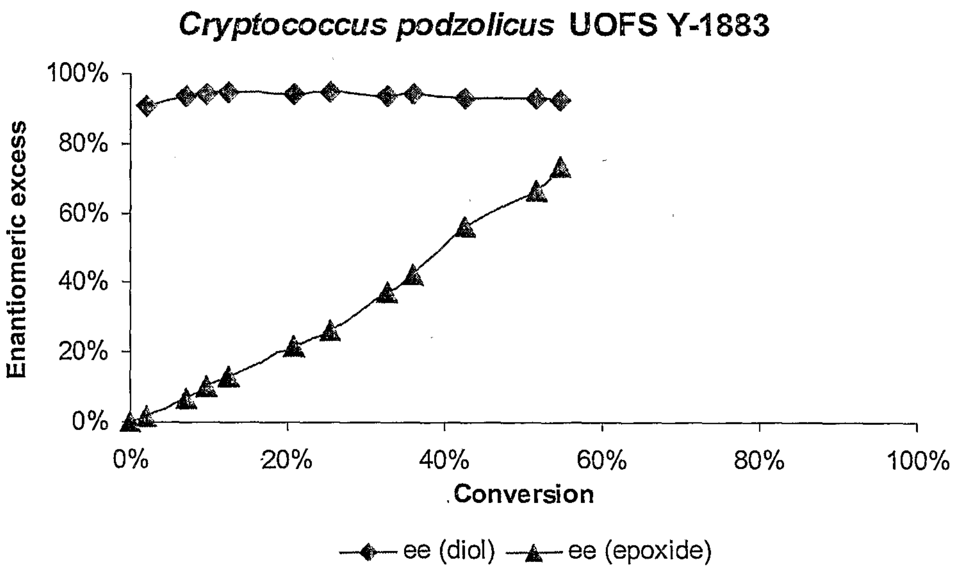


FIG 2B

Sample 212

*Cryptococcus podzolicus* UOFS Y-1904

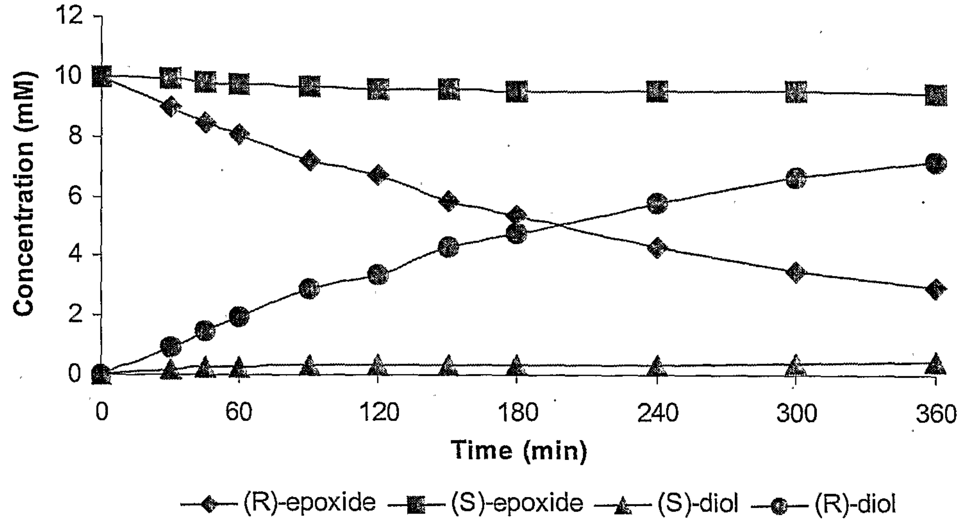


FIG 3A

*Cryptococcus podzolicus* UOFS Y-1904

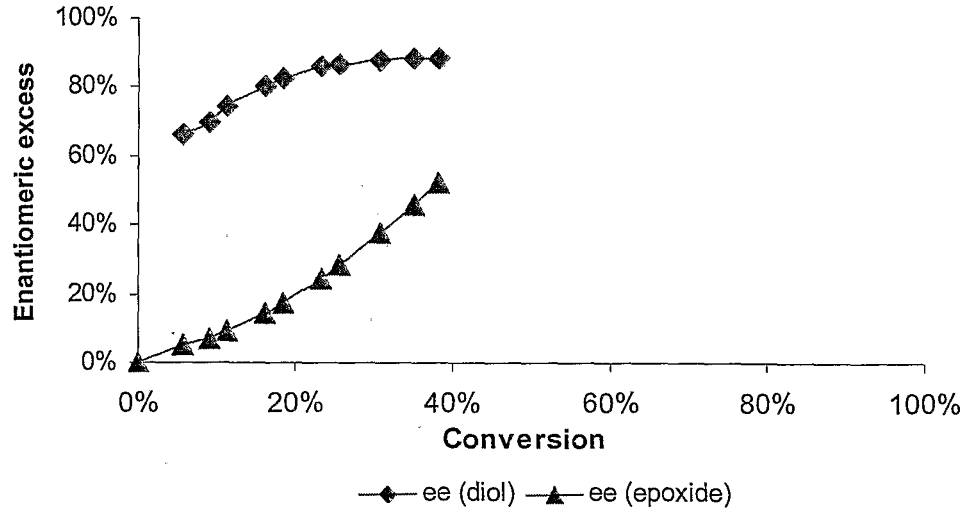


FIG 3B

Sample 213

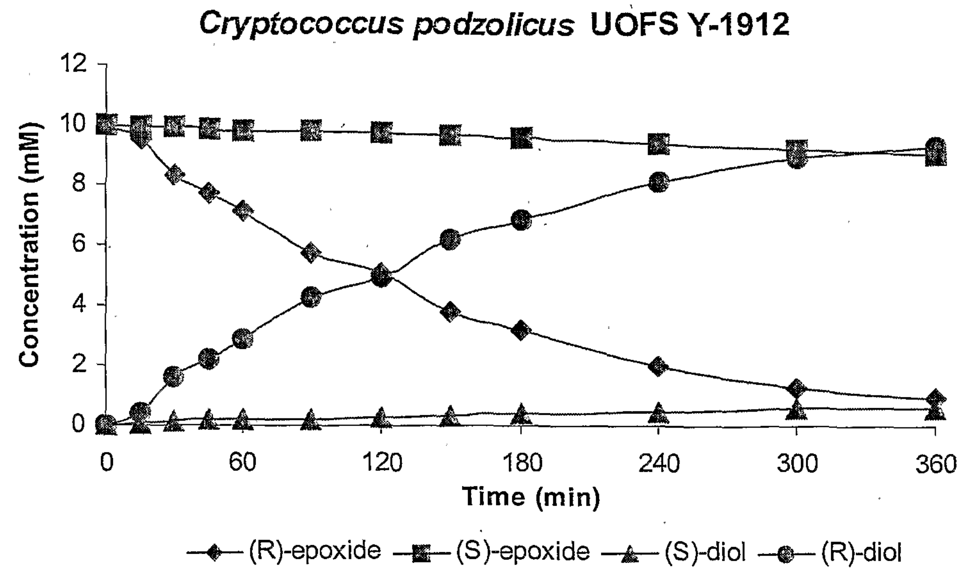


FIG 4A

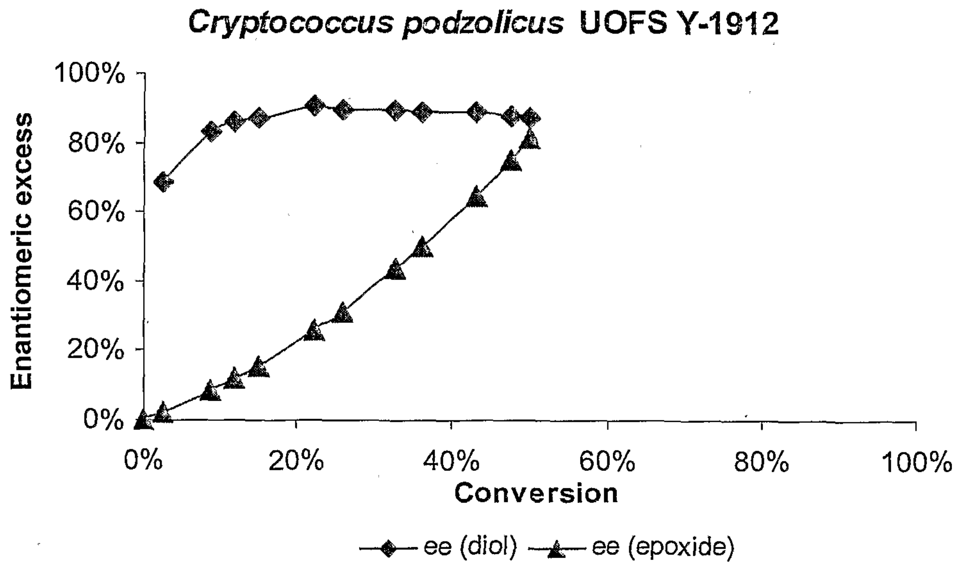


FIG 4B

Sample 214

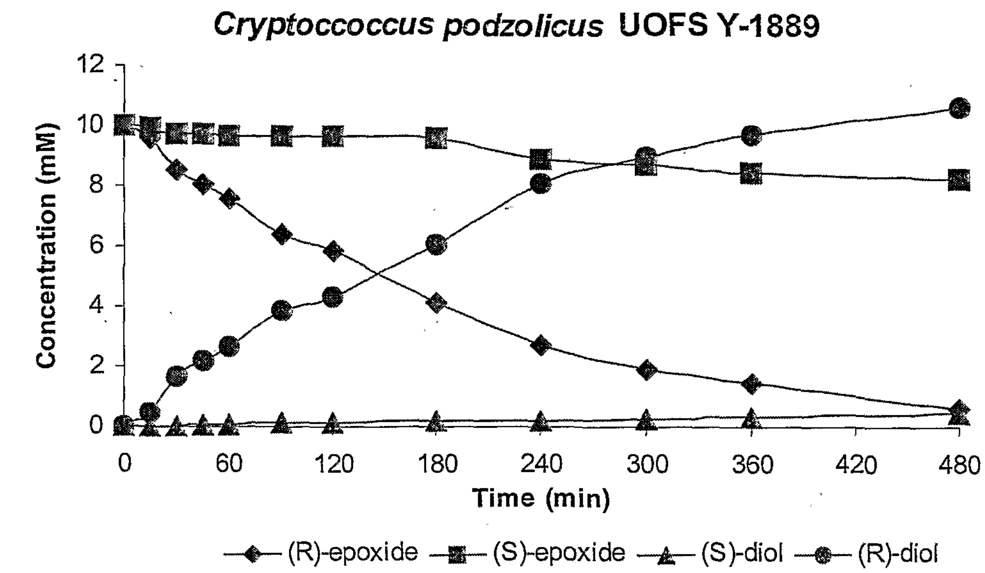


FIG 5A

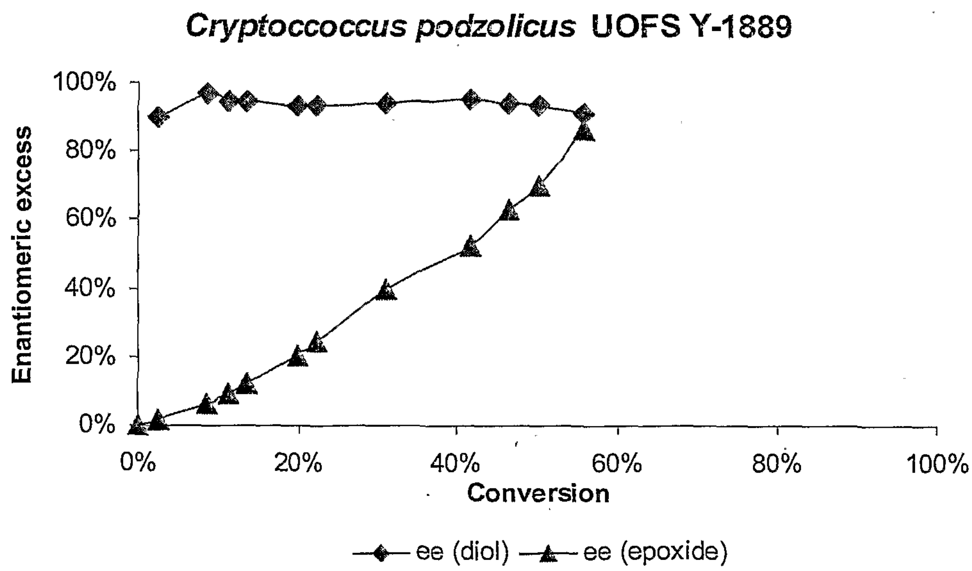


FIG 5B

Sample 215

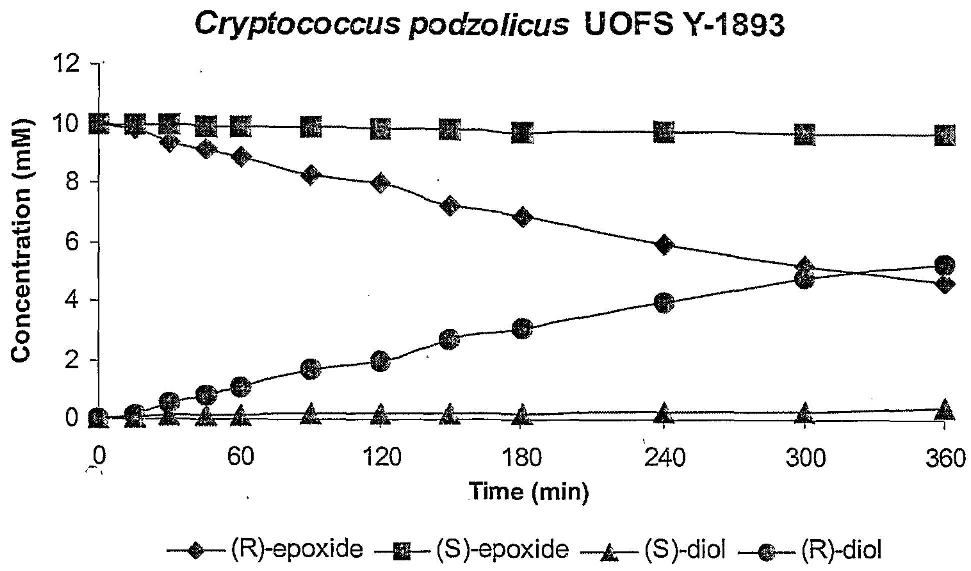


FIG 6A

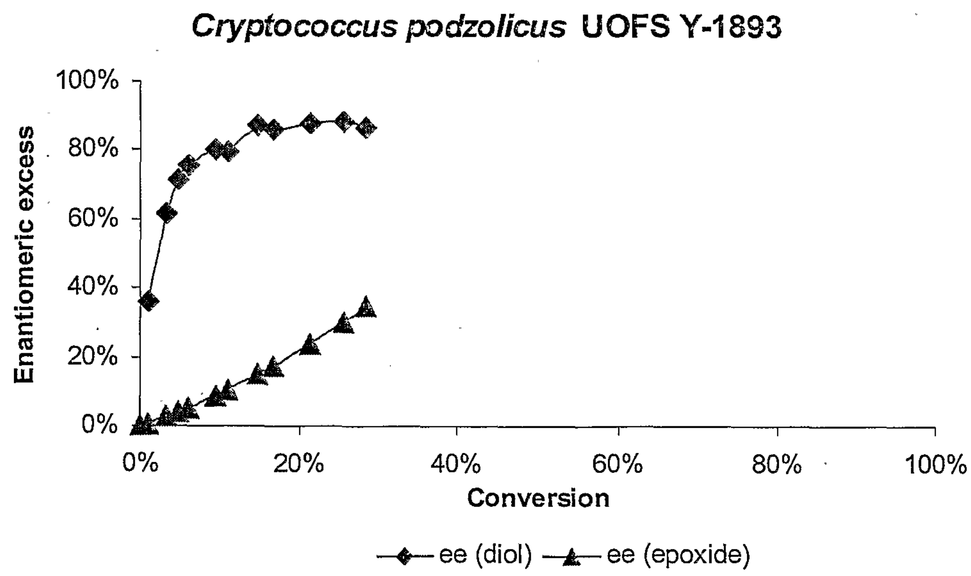


FIG 6B

Sample 216

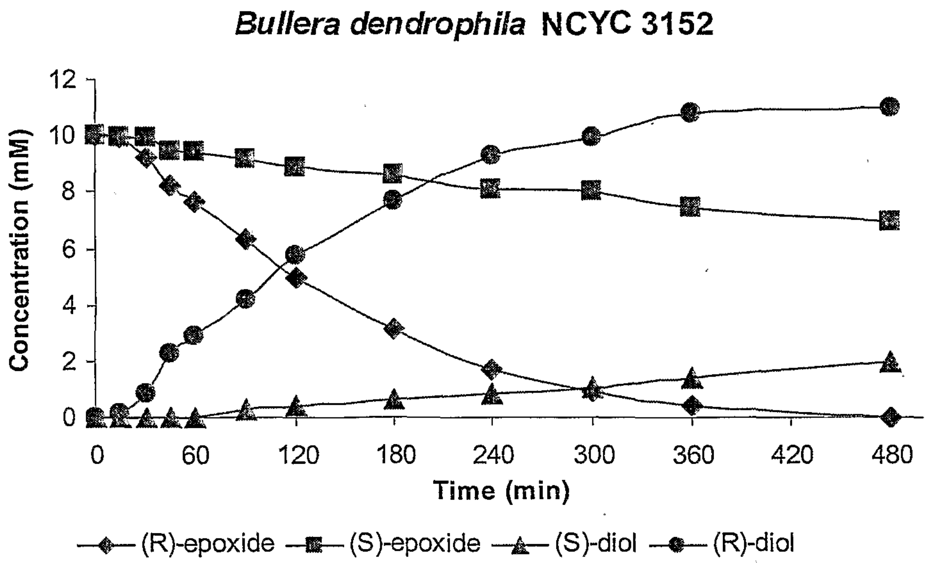


FIG 7A

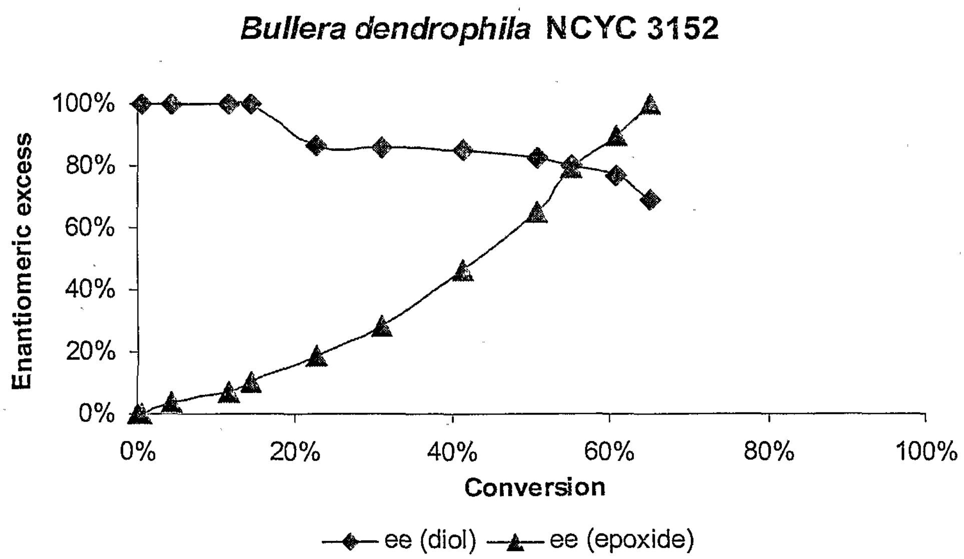


FIG 7B

<u>Sample 217</u>

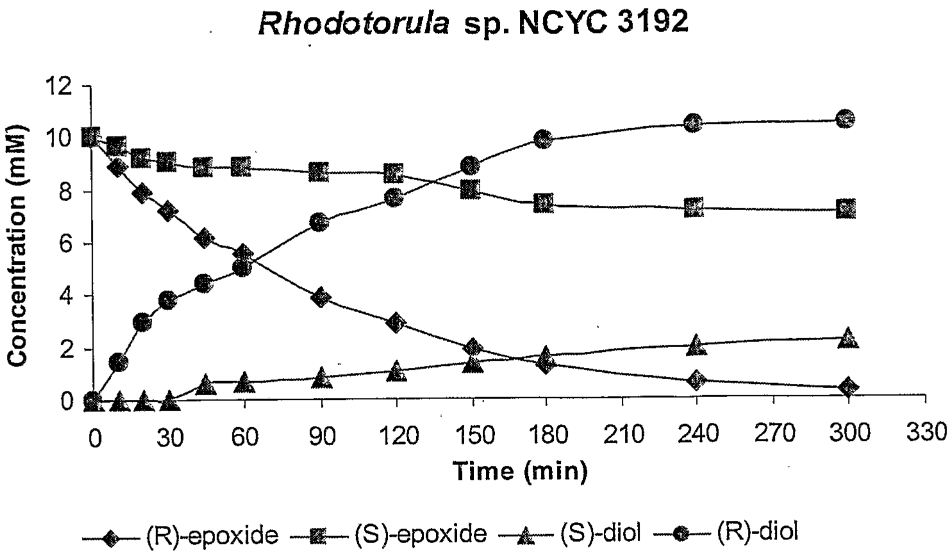


FIG 8A

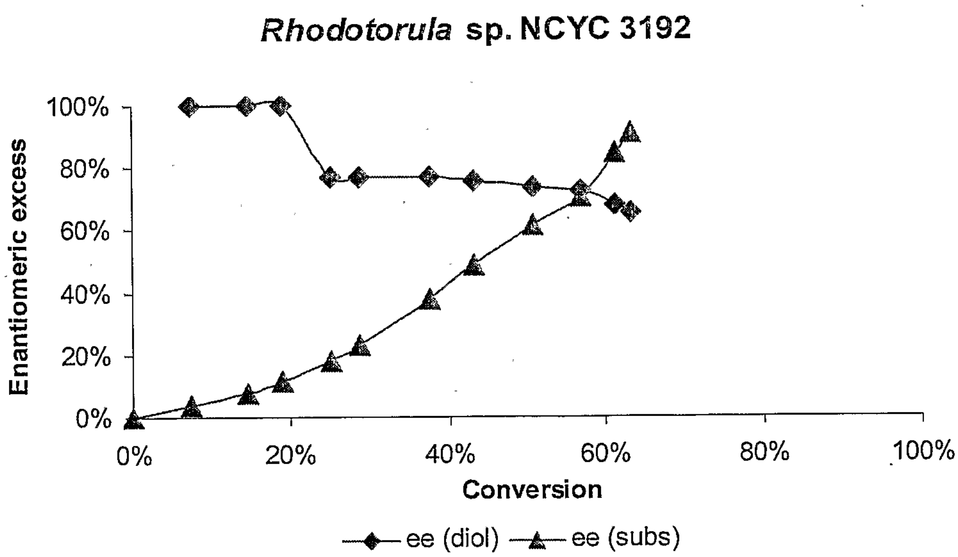


FIG 8B

Sample 218

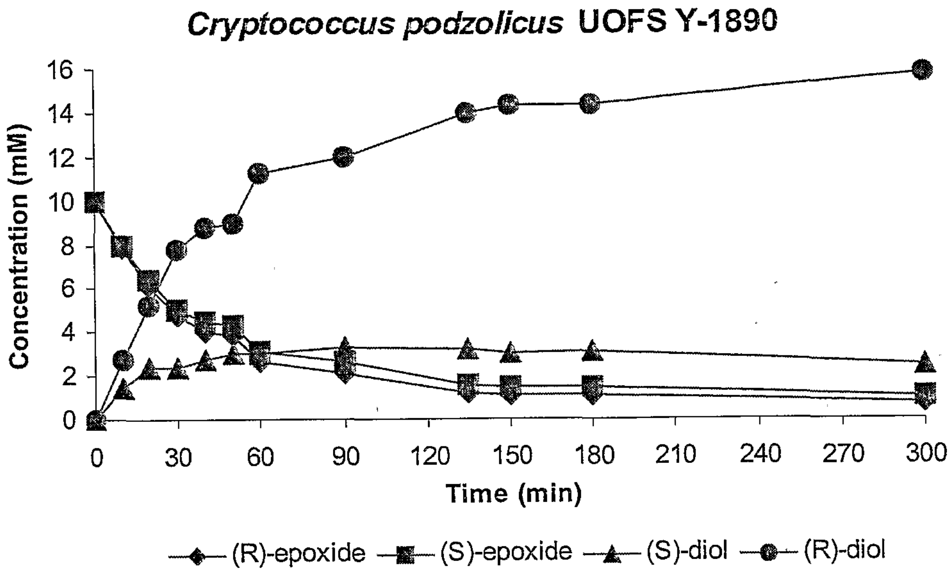


FIG 9A

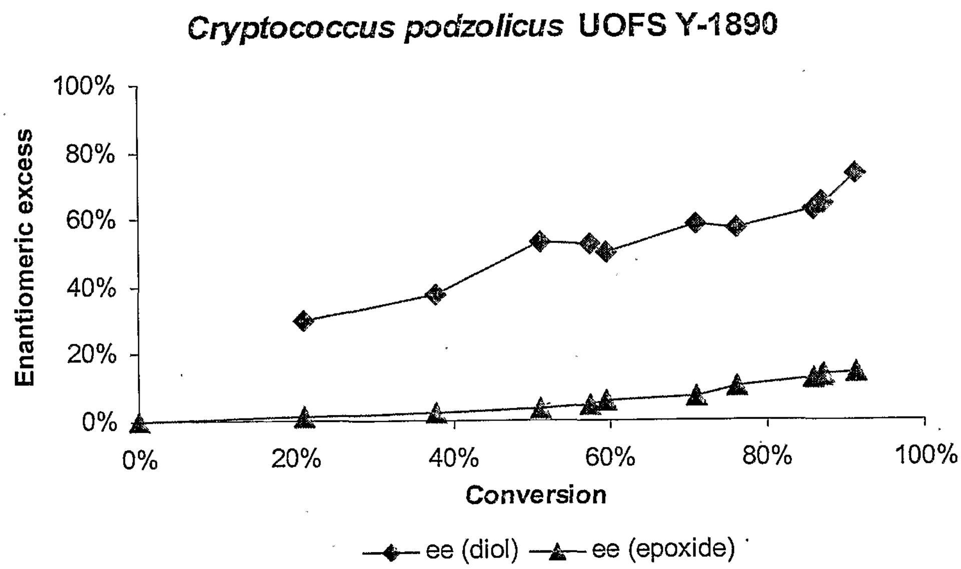


FIG 9B

Sample 219

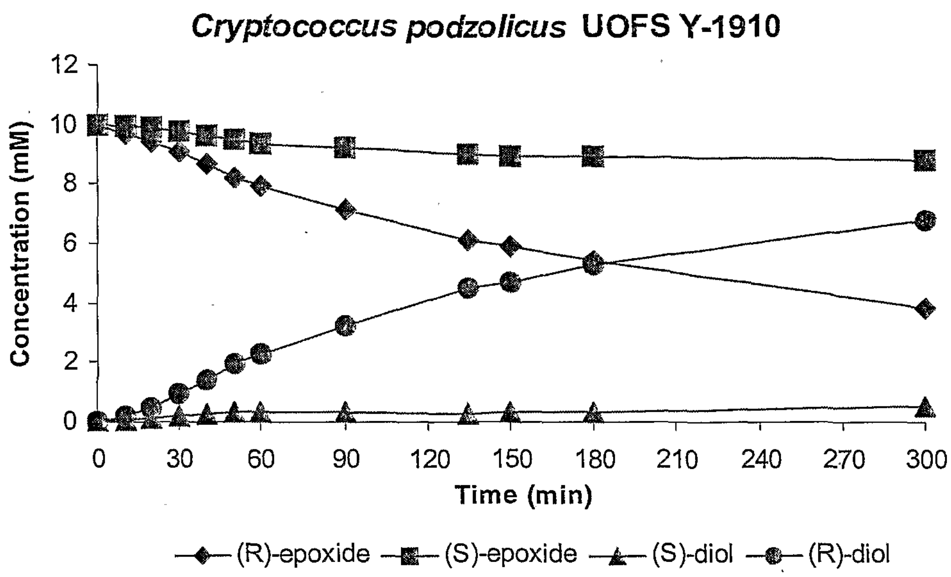


FIG 10A

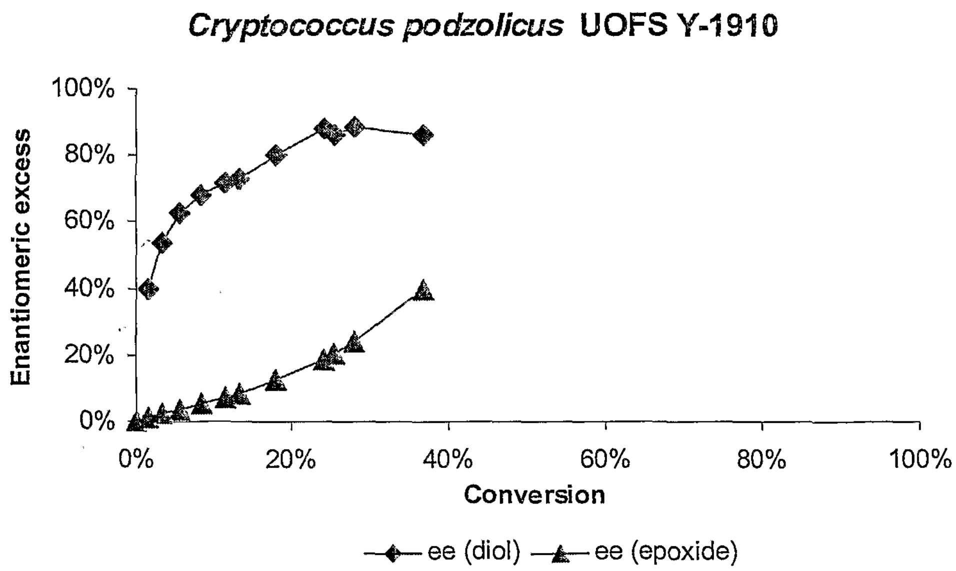


FIG 10B

<u>Sample 220</u>

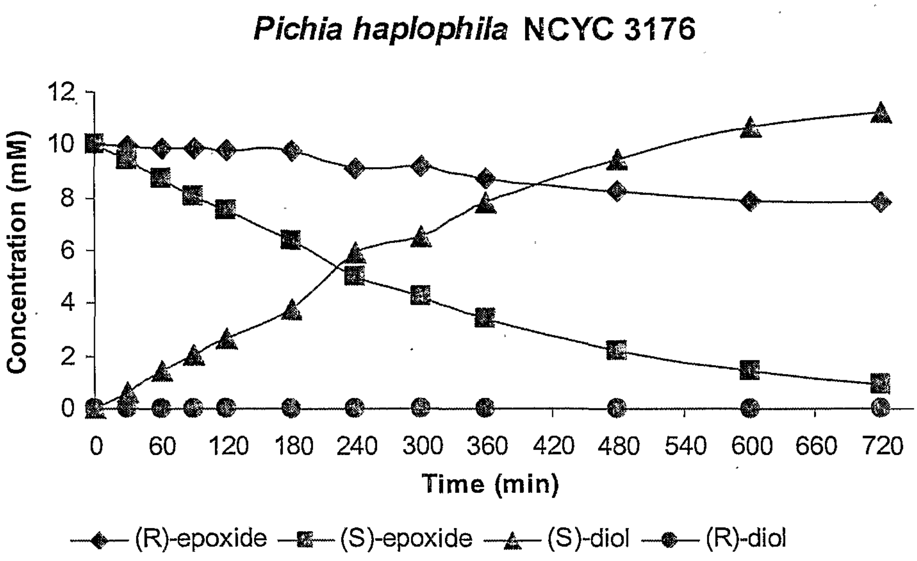


FIG 11A

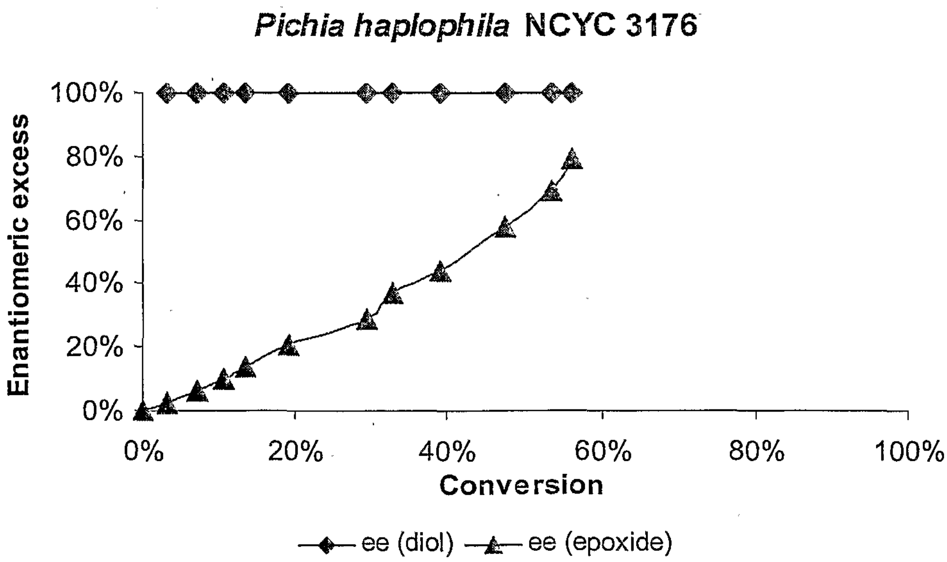


FIG 11B

Sample 221

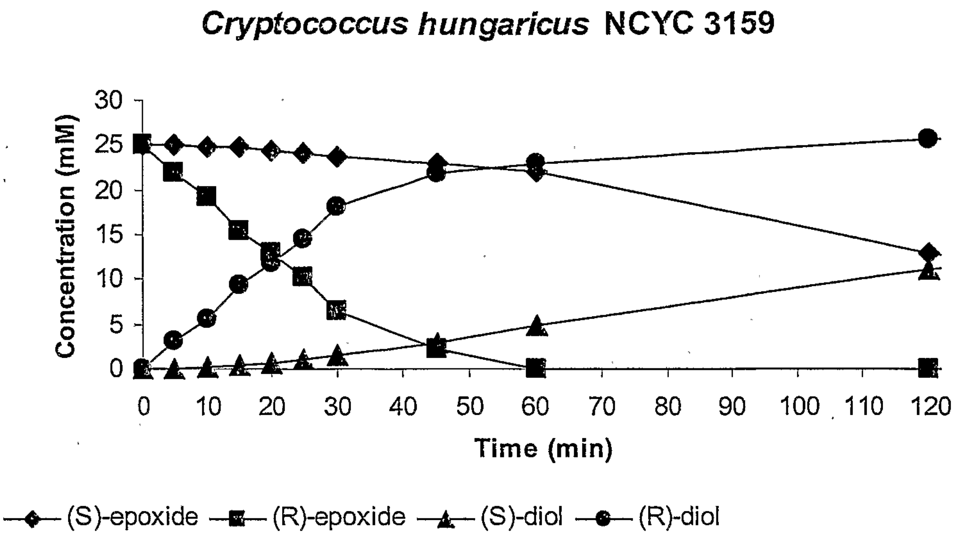


FIG 12A

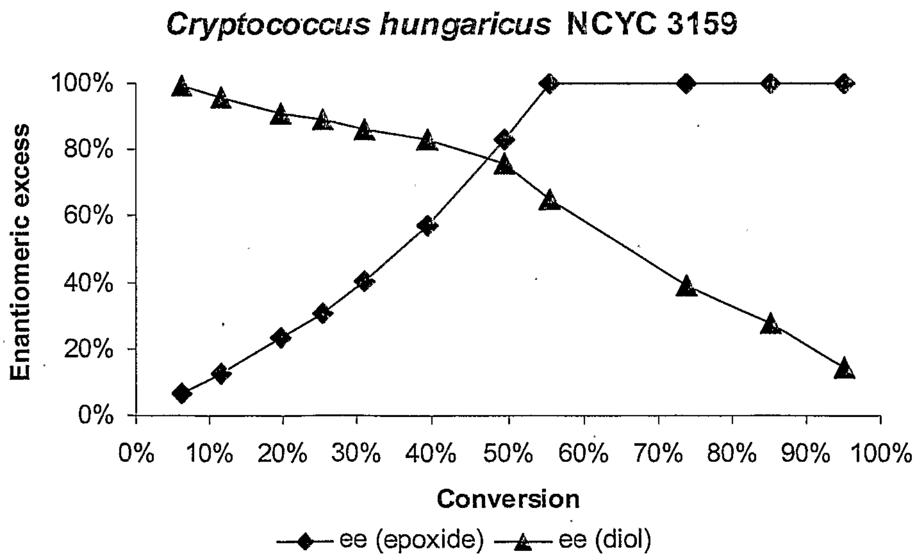


FIG 12B

Sample 222

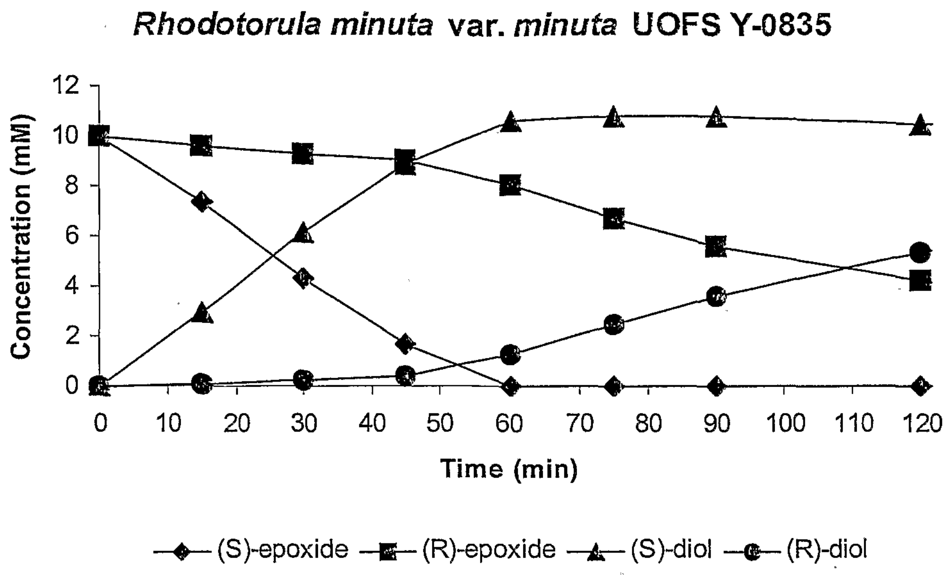


FIG 13A

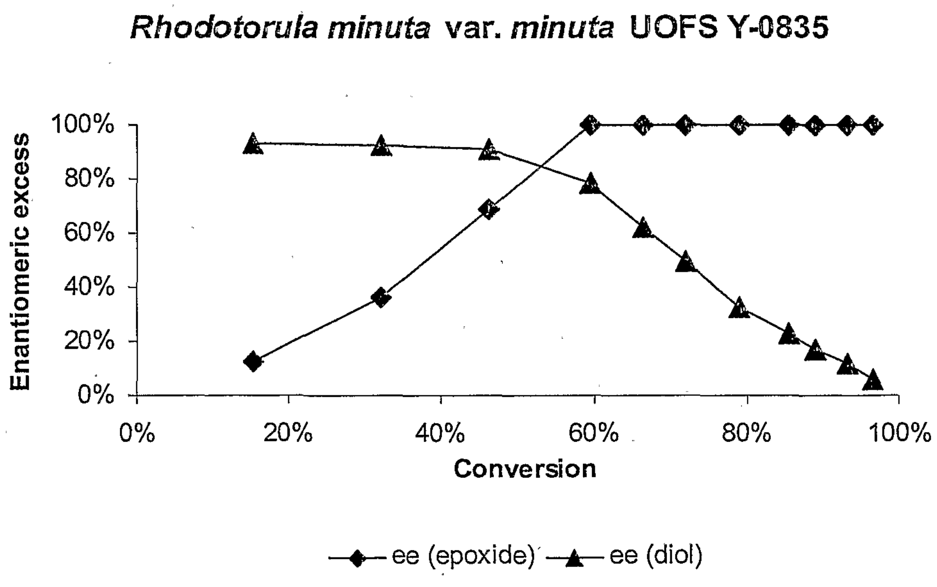


FIG 13B

Sample 223

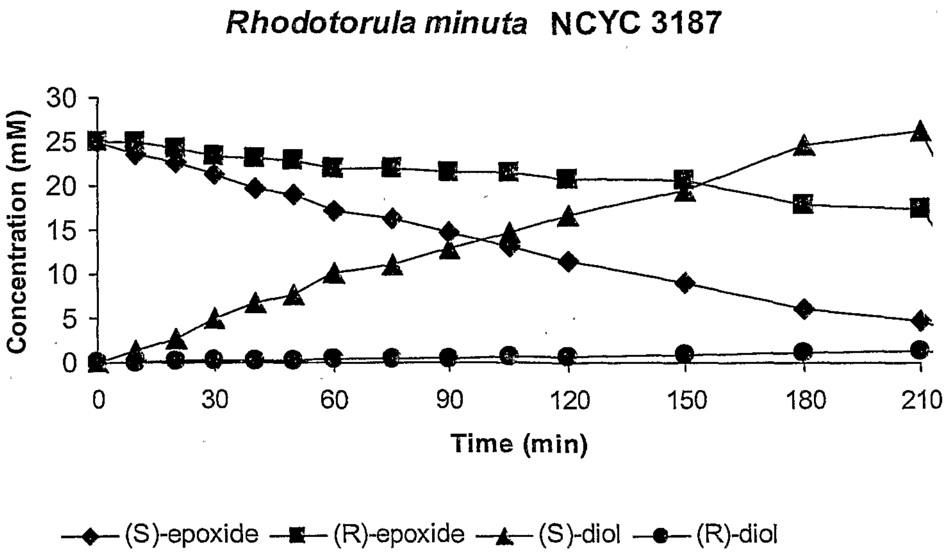


FIG 14A

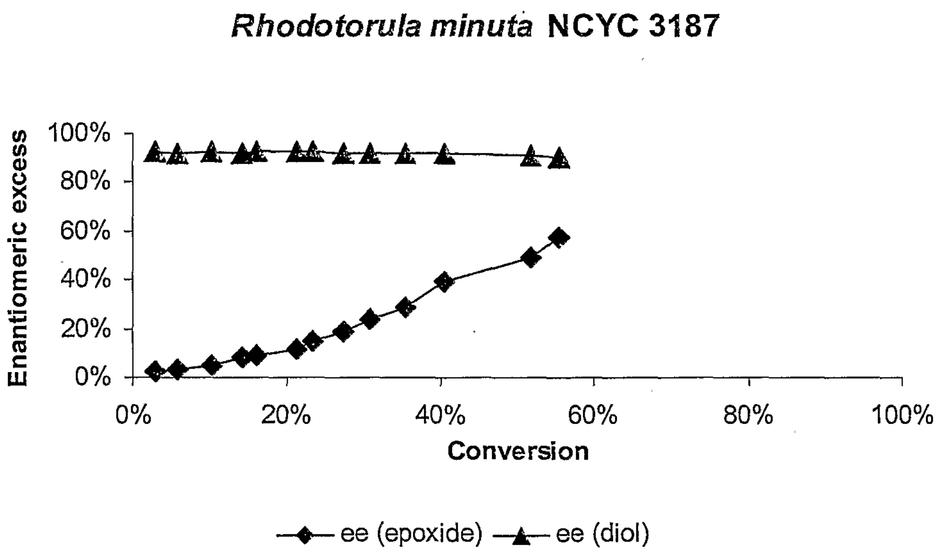


FIG 14B

**Sample 224.**

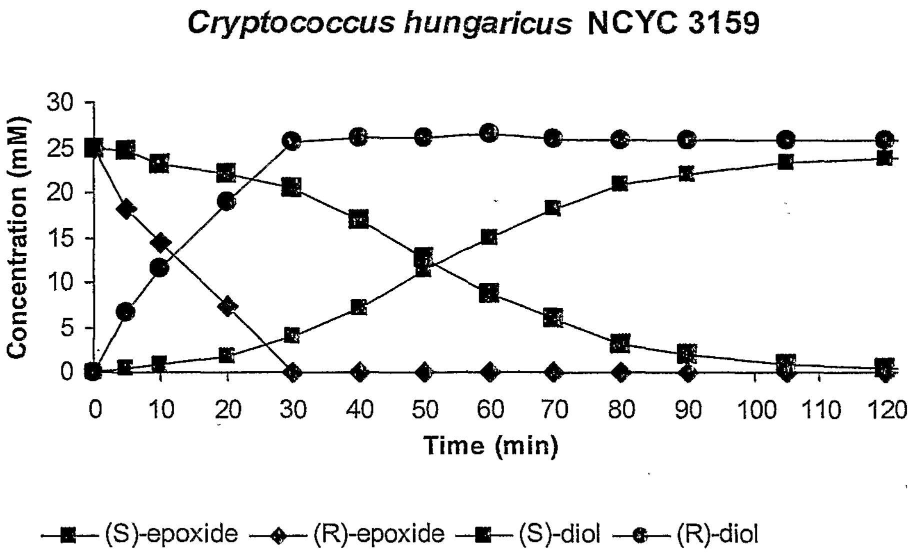


FIG 15A

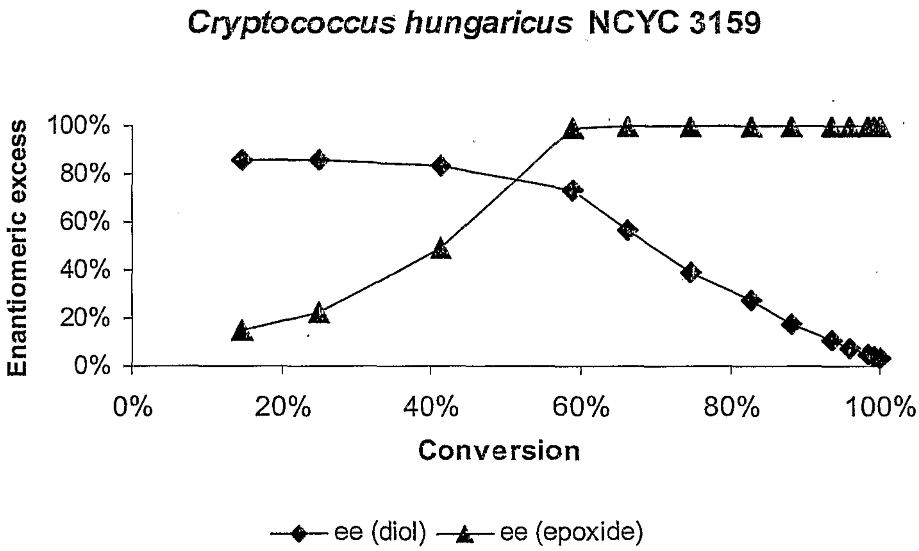


FIG 15B

## Sample 225

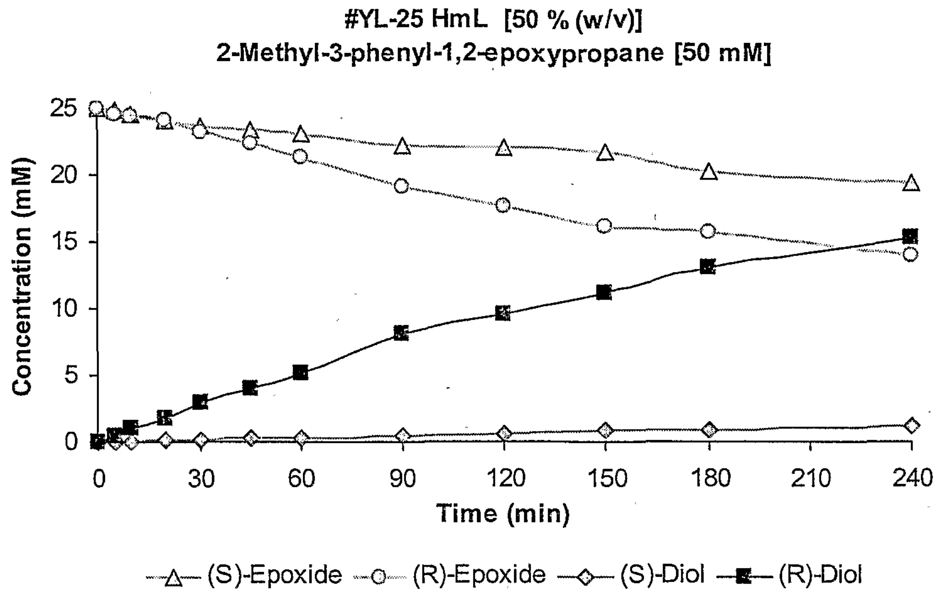


FIG 16

## Sample 226

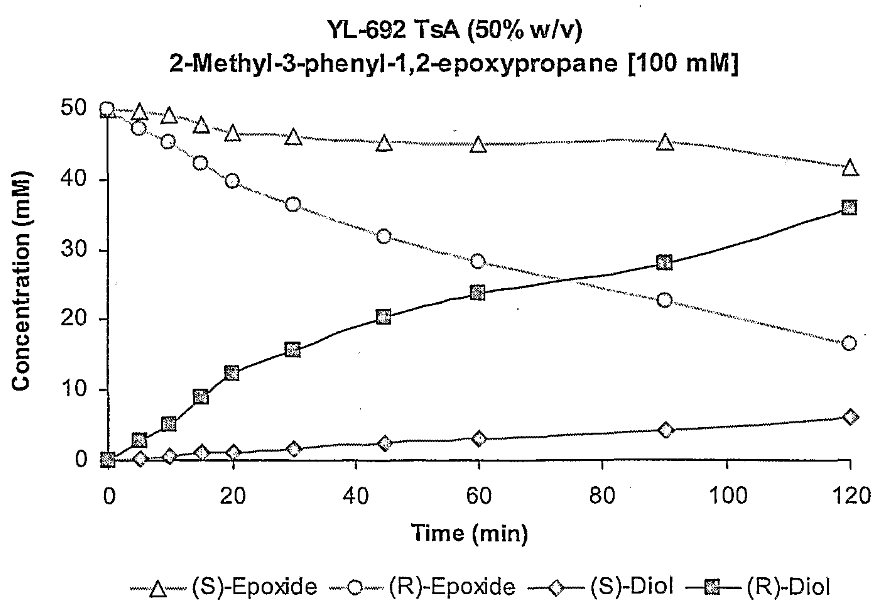


FIG 17

Sample 227

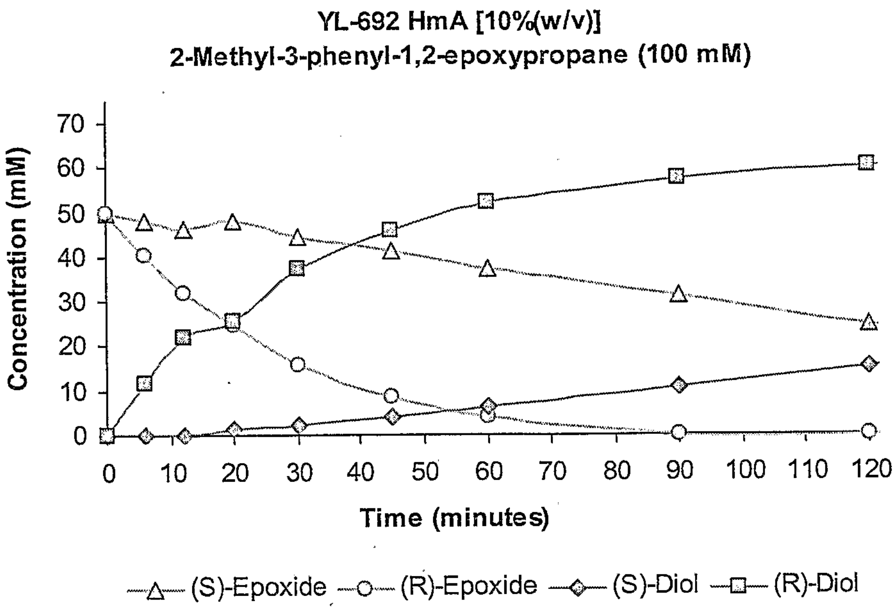


FIG 18

Sample 228

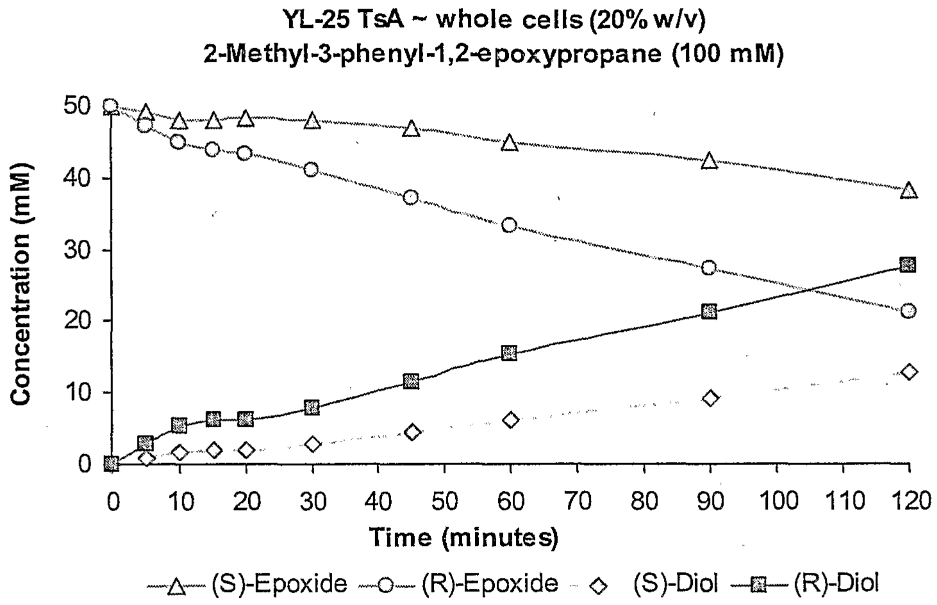


FIG 19 A

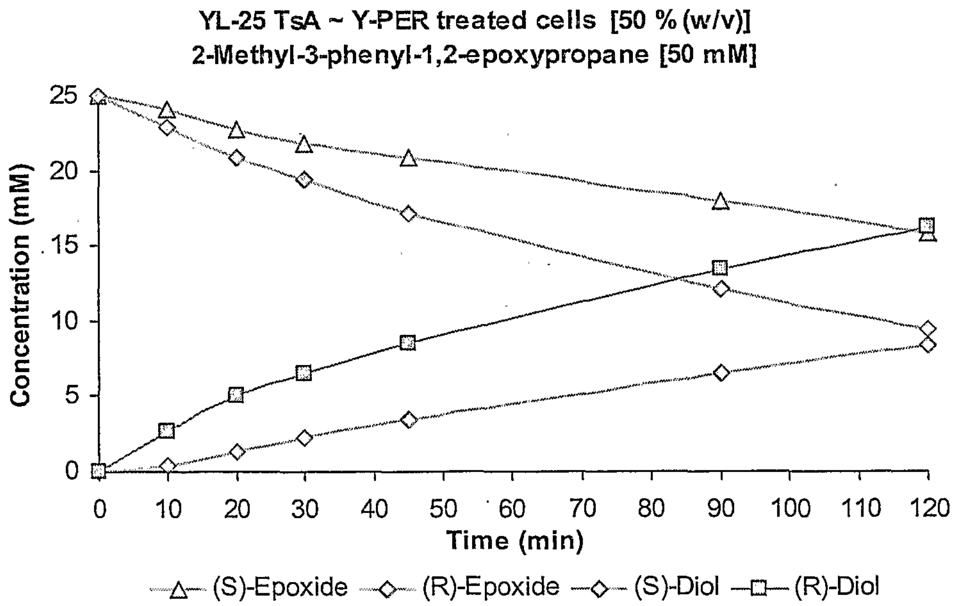


FIG 19 B

FIG 20

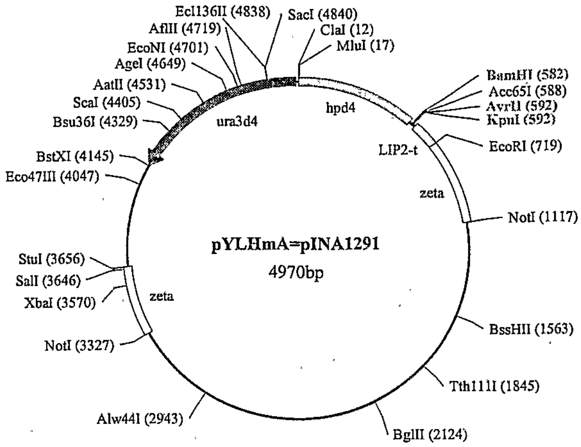

Ecl136II (4838)
SacI (4840)
AflII (4719)
ClaI (12)
EcoNI (4701)
MluI (17)
AgeI (4649)
AatII (4531)
BamHI (582)
Acc65I (588)
ScaI (4405)
AvrII (592)
Bsu36I (4329)
KpnI (592)
ura3d4
hpd4
BstXI (4145)
LIP2-t
EcoRI (719)
Eco47III (4047)
zeta
NotI (1117)
pYLHmA=pINA1291
4970bp
StuI (3656)
SalI (3646)
zeta
XbaI (3570)
BssHII (1563)
NotI (3327)
Tth111I (1845)
Alw44I (2943)
BglII (2124)


FIG 21

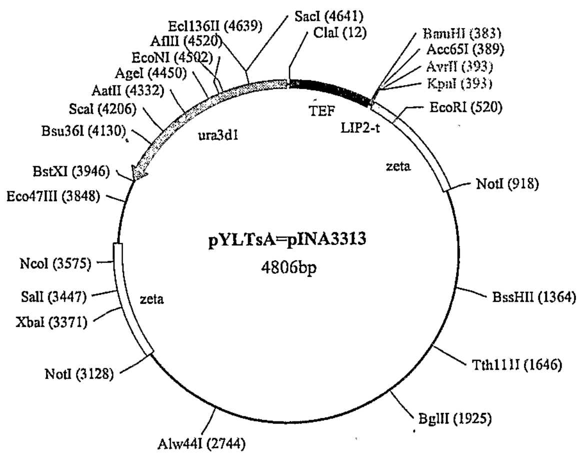

Ecl136II (4639)
SacI (4641)
AflII (4520)
ClaI (12)
EcoNI (4502)
BamHI (383)
AgeI (4450)
Acc65I (389)
AatII (4332)
AvrII (393)
KpnI (393)
ScaI (4206)
TEF
EcoRI (520)
Bsu36I (4130)
LIP2-t
ura3d1
BstXI (3946)
zeta
NotI (918)
Eco47III (3848)
pYLTsA=pINA3313
4806bp
NcoI (3575)
SalI (3447)
zeta
BssHII (1364)
XbaI (3371)
NotI (3128)
Tth111I (1646)
BglII (1925)
Alw44I (2744)

SEQ. ID. NO. 1

***Rhodosporidium toruloides NCYC 3181* (#1)**

Amino acid sequence

```
ORIGIN
1       MATHTFASPP TRFTVDIPQS ELDELDFRLD KTRWPATEIV PEDGADDPTA FGLGAGPTLP
61      LMKELAKGWR EFDWKKAQDH LNTFEHYTVE IEDLSIHFLH HRSTRPNAVP LILCHGWPGH
121     FGEFLNVIPL LTEPSDPSAQ AFHVVAPSMP GYAWSSPPPS SKWSMPDTAR VFDKLMTGLG
181     YEKYMAQGGD WGSIAARCLG SLHKDHCKAV HLNFLPVFPP VPMWLINPHT LLAWAPRFLV
241     PEKQAARMKR GLAYLEKGSA YYVMQQLTPR TPAYGLTDSP VGLLAWIGEK FEPTIQEASK
301     QAQPTLTRDE LYFTCSLYWF TRSIGTSFLP YSLNPHFTTF LTDSSTTCPT LPCPSTRARS
361     TARQNGTLSV PATSSDQGRA GGRTLCCARE ARRVCRPSQG GVRRHVGEV
```

**FIG 22**

SEQ. ID. NO. 2

***Rhodosporidium toruloides UOFS Y-0471* (#46)**

Amino acid sequence

```
ORIGIN
1       MKMATHTFAS PPTRFTVDIP QSELDELHSR LDKTRWPATE IVPEDGTDDP TAFGLGAGPT
61      LPLMKELAKG WREFDWKKAQ DHLNTFEHYM VEIEDLSIHF LHHRSTRPNA VPLILCHGWP
121     GHFGEFLNVI PLLTEPSDPS AQAFHVVAPS MPGYAWSLPP PSSKWNMPDT ARVFDKLMTG
181     LGYEKYMAQG GDWGSIAARC LGSLHKDHCK AVHLNFLPVF PPVPMWLINP HTLLAWAPRF
241     LVPEKQAARM KRGLAYLEKG SAYYVMQQLT PRTPAYGLTD SPVGLLAWIG EKFEPTIQEA
301     SKQAQPTLTR DELYFTCSLY WFTRSIGTSF LPYSLNPHFT TFLTDSKYHL PNFALSLYPG
361     EIYCPAERDA KRTGNLKWIK DAPEGGHFAA LEKPDVFVEH LREAFGVMWE K
```

**FIG 23**

SEQ. ID. NO. 3

*Rhodotorula araucariae NCYC 3183* (#25)

Amino acid sequence

ORIGIN

1 MSEHSFEAPP QPFTVDFAPH IEDLHRRLDN ARWPTQEIVP VDVSETEHHN AFGLGMGPQL
61 NLMKELANGW RAFDQSALQD HLNSFNNWKV EIEGLSIHFL HHRSTRAGAL PLILCHGWPG
121 GYHEFLHVVQ LLTEPEGADA QAFHLVVPSM PGYAFSSPPP TAKWGMEDTA RVFDKLMTGL
181 GYNKYVAQGG DWGSITARCL GALHKDHCIA VHLNFCPVPP PFPFDQFNPR TLLNWMPRFV
241 ISDQQRAKLE RGLAYLEKGS AYYVMQQLTP RTPAYALNDS PIGLLAWIGE KMIPGINEAS
301 AQPNPTLNRD ALYTTLSLYW FTNSIGTSFL PYSLNPHFST FLTSPRYRLP NFALSSFPGE
361 LFSPTPRDAA RTGVMRWYKE ADDGGHFAAL EKPDVFSQHV REAVKAMLSS

**FIG 24**

SEQ. ID. NO. 4

*Cryptococcus curvatus NCYC 3158* (Car 054)

Amino acid sequence

ORIGIN

1 MATHTFASPP TRFTVDIPQS EVDQLHSRLD KTRWPGTEIV PEDGADDPTA FGLGAGPTLP
61 LMKELAKGWR DFDWKKAQDH LNTFEHYTVE IEDLSIHFLH HRSTRPNAVP LILCHGWPGH
121 FGEFLHVIPL LTEPSDPSAQ AFHVVAPSMP GYAWSSPPPT SKWNMPDTAR VSDKLMNGLG
181 YEKYMAQGGD WGSIAARCLG ALHKDHCKAV HLNFLPVFPP VPMWLINPHT LLAWAPRFLV
241 PEKQAARMKR GLAYLEKGSA YYVMQQLTPR TSAYGLTDSP VGLLAWIGEK FEPTIQEASK
301 QAQPTLTRDE LYFTCSLYWF TRSIGTSFLP YSLNPHFTTF LTDSKYYLPN FALSLYPGEI
361 YCPAERDAKR TGNLKWIKDA PEGGHFAALE KPDVFVDHLR EAFGVMWEK

**FIG 25**

SEQ. ID. NO.5

***Rhodosporidium paludigenum*** **NCYC 3179 ( #692)**

Amino acid sequence

ORIGIN

```
1       MAAHSFTAPP APYNIDFAPQ VNDLHRRLDA ARWPEHDVVP DDVDHGEHGA FGLGAGPSLA
61      LMKELAQEWR GQDQKQLQDH LNSYKNYRVE IEGLNIHFLH YPSSRADAFP LILCHGWPGG
121     YHEFLHVLER LTEPEDQGSR AFHVVVPSMP GYAFSSPPKT AKWGMEDTAR VFDKLMTGLG
181     YAKYAAQGGD WGSITARCLG SLHKENCVAV HLNFCPVPPP FPLNMFNPRT LLDWMPRFVL
241     PDQRRAKIER GVAYIERGSA YYAMQNLTPR TPAYGLNDSP IGLLAWIGEK MIPGIDKAVK
301     HPNATLNREA LFTTLSIYWF TGSIGSSFLP YALNPHFSTF LVSPRHQLPN FALSNFPDEL
361     FTPEERDARR TGNLRWYKDA EDGGHFAALE KPEVFAEHVR EAMGVLLSNQ A
```

**FIG 26**

SEQ. ID. NO. 6

***Debaromyces hansenii NCYC 3167*** **(#113)**

Amino acid sequence

ORIGIN

```
1       MMQGQYKIQL RNGVRIFSTF SNYDNHDVFC SGGERKWTRV IFLLHGFPDN NSSYNSVWTV
61      ILDSVPEDEK VLLLAPSMRG YEPSSQGEQC DYKMSDLAGD VSSWIKNISP DGIPVHLVGH
121     DWGAIVAFKT ASMYPEMITS MACLAIPYIT NIRLWEFAWY FPDQIYHSSY MFTMQFGWLY
181     KKRLSDTSSN SYLDGLWRCW SPTWNYTVDE IDSVKRTLQE PGVIDASTAY YRCIANPLNI
241     SNRRWNVDFK KVPTLLLGGE RDGCMNEKLY RLEASKLANN PYVKVKTISN VGHFLHREDP
301     TKVGELITDW FSEYP*
```

**FIG 27**

SEQ. ID. NO. 7

*Rhodotorula minuta var. minuta* UOFS Y-0835 **(#712)**

Partial Amino acid sequence

```
ORIGIN
1       HWRFPSISFH YRSTRPERYS RSFFCHGWPG HFRLSFLNVI PLLTEPSDPS AQAFHVVAPS
61      MPGYAWYSPP PSSKWSMPDT ARVFDKLMTG LGYEKYMAQG GDWGSIAARC LGSLYKDHCK
121     AVHLNFLPVF PPVPMWLINP HTLLAWAPRF LVPEKQAARM KRGLAYLEKG SAYYVMQQLT
181     PRTPAYGLTD SPVGLLAWIG EKFEPTIQEA SKQAQPTLTR DELYFTCSLY WFARSIGTSF
241     LPYSLNPHFT TFLTDSRYHL PNFALSLYPG EIYCPAERDA KRTGNLKWIK DAPEGGHFAA
301     LEKPDVFVEH LREAFGVMWE K
```

**FIG 28**

SEQUENCE ID NO 8

***Rhodosporidium toruloides NCYC 3181* (#1)**

Nucleotide sequence

```
ORIGIN
1       ATGGCGACAC ACACATTCGC TTCGCCTCCC ACGCGCTTCA CCGTCGACAT CCCACAGTCA
61      GAACTCGACG AACTCGACTT CCGACTCGAC AAGACCCGCT GGCCGGCGAC AGAGATCGTT
121     CCAGAGGATG GGGCGGACGA CCCGACGGCG TTTGGGCTCG GAGCAGGGCC GACGCTGCCG
181     CTCATGAAGG AACTGGCGAA GGGTTGGCGC GAGTTCGACT GGAAGAAGGC GCAGGACCAC
241     CTCAACACCT TTGAGCACTA CACGGTCGAA ATCGAGGACC TCTCCATCCA CTTCCTCCAC
301     CACCGCTCGA CTCGCCCGAA TGCTGTTCCG CTCATCCTCT GCCACGGCTG GCCAGGCCAC
361     TTCGGCGAGT TCCTGAACGT CATACCGCTC TTGACGGAGC CGTCGGACCC GTCCGCCCAG
421     GCGTTCCACG TCGTCGCGCC TTCGATGCCC GGTTATGCTT GGTCTTCGCC TCCTCCGTCC
481     TCCAAGTGGA GCATGCCTGA TACCGCGAGG GTCTTCGACA AGCTCATGAC CGGGCTTGGC
541     TACGAGAAGT ACATGGCGCA GGGCGGAGAC TGGGGCAGCA TCGCTGCTCG CTGCCTTGGA
601     TCGCTTCACA AAGACCACTG CAAAGCCGTC CACCTCAACT TCCTCCCCGT CTTCCCACCC
661     GTCCCGATGT GGCTTATCAA CCCGCACACG CTCCTTGCCT GGGCACCGCG CTTCCTCGTG
721     CCGGAGAAGC AGGCTGCGCG TATGAAGCGC GGGTTGGCGT ACCTTGAGAA GGGCTCCGCC
781     TACTACGTCA TGCAGCAGTT GACGCCTCGC ACGCCTGCGT ACGGCCTGAC CGACAGTCCC
841     GTCGGCTTGC TGGCCTGGAT CGGCGAGAAG TTCGAGCCGA CCATTCAGGA GGCGAGCAAG
901     CAAGCCCAGC CGACCCTGAC TCGCGACGAG CTCTACTTCA CCTGCTCGCT CTACTGGTTC
961     ACCCGCTCAA TCGGCACCTC CTTCCTTCCC TACTCGCTCA ACCCGCACTT CACCACCTTC
1021    CTGACCGACA GCAGGTACCA CCTGCCCAAC TTTGCCCTGT CCCTCTACCC GGGCGAGATC
1081    TACTGCCCGG CAGAACGGGA CGCCAAGCGT ACCGGCAACC TCAAGTGGAT CAAGGACGCG
1141    CCCGATGGAG GACACTTTGC TGCGCTCGAG AAGCCCGATG TGTTTGTCGA GCATCTCAGG
1201    GAGGCGTTTG GCGTCATGTG GGAGAAGTAG
```

**FIG 29**

SEQUENCE ID NO 9

***Rhodosporidium toruloides UOFS Y-0471* (#46)**

Nucleotide sequence

ORIGIN

1 ATGGCGACAC ACACATTCGC TTCGCCTCCC ACCCGCTTCA CCGTCGACAT CCCACAGTCG
61 GAACTCGACG AACTTCACTC GCGACTCGAC AAGACCCGCT GGCCGGCGAC AGAGATCGTT
121 CCAGAGGATG GGACGGACGA TCCGACGGCG TTTGGGCTCG GAGCAGGGCC GACGCTGCCG
181 CTCATGAAGG AATTGGCGAA GGGTTGGCGC GAGTTCGACT GGAAAAAGGC GCAGGACCAC
241 CTCAACACCT TCGAGCACTA CATGGTCGAA ATTGAGGACC TCTCGATCCA CTTCCTCCAC
301 CATCGCTCGA CTCGCCCGAA CGCTGTTCCC CTCATCCTCT GCCACGGCTG GCCAGGCCAC
361 TTTGGCGAGT TCCTGAACGT TATCCCGCTC TTGACGGAGC CGTCGGACCC CTCCGCTCAG
421 GCGTTCCACG TCGTCGCCCC TTCGATGCCT GGCTATGCTT GGTCTTTGCC TCCTCCGTCC
481 TCCAAGTGGA ACATGCCTGA CACCGCGAGG GTCTTCGACA AGCTCATGAC CGGGCTTGGC
541 TACGAGAAGT ACATGGCGCA GGGCGGAGAC TGGGGAAGCA TCGCCGCTCG CTGCCTTGGA
601 TCGCTGCACA AGGACCATTG CAAAGCCGTC CACCTCAACT TCCTCCCCGT CTTCCCACCC
661 GTCCCGATGT GGCTTATCAA CCCGCACACG CTCCTTGCCT GGGCACCGCG CTTCCTCGTG
721 CCGGAGAAGC AGGCTGCGCG TATGAAGCGC GGGTTGGCGT ACCTTGAGAA GGGCTCCGCC
781 TACTACGTCA TGCAGCAGTT GACGCCTCGC ACGCCTGCGT ACGGCCTGAC CGACAGTCCC
841 GTCGGCTTGC TGGCCTGGAT CGGCGAGAAG TTCGAGCCGA CCATTCAGGA GGCGAGCAAG
901 CAAGCCCAGC CGACCCTGAC TCGCGACGAG CTCTACTTCA CCTGCTCGCT CTACTGGTTC
961 ACCCGCTCAA TCGGCACCTC CTTCCTTCCC TACTCGCTCA ACCCGCACTT CACCACCTTC
1021 CTGACCGACA GCAAGTACCA CCTGCCCAAC TTTGCCCTCT CGCTTTACCC AGGCGAGATC
1081 TACTGCCCCG CCGAGCGGGA CGCCAAGCGC ACCGGCAACC TCAAGTGGAT CAAGGACGCG
1141 CCTGAGGGAG GACACTTTGC TGCGCTCGAA AAGCCGGATG TGTTTGTCGA GCACCTCAGG
1201 GAGGCGTTTG GCGTCATGTG GGAGAAGTAG

**FIG 30**

SEQUENCE ID NO 10

***Rhodotorula araucariae NCYC 3183* (#25)**

Nucleotide sequence

```
ORIGIN
1       ATGAGCGAGC ACAGCTTCGA GGCCCCGCCA CAGCCGTTTA CGGTCGACTT TGCTCCCCAC
61      ATCGAGGATC TCCACCGCCG TCTCGACAAT GCGCGCTGGC CGACGCAAGA GATTGTCCCC
121     GTCGACGTGT CCGAGACGGA GCATCACAAC GCGTTCGGAC TCGGGATGGG CCCGCAGCTC
181     AACCTTATGA AGGAGCTCGC CAACGGCTGG CGCGCGTTCG ACCAGTCGGC GCTCCAGGAC
241     CACCTCAACA GCTTCAACAA CTGGAAGGTC GAGATCGAGG GATTGTCGAT CCACTTCCTC
301     CACCATCGCT CGACGCGCGC CGGCGCTCTC CCGCTCATCC TGTGCCATGG CTGGCCCGGC
361     GGGTACCACG AGTTCCTCCA CGTCGTCCAG CTCCTCACCG AACCAGAGGG GGCGGATGCG
421     CAGGCGTTTC ACCTCGTCGT CCCCTCGATG CCCGGGTACG CCTTCTCGTC TCCGCCGCCG
481     ACGGCCAAGT GGGGCATGGA AGACACTGCA AGGGTTTTTG ACAAGCTCAT GACCGGTTTG
541     GGGTACAACA AGTATGTCGC GCAGGGCGGT GACTGGGGGT CCATCACGGC GCGATGCCTC
601     GGCGCGCTGC ACAAGGACCA CTGCATTGCT GTCCACCTCA ACTTCTGCCC CGTCCCGCCG
661     CCGTTCCCAT TCGACCAGTT CAACCCGCGC ACGCTGCTCA ACTGGATGCC GCGCTTCGTG
721     ATCTCGGACC AGCAGCGTGC GAAGCTCGAG CGTGGGCTGG CGTACCTCGA GAAGGGGTCT
781     GCTTACTATG TCATGCAGCA GCTCACACCG CGTACCCCGG CCTACGCTCT CAATGACAGC
841     CCGATTGGCC TGCTCGCCTG GATTGGCGAA AAGATGATCC CAGGCATCAA CGAGGCGAGC
901     GCGCAGCCGA ACCCGACGCT CAATCGCGAT GCGTTGTACA CCACGCTCTC GCTGTACTGG
961     TTCACCAACT CCATCGGCAC CTCTTTCCTC CCCTACTCGC TTAACCCGCA CTTCAGCACG
1021    TTCCTCACCT CGCCCCGCTA TCGCCTGCCG AACTTTGCGC TGTCTTCCTT CCCGGGCGAG
1081    CTGTTCTCGC CGACGCCGCG CGATGCTGCG AGGACGGGCG TGATGCGCTG GTACAAGGAG
1141    GCGGACGATG GCGGGCACTT TGCGGCGCTC GAGAAGCCCG ATGTGTTCAG CCAGCATGTC
1201    AGGGAGGCAG TCAAGGCCAT GCTGTCGTCG TGA
```

**FIG 31**

SEQUENCE ID NO 11

*Cryptococcus curvatus NCYC 3158* (Car 054)

Nucleotide sequence

ORIGIN

1 ATGGCGACAC ACACATTCGC TTCGCCTCCC ACCCGCTTCA CCGTCGACAT TCCGCAGTCG
61 GAAGTTGACC AACTTCACTC GCGACTCGAT AAGACTCGCT GGCCAGGGAC AGAGATCGTT
121 CCAGAGGATG GGGCGGACGA CCCGACGGCG TTTGGACTCG GAGCAGGACC AACGCTGCCG
181 CTCATGAAGG AACTGGCGAA GGGTTGGCGC GACTTCGACT GGAAGAAGGC GCAGGACCAC
241 CTCAACACCT TCGAGCACTA CACGGTGGAG ATCGAGGACC TCTCGATCCA CTTCCTCCAC
301 CATCGCTCGA CTCGTCCGAA TGCTGTTCCC CTCATCCTCT GCCACGGCTG GCCAGGACAC
361 TTCGGCGAGT TTCTACACGT TATACCACTC TTGACGGAGC CGTCGGACCC GTCCGCTCAG
421 GCGTTTCACG TCGTCGCGCC TTCGATGCCT GGTTATGCTT GGTCTTCGCC TCCTCCTACC
481 TCCAAGTGGA ATATGCCTGA CACCGCAAGG GTGTCCGACA AGCTTATGAA CGGACTCGGC
541 TACGAGAAGT ACATGGCGCA GGGCGGAGAC TGGGGCAGCA TCGCCGCTCG CTGCCTCGGA
601 GCGCTGCACA AAGATCACTG CAAAGCCGTC CACCTCAACT TCCTCCCCGT CTTCCCGCCC
661 GTCCCTATGT GGCTCATCAA CCCACACACA CTCCTCGCAT GGGCTCCGCG CTTCCTTGTG
721 CCGGAGAAGC AGGCTGCGCG CATGAAGCGC GGGTTGGCGT ACCTCGAAAA GGGCTCCGCC
781 TACTACGTCA TGCAGCAATT GACGCCTCGC ACGTCTGCGT ACGGCCTTAC TGACAGTCCC
841 GTCGGCTTGC TGGCCTGGAT CGGCGAGAAG TTTGAGCCGA CCATTCAGGA AGCGAGCAAG
901 CAAGCCCAGC CGACCCTCAC TCGCGACGAG CTCTACTTCA CCTGCTCCTT GTACTGGTTC
961 ACCCGCTCAA TCGGCACCTC CTTCCTGCCC TACTCGCTCA ACCCGCACTT CACGACCTTC
1021 CTGACCGACA GCAAGTATTA CCTGCCCAAT TTTGCCCTCT CCCTGTACCC GGGCGAGATC
1081 TACTGCCCTG CCGAGCGGGA CGCCAAGCGC ACTGGCAACC TCAAGTGGAT CAAGGACGCG
1141 CCTGAGGGAG GACACTTTGC GGCGCTCGAG AAGCCCGACG TGTTTGTCGA CCATCTCAGG
1201 GAGGCATTTG GCGTTATGTG GGAGAAGTAG

**FIG 32**

SEQUENCE ID NO 12

***Rhodosporidium paludigenum*** NCYC 3179 ( #692)

Nucleotide sequence

ORIGIN

1 ATGGCTGCCC ATTCCTTTAC TGCACCTCCT GCACCCTACA ACATCGACTT TGCGCCCCAG
61 GTAAATGACC TGCACCGCCG TCTCGATGCT GCCCGCTGGC CGGAACACGA CGTGGTGCCC
121 GACGATGTGG ATCACGGAGA GCACGGCGCA TTCGGACTCG GCGCTGGTCC CAGCCTCGCC
181 CTCATGAAGG AGCTCGCGCA GGAATGGAGG GGCCAGGACC AGAAGCAGCT GCAGGACCAC
241 CTCAACTCCT ACAAGAACTA TCGCGTCGAG ATCGAGGGTC TCAACATCCA CTTCCTGCAC
301 TACCCGTCGT CTCGCGCCGA TGCGTTCCCG CTCATCCTGT GCCACGGCTG GCCTGGCGGC
361 TACCACGAGT TCCTGCACGT CCTAGAGCGC CTCACGGAGC CCGAGGATCA GGGGTCGCGG
421 GCCTTCCATG TCGTCGTGCC TTCCATGCCG GGTTACGCCT TCTCCTCGCC GCCCAAGACG
481 GCAAAATGGG GCATGGAGGA CACGGCTCGC GTGTTCGACA AGCTCATGAC GGGGCTAGGT
541 TACGCCAAGT ATGCGGCCCA AGGCGGTGAC TGGGGGTCTA TCACGGCGCG CTGCCTAGGT
601 TCGCTGCACA AGGAGAACTG CGTCGCTGTC CACCTCAACT TCTGCCCGGT TCCCCCGCCG
661 TTCCCGCTCA ACATGTTCAA CCCGCGCACA CTTCTGGACT GGATGCCTCG CTTTGTCCTG
721 CCTGATCAAC GGCGGGCCAA GATTGAGCGC GGCGTGGCCT ATATCGAGCG CGGCTCTGCC
781 TACTACGCCA TGCAAAACTT GACGCCGCGC ACGCCTGCGT ACGGCTTGAA CGATAGTCCG
841 ATTGGTTTGC TCGCGTGGAT TGGCGAGAAG ATGATTCCGG GCATTGACAA GGCTGTCAAG
901 CATCCGAACG CAACCCTCAA TCGCGAAGCT CTTTTCACGA CACTCTCGAT CTACTGGTTC
961 ACGGGCTCGA TTGGCTCCTC CTTCCTGCCA TACGCTCTCA ACCCGCACTT CTCTACCTTC
1021 CTCGTCTCGC CGCGGCACCA ACTGCCGAAC TTTGCTCTGT CCAACTTTCC CGACGAGCTG
1081 TTCACGCCCG AAGAACGCGA TGCTCGCCGA ACCGGAAACT TGCGGTGGTA CAAGGATGCA
1141 GAGGATGGAG GGCACTTCGC GGCGCTGGAG AAGCCCGAGG TCTTCGCCGA GCACGTAAGG
1201 GAGGCGATGG GGGTCTTGCT GTCGAACCAG GCCTGA

**FIG 33**

SEQUENCE ID NO 13

***Debaromyces hansenii NCYC 3167* (#113)**

Nucleotide sequence

ORIGIN

```
1       ATGATGCAAG GTCAATACAA GATTCAACTT CGCAACGGAG TAAGAATATT TTCTACCTTC
61      AGCAATTATG ATAACCACGA TGTATTTTGT AGTGGTGGTG AGCGTAAATG GACTCGAGTT
121     ATATTCCTCC TCCATGGGTT TCCCGATAAT AACAGTTCAT ATAATAGCGT TTGGACAGTG
181     ATATTAGATT CTGTCCCAGA AGATGAAAAG GTATTGTTGC TAGCGCCATC AATGAGAGGT
241     TACGAACCGT CTAGTCAAGG TGAACAATGT GATTATAAGA TGTCAGATCT TGCGGGCGAT
301     GTAAGCTCAT GGATTAAAAA TATTTCACCA GATGGTATTC CAGTACACCT TGTTGGTCAT
361     GATTGGGGAG CGATAGTGGC TTTTAAGACC GCTAGTATGT ACCCTGAGAT GATAACATCA
421     ATGGCTTGTT TGGCCATTCC ATATATCACC AATATTCGAT TATGGGAGTT TGCATGGTAT
481     TTTCCAGACC AGATTTATCA TTCCAGCTAT ATGTTCACCA TGCAGTTTGG ATGGCTCTAT
541     AAAAAGAGGC TTTCTGATAC GAGTAGCAAC AGTTATTTGG ACGGATTATG GCGCTGCTGG
601     TCTCCGACAT GGAATTATAC CGTAGATGAA ATCGATAGTG TTAAGAGAAC ACTTCAAGAA
661     CCTGGAGTTA TCGATGCATC AACTGCATAC TACAGATGTA TTGCTAACCC CCTAAACATT
721     TCTAATAGGC GCTGGAACGT AGACTTTAAA AAAGTTCCAA CGTTGTTGCT CGGCGGAGAG
781     CGGGATGGGT GCATGAATGA AAAGTTGTAT CGTTTAGAAG CCAGTAAGCT TGCAAATAAT
841     CCATATGTTA AGGTCAAGAC AATTTCAAAT GTCGGGCATT TCTTGCATCG TGAAGACCCT
901     ACAAAAGTTG GTGAATTGAT AACTGACTGG TTCAGTGAAT ATCCTTAG
```

**FIG 34**

SEQUENCE ID NO 14

*Rhodotorula minuta var. minuta* UOFS Y-0835 **(#712)**

Partial Nucleotide sequence

```
ORIGIN
1     CACTGGCGTT TTCCATCCAT TTCTTTCCAC TACCGCTCGA CTCGCCCCGA ACGCTATTCC
61    CGCTCATTCT TCTGCCACGG CTGGCCAGGC CACTTTCGGT TGAGTTTCTT GAACGTCATA
121   CCGCTCTTAA CGGAGCCGTC GGACCCGTCC GCCCAGGCGT TCCACGTCGT CGCGCCTTCG
181   ATGCCCGGTT ATGCTTGGTA TTCGCCTCCT CCGTCCTCCA AGTGGAGCAT GCCTGATACC
241   GCGAGGGTCT TCGACAAGCT CATGACCGGG CTTGGCTACG AGAAGTACAT GGCGCAGGGC
301   GGAGACTGGG GCAGCATCGC TGCTCGCTGC CTTGGATCGC TGTACAAAGA CCACTGCAAA
361   GCCGTCCACC TCAACTTCCT CCCCGTCTTC CCACCCGTCC CGATGTGGCT TATCAACCCG
421   CACACGCTCC TTGCCTGGGC ACCGCGCTTC CTCGTGCCGG AGAAGCAGGC TGCGCGTATG
481   AAGCGCGGGT TGGCGTACCT TGAGAAGGGC TCCGCCTACT ACGTCATGCA GCAGTTGACG
541   CCTCGCACGC CTGCGTACGG CCTGACCGAC AGTCCCGTCG GCTTGCTGGC CTGGATCGGC
601   GAGAAGTTCG AGCCGACCAT TCAGGAGGCG AGCAAGCAAG CCCAGCCGAC CCTGACTCGC
661   GACGAGCTCT ACTTCACCTG CTCGCTCTAC TGGTTCGCCC GCTCAATCGG CACCTCCTTC
721   CTTCCCTACT CGCTCAACCC GCACTTCACC ACCTTCCTGA CCGACAGCAG GTACCACCTG
781   CCCAACTTTG CCCTGTCCCT CTACCCGGGC GAGATCTACT GCCCGGCAGA ACGGGACGCC
841   AAGCGTACCG GCAACCTCAA GTGgATCAAG GACGCGCCCG AGGGAGGACA CTTTGCTGCG
901   CTCGAGAAGC CCGATGTGTT TGTCGAGCAT CTCAGGGAGG CGTTTGGTGT CATGTGGGAG
961   AAGTAG
```

**FIG 35**

**Alignments (homology at amino acid level)**

| Strain | NCYC 3181 (#1) | NCYC 3183 (#25) | UOFS Y-0471 (#46) | NCYC 3179 (#692) | NCYC 3158 (Car 54) | NCYC 3167 (#113 |
|---|---|---|---|---|---|---|
| **NCYC 3181 (#1)** | | 68.37 | 98.04 | 63.11 | 95.84 | 12.17 |
| **NCYC 3183 (#25)** | 68.37 | | 67.64 | 74.51 | 67.64 | 12.38 |
| **UOFS Y-0471 (#46)** | 98.04 | 67.64 | | 62.62 | 96.33 | 12.65 |
| **NCYC 3179 (#692)** | 63.11 | 74.51 | 62.62 | | 62.14 | 13.43 |
| **NCYC 3158 (Car 054)** | 95.84 | 67.64 | 96.33 | 62.14 | | 12.17 |
| **NCYC 3167 (#113)** | 12.17 | 12.38 | 12.65 | 13.43 | 12.17 | |

*Alignments done in DNAMAN Version 5.2.9 (Lynnon Biosoft) – multiple sequence alignment mode (Fast alignment/Quick alignment settings)*

**FIG 36**

**Alignments (homology at nucleic acid level)**

| Strain | NCYC 3181 (#1) | NCYC 3183 (#25) | UOFS Y-0471 (#46) | NCYC 3179 (#692) | NCYC 3158 (Car 54) | NCYC 3167 (#113 |
|---|---|---|---|---|---|---|
| NCYC 3181 (#1) | | 69.58 | 96.18 | 67.88 | 92.36 | 21.59 |
| NCYC 3183 (#25) | 69.58 | | 68.69 | 72.32 | 67.72 | 23.37 |
| UOFS Y-0471 (#46) | 96.18 | 68.69 | | 66.34 | 92.60 | 23.11 |
| NCYC 3179 (#692) | 67.88 | 72.32 | 66.34 | | 66.10 | 22.34 |
| NCYC 3158 (Car 54) | 92.36 | 67.72 | 92.60 | 66.10 | | 21.14 |
| NCYC 3167 (#113) | 21.59 | 23.37 | 23.11 | 22.34 | 21.14 | |

*Alignments done in DNAMAN Version 5.2.9 (Lynnon Biosoft) – multiple sequence alignment mode (Fast alignment/Quick alignment settings)*

**FIG 37**

```
#1        - - - - M A T H T F A S P P T R F T V D I P Q S E L D E L D F R L D K T R W P A T E I V P E D G A D  46
#25       - - - - M S E H S F E A P P Q P F T V D F A P H I E D L H R - R L D N A R W P T Q E I V P V D V S E  45
#46       - - M K M A T H T F A S P P T R F T V D I P Q S E L D E L H S R L D K T R W P A T E I V P E D G T D  48
#692      - - - - M A A H S F T A P P A P Y N I D F A P Q V N D L H R - R L D A A R W P E H D V V P D D V D H  45
Car054    - - - - M A T H T F A S P P T R F T V D I P Q S E V D Q L H S R L D K T R W P G T E I V P E D G A D  46
#23       - - - - - - M P A R S L T L R P F S P S F T A P E L D G L A R S L E S S R L P A E T Y A S R Q A K Y  44
PolH      - - - - - - - - - - - - - - - - - - - - - - - - - - - - - - - - - - - - - - - - - - - - - - - - - -  0
#777      M S Y S D L P H K P T I P V E P F K L S V P H E D L N G L L T L L K S T R I A K E S Y E N V S A H E  50
Homology          # #   *   #     * #   # * # # #   #     # # *   #     # * #   # * #       # # # #   #

#1        D P T - - A F G L G A G P T L P L M K E - L A K G W R E F D W K K A Q D H L N T F E H Y T V E I E D  93
#25       T E H H N A F G L G M G P Q L N L M K E - L A N G W R A F D Q S A L Q D H L N S F N N W K V E I E G  94
#46       D P T - - A F G L G A G P T L P L M K E - L A K G W R E F D W K K A Q D H L N T F E H Y M V E I E D  95
#692      G E - H G A F G L G A G P S L A L M K E - L A Q E W R G Q D Q K Q L Q D H L N S Y K N Y R V E I E G  93
Car054    D P T - - A F G L G A G P T L P L M K E - L A K G W R D F D W K K A Q D H L N T F E H Y T V E I E D  93
#23       - - - - - - - - - - - G I K H A W M K N A L Q R W K D G F D W K K H E Q D I N E V D H Y M V Q V Q S  83
PolH      - - - - - - - - - - - - - - - - - M T D S L S L G Y Y Q L F H K Y A V L G D K R W H Y L D I P P T S  33
#777      N K F - - - - - - - - G I T R K W L V N M K D E W I K Q D W R K Q E E R I N S L P A F K A K V K N S  92
Homology            # # # # # # * # # #   # *   #   * #   # # #   # * # * # # # # # # *   #   # #   * # # #

                                                            H G X P
#1        L S I H F L H - - - - H R S T R P N A V P L I L C H G W P G H F G E F L N V I P L L T E P S D P S A  139
#25       L S I H F L H - - - - H R S T R A G A L P L I L C H G W P G G Y H E F L H V V Q L L T E P E G A D A  140
#46       L S I H F L H - - - - H R S T R P N A V P L I L C H G W P G H F G E F L N V I P L L T E P S D P S A  141
#692      L N I H F L H - - - - Y P S S R A D A F P L I L C H G W P G G Y H E F L H V L E R L T E P E D Q G S  139
Car054    L S I H F L H - - - - H R S T R P N A V P L I L C H G W P G H F G E F L H V I P L L T E P S D P S A  139
#23       D G I Q H D L H V I Y H E S K D P N A I P L L L L H G W P G S A F E F I E A I K I L R K S T S P - -  131
PolH      P S P A L L G - - - - S G K T - - - - - - L M L C H G F P D S W Y G W R K Q I P I L R N L G Y R L L  73
#777      D G S V F S I H F T A L F S K K K V A I P I I L S H G W P G S F Y E F V P M M E M V K K K Y S P - -  140
Homology  # # * # * * #         # # * # # # # *   * * * L * H G * P *   #   * * #   # # # # * # # #   # #   #

#1        Q A - - - F H V V A P S M P G Y A W S S P P P S S K W - S M P D T - - - - - - - - - - - - - - - - -  168
#25       Q A - - - F H L V V P S M P G Y A F S S P P P T A K W - G M E D T - - - - - - - - - - - - - - - - -  169
#46       Q A - - - F H V V A P S M P G Y A W S L P P P S S K W - N M P D T - - - - - - - - - - - - - - - - -  170
#692      R A - - - F H V V V P S M P G Y A F S S P P K T A K W - G M E D T - - - - - - - - - - - - - - - - -  168
Car054    Q A - - - F H V V A P S M P G Y A W S S P P P T S K W - N M P D T - - - - - - - - - - - - - - - - -  168
#23       - A - - - F H L I A P M E P G Y G W S T P P P L D R G F N M N D C - - - - - - - - - - - - - - - - -  160
PolH      V P - - - S Q M G Y T R S E A P L Y P T P E P N E K G - E Y P E L G L E D G T D A L K D L Y C Y T G  119
#777      - E D L P F H L I V P S L P G W L F S T P P P N D R E F N V T D V - - - - - - - - - - - - - - - - -  172
Homology  # *       * * # # # * * # * * * # # * # P * *     * #   # * # * #

                                                      s x s N x s s
#1        - - - A R V F D K L M T G L G Y E K - Y M A Q G G D W G S I A A R C L G S L H K D H C K A V H L N F  214
#25       - - - A R V F D K L M T G L G Y N K - Y V A Q G G D W G S I T A R C L G A L H K D H C I A V H L N F  215
#46       - - - A R V F D K L M T G L G Y E K - Y M A Q G G D W G S I A A R C L G S L H K D H C K A V H L N F  216
#692      - - - A R V F D K L M T G L G Y A K - Y A A Q G G D W G S I T A R C L G S L H K E N C V A V H L N F  214
Car054    - - - A R V S D K L M N G L G Y E K - Y M A Q G G D W G S I A A R C L G A L H K D H C K A V H L N F  214
#23       - - - T A L M N D L M V G L G Y G D G Y A A Q G G D I G S G L A R - L L A V N Y D A C K C I N I N Y  206
PolH      K F Y A Q C M D E L L T Q L G I P T - V T I I G H D Y G A Y L G P K L Y L Y F P H R V E A I A T S C  168
#777      - - - G Y L F N G L M E G L G F G D G Y V A Q G G D I G S Y V T N - E L G A K Y P A C K I I H V N Y  218
Homology        * # # # * # L * # * L G *   #   *   * * G * D # G * #   * * # * #   # # # # # * # * # * # * #

#1        L P V F P P V P M W L I N P H T L L A W A P R F L V P E K Q A A R M K R G L A Y L E K G S A Y Y V M  264
#25       C P V P P P F P F D Q F N P R T L L N W M P R F V I S D Q Q R A K L E R G L A Y L E K G S A Y Y V M  265
#46       L P V F P P V P M W L I N P H T L L A W A P R F L V P E K Q A A R M K R G L A Y L E K G S A Y Y V M  266
#692      C P V P P P F P L N M F N P R T L L D W M P R F V L P D Q R R A K I E R G V A Y I E R G S A Y Y A M  264
Car054    L P V F P P V P M W L I N P H T L L A W A P R F L V P E K Q A A R M K R G L A Y L E K G S A Y Y V M  264
#23       M P A V A P P E D A P E - - - - - - - - - - R H Q I K P H E E D A L R R A D E F Q K T G R G Y A N M  246
PolH      W - - - - - - - - - - - - - H Y V P A L K K F F A I P D F I K L A P S L S Y Q A Y L I G D A S K D F  205
#777      S N P P P R P L P S P G - - - - - - - - - - S P G Q E A S P P S A E D L L E L L Q K F - - G Y A L E  256
Homology    * #   * *   #         # # # # # # # #   # * *     #     #   #       * # # # # # # # * # * * # # *
```

```
#1        Q Q L T P R T P A Y G L T D S P V G L L A W I G E K F E P T I Q E A S K Q A Q P T L T R D E L Y F T   314
#25       Q Q L T P R T P A Y A L N D S P I G L L A W I G E K M I P G I N E A S A Q P N P T L N R D A L Y T T   315
#46       Q Q L T P R T P A Y G L T D S P V G L L A W I G E K F E P T I Q E A S K Q A Q P T L T R D E L Y F T   316
#692      Q N L T P R T P A Y G L N D S P I G L L A W I G E K M I P G I D K A V K H P N A T L N R E A L F T T   314
Car054    Q Q L T P R T S A Y G L T D S P V G L L A W I G E K F E P T I Q E A S K Q A Q P T L T R D E L Y F T   314
#23       H A T R P G T V G I V V G S S P V A L L A W I A E K Y L - - - - - A W T D E D P P L D T I L A I C T   291
PolH      A T R E G S E P F L R Q V F G G E G W G G S D S S V H M T E P E F Q N Y M T E F A T G G R F L S Y M   255
#777      H S T R P A T V G L V V G S N P L S L L A W V G E K F L - - - - - E W T D E S P S E E T I L T M T S   301
Homology  # # # # * # * # # # # #   # * * # * * * * * * * * * #   #   #   # * # # #     * # *   # #   * #   *

#1        C S L Y W F S - - - - - - - - - - - - P L N R H L L P S L L A Q P A L H H L P D - - R Q Q G T T C P   350
#25       L S L Y W F T N S I G T S - - - - F L P Y S L N P H F S T F L T S P R Y R L P N - - F A L S S F P G   359
#46       C S L Y W F T R S I G T S - - - - F L P Y S L N P H F T T F L T D S K Y H L P N - - F A L S L Y P G   360
#692      L S I Y W F T G S I G S S - - - - F L P Y A L N P H F S T F L V S P R H Q L P N - - F A L S N F P D   358
Car054    C S L Y W F T R S I G T S - - - - F L P Y S L N P H F T T F L T D S K Y Y L P N - - F A L S L Y P G   358
#23       I W W I R D S Y P S S I W - - - - A Y A D F L E T G I S A L H N D P K Y K L D K K P F G F S S F K E   337
PolH      V S T Y K A R R L T F E I E K Q D F L D K G R T P E S M Q V D V P Y L H V G A E - - Q D M A F R P P   303
#777      L Y W F T D C F T T S I Y - - - - T Y R Y G L - - G A K R H E S A K Q A S Y Q K C P L G Y S Q F P K   345
Homology    * # * # # #   # # #   #         # # # #   * # # # # # # # #         #   * # #     # # # *   # *

          A                                                                   B
#1        T L P C P S T R A R S T A R Q N G T L S V P A T S S G I K D A P E G G H F A A L E K P D V F V D H L   400
#25       E L F S P - - - T P R D A A R T G V M R W - - - - - - Y K E A D D G G H F A A L E K P D V F S Q H V   400
#46       E I Y C P - - - A E R D A K R T G N L K W - - - - - - I K D A P E G G H F A A L E K P D V F V E H L   401
#692      E L F T P - - - E E R D A R R T G N L R W - - - - - - Y K D A E D G G H F A A L E K P E V F A E H V   399
Car054    E I Y C P - - - A E R D A K R T G N L K W - - - - - - I K D A P E G G H F A A L E K P D V F V D H L   399
#23       E I S A T - - - P E A W A G R N G N L Q F - - - - - - Y R Y H D K G G H F A A L E Q P E A F A Q D M   378
PolH      M I K N L - - - R K Y V K P G R L D E V W - - - - - - I D A S H W L M F E K A D E F N H E L V K W L   344
#777      E I V E I - - - P A E W V K A Q S N M V W - - - - - - S K K H E S G G H F A A L E K P E L L W A D I   386
Homology  * #     #         # # # *   # # * # #   *             # * # #     * * * * * A * E * * # # * #   # #

#1        R E A F G V M W E K - - - - - - -                                                                   410
#25       R E A V K A M L S S - - - - - - -                                                                   410
#46       R E A F G V M W E K - - - - - - -                                                                   411
#692      R E A M G V L L S N Q A - - - - -                                                                   411
Car054    R E A F G V M W E K - - - - - - -                                                                   409
#23       Q D C F G K I W P L S Q E Q K S -                                                                   394
PolH      D K I H G S Q S K L - - - - - - -                                                                   354
#777      E E F V H S Q W E N Y K G N Y - -                                                                   401
Homology  # * # # * # # # #
```

Amino acid alignments of EH proteins, indicating conserved sequence motifs and regions surrounding the catalytic triad. The nucleophile, acid and base of the catalytic triad are indicated by N, A and B, respectively. HGXP represents the region of the oxy-anion hole of the enzyme. sxNxss represents the genetic motif found in α/β-hydrolase fold enzymes. (P = 100% homology; * = identity of 75-100%; # = identity of 50-75%; - = gap).

**FIG 38**

SEQ. ID. NO. 1

***Rhodosporidium toruloides NCYC 3181* (#1)**

Amino acid sequence

```
ORIGIN
1       MATHTFASPP TRFTVDIPQS ELDELDFRLD KTRWPATEIV PEDGADDPTA FGLGAGPTLP
61      LMKELAKGWR EFDWKKAQDH LNTFEHYTVE IEDLSIHFLH HRSTRPNAVP LILCHGWPGH
121     FGEFLNVIPL LTEPSDPSAQ AFHVVAPSMP GYAWSSPPPS SKWSMPDTAR VFDKLMTGLG
181     YEKYMAQGGD WGSIAARCLG SLHKDHCKAV HLNFLPVFPP VPMWLINPHT LLAWAPRFLV
241     PEKQAARMKR GLAYLEKGSA YYVMQQLTPR TPAYGLTDSP VGLLAWIGEK FEPTIQEASK
301     QAQPTLTRDE LYFTCSLYWF TRSIGTSFLP YSLNPHFTTF LTDSSTTCPT LPCPSTRARS
361     TARQNGTLSV PATSSDQGRA GGRTLCCARE ARRVCRPSQG GVRRHVGEV
```

SEQ. ID. NO. 2

***Rhodosporidium toruloides UOFS Y-0471* (#46)**

Amino acid sequence

```
ORIGIN
1       MKMATHTFAS PPTRFTVDIP QSELDELHSR LDKTRWPATE IVPEDGTDDP TAFGLGAGPT
61      LPLMKELAKG WREFDWKKAQ DHLNTFEHYM VEIEDLSIHF LHHRSTRPNA VPLILCHGWP
121     GHFGEFLNVI PLLTEPSDPS AQAFHVVAPS MPGYAWSLPP PSSKWNMPDT ARVFDKLMTG
181     LGYEKYMAQG GDWGSIAARC LGSLHKDHCK AVHLNFLPVF PPVPMWLINP HTLLAWAPRF
241     LVPEKQAARM KRGLAYLEKG SAYYVMQQLT PRTPAYGLTD SPVGLLAWIG EKFEPTIQEA
301     SKQAQPTLTR DELYFTCSLY WFTRSIGTSF LPYSLNPHFT TFLTDSKYHL PNFALSLYPG
361     EIYCPAERDA KRTGNLKWIK DAPEGGHFAA LEKPDVFVEH LREAFGVMWE K
```

SEQ. ID. NO. 3

***Rhodotorula araucariae NCYC 3183* (#25)**

Amino acid sequence

```
ORIGIN
1       MSEHSFEAPP QPFTVDFAPH IEDLHRRLDN ARWPTQEIVP VDVSETEHHN AFGLGMGPQL
61      NLMKELANGW RAFDQSALQD HLNSFNNWKV EIEGLSIHFL HHRSTRAGAL PLILCHGWPG
121     GYHEFLHVVQ LLTEPEGADA QAFHLVVPSM PGYAFSSPPP TAKWGMEDTA RVFDKLMTGL
181     GYNKYVAQGG DWGSITARCL GALHKDHCIA VHLNFCPVPP PFPFDQFNPR TLLNWMPRFV
241     ISDQQRAKLE RGLAYLEKGS AYYVMQQLTP RTPAYALNDS PIGLLAWIGE KMIPGINEAS
301     AQPNPTLNRD ALYTTLSLYW FTNSIGTSFL PYSLNPHFST FLTSPRYRLP NFALSSFPGE
361     LFSPTPRDAA RTGVMRWYKE ADDGGHFAAL EKPDVFSQHV REAVKAMLSS
```

SEQ. ID. NO. 4

***Cryptococcus curvatus NCYC 3158* (Car 054)**

Amino acid sequence

```
ORIGIN
1     MATHTFASPP TRFTVDIPQS EVDQLHSRLD KTRWPGTEIV PEDGADDPTA FGLGAGPTLP
61    LMKELAKGWR DFDWKKAQDH LNTFEHYTVE IEDLSIHFLH HRSTRPNAVP LILCHGWPGH
121   FGEFLHVIPL LTEPSDPSAQ AFHVVAPSMP GYAWSSPPPT SKWNMPDTAR VSDKLMNGLG
181   YEKYMAQGGD WGSIAARCLG ALHKDHCKAV HLNFLPVFPP VPMWLINPHT LLAWAPRFLV
241   PEKQAARMKR GLAYLEKGSA YYVMQQLTPR TSAYGLTDSP VGLLAWIGEK FEPTIQEASK
301   QAQPTLTRDE LYFTCSLYWF TRSIGTSFLP YSLNPHFTTF LTDSKYYLPN FALSLYPGEI
361   YCPAERDAKR TGNLKWIKDA PEGGHFAALE KPDVFVDHLR EAFGVMWEK
```

SEQ. ID. NO.5

***Rhodosporidium paludigenum* NCYC 3179 ( #692)**

Amino acid sequence

```
ORIGIN
1     MAAHSFTAPP APYNIDFAPQ VNDLHRRLDA ARWPEHDVVP DDVDHGEHGA FGLGAGPSLA
61    LMKELAQEWR GQDQKQLQDH LNSYKNYRVE IEGLNIHFLH YPSSRADAFP LILCHGWPGG
121   YHEFLHVLER LTEPEDQGSR AFHVVVPSMP GYAFSSPPKT AKWGMEDTAR VFDKLMTGLG
181   YAKYAAQGGD WGSITARCLG SLHKENCVAV HLNFCPVPPP FPLNMFNPRT LLDWMPRFVL
241   PDQRRAKIER GVAYIERGSA YYAMQNLTPR TPAYGLNDSP IGLLAWIGEK MIPGIDKAVK
301   HPNATLNREA LFTTLSIYWF TGSIGSSFLP YALNPHFSTF LVSPRHQLPN FALSNFPDEL
361   FTPEERDARR TGNLRWYKDA EDGGHFAALE KPEVFAEHVR EAMGVLLSNQ A
```

SEQ. ID. NO. 6

***Debaromyces hansenii NCYC 3167* (#113)**

Amino acid sequence

```
ORIGIN
1     MMQGQYKIQL RNGVRIFSTF SNYDNHDVFC SGGERKWTRV IFLLHGFPDN NSSYNSVWTV
61    ILDSVPEDEK VLLLAPSMRG YEPSSQGEQC DYKMSDLAGD VSSWIKNISP DGIPVHLVGH
121   DWGAIVAFKT ASMYPEMITS MACLAIPYIT NIRLWEFAWY FPDQIYHSSY MFTMQFGWLY
181   KKRLSDTSSN SYLDGLWRCW SPTWNYTVDE IDSVKRTLQE PGVIDASTAY YRCIANPLNI
241   SNRRWNVDFK KVPTLLLGGE RDGCMNEKLY RLEASKLANN PYVKVKTISN VGHFLHREDP
301   TKVGELITDW FSEYP*
```

SEQ. ID. NO. 7

*Rhodotorula minuta var. minuta* UOFS Y-0835 **(#712)**

Partial Amino acid sequence

ORIGIN

1 HWRFPSISFH YRSTRPERYS RSFFCHGWPG HFRLSFLNVI PLLTEPSDPS AQAFHVVAPS
61 MPGYAWYSPP PSSKWSMPDT ARVFDKLMTG LGYEKYMAQG GDWGSIAARC LGSLYKDHCK
121 AVHLNFLPVF PPVPMWLINP HTLLAWAPRF LVPEKQAARM KRGLAYLEKG SAYYVMQQLT
181 PRTPAYGLTD SPVGLLAWIG EKFEPTIQEA SKQAQPTLTR DELYFTCSLY WFARSIGTSF
241 LPYSLNPHFT TFLTDSRYHL PNFALSLYPG EIYCPAERDA KRTGNLKWIK DAPEGGHFAA
301 LEKPDVFVEH LREAFGVMWE K

SEQUENCE ID NO 8

***Rhodosporidium toruloides NCYC 3181* (#1)**

Nucleotide sequence

ORIGIN

1 ATGGCGACAC ACACATTCGC TTCGCCTCCC ACGCGCTTCA CCGTCGACAT CCCACAGTCA
61 GAACTCGACG AACTCGACTT CCGACTCGAC AAGACCCGCT GGCCGGCGAC AGAGATCGTT
121 CCAGAGGATG GGGCGGACGA CCCGACGGCG TTTGGGCTCG GAGCAGGGCC GACGCTGCCG
181 CTCATGAAGG AACTGGCGAA GGGTTGGCGC GAGTTCGACT GGAAGAAGGC GCAGGACCAC
241 CTCAACACCT TTGAGCACTA CACGGTCGAA ATCGAGGACC TCTCCATCCA CTTCCTCCAC
301 CACCGCTCGA CTCGCCCGAA TGCTGTTCCG CTCATCCTCT GCCACGGCTG GCCAGGCCAC
361 TTCGGCGAGT TCCTGAACGT CATACCGCTC TTGACGGAGC CGTCGGACCC GTCCGCCCAG
421 GCGTTCCACG TCGTCGCGCC TTCGATGCCC GGTTATGCTT GGTCTTCGCC TCCTCCGTCC
481 TCCAAGTGGA GCATGCCTGA TACCGCGAGG GTCTTCGACA AGCTCATGAC CGGGCTTGGC
541 TACGAGAAGT ACATGGCGCA GGGCGGAGAC TGGGGCAGCA TCGCTGCTCG CTGCCTTGGA
601 TCGCTTCACA AAGACCACTG CAAAGCCGTC CACCTCAACT TCCTCCCCGT CTTCCCACCC
661 GTCCCGATGT GGCTTATCAA CCCGCACACG CTCCTTGCCT GGGCACCGCG CTTCCTCGTG
721 CCGGAGAAGC AGGCTGCGCG TATGAAGCGC GGGTTGGCGT ACCTTGAGAA GGGCTCCGCC
781 TACTACGTCA TGCAGCAGTT GACGCCTCGC ACGCCTGCGT ACGGCCTGAC CGACAGTCCC
841 GTCGGCTTGC TGGCCTGGAT CGGCGAGAAG TTCGAGCCGA CCATTCAGGA GGCGAGCAAG
901 CAAGCCCAGC CGACCCTGAC TCGCGACGAG CTCTACTTCA CCTGCTCGCT CTACTGGTTC
961 ACCCGCTCAA TCGGCACCTC CTTCCTTCCC TACTCGCTCA ACCCGCACTT CACCACCTTC
1021 CTGACCGACA GCAGGTACCA CCTGCCCAAC TTTGCCCTGT CCCTCTACCC GGGCGAGATC
1081 TACTGCCCGG CAGAACGGGA CGCCAAGCGT ACCGGCAACC TCAAGTGGAT CAAGGACGCG
1141 CCCGATGGAG GACACTTTGC TGCGCTCGAG AAGCCCGATG TGTTTGTCGA GCATCTCAGG
1201 GAGGCGTTTG GCGTCATGTG GGAGAAGTAG

SEQUENCE ID NO 9

***Rhodosporidium toruloides UOFS Y-0471* (#46)**

Nucleotide sequence

ORIGIN

1 ATGGCGACAC ACACATTCGC TTCGCCTCCC ACCCGCTTCA CCGTCGACAT CCCACAGTCG
61 GAACTCGACG AACTTCACTC GCGACTCGAC AAGACCCGCT GGCCGGCGAC AGAGATCGTT
121 CCAGAGGATG GGACGGACGA TCCGACGGCG TTTGGGCTCG GAGCAGGGCC GACGCTGCCG
181 CTCATGAAGG AATTGGCGAA GGGTTGGCGC GAGTTCGACT GGAAAAAGGC GCAGGACCAC
241 CTCAACACCT TCGAGCACTA CATGGTCGAA ATTGAGGACC TCTCGATCCA CTTCCTCCAC
301 CATCGCTCGA CTCGCCCGAA CGCTGTTCCC CTCATCCTCT GCCACGGCTG GCCAGGCCAC
361 TTTGGCGAGT TCCTGAACGT TATCCCGCTC TTGACGGAGC CGTCGGACCC CTCCGCTCAG
421 GCGTTCCACG TCGTCGCCCC TTCGATGCCT GGCTATGCTT GGTCTTTGCC TCCTCCGTCC
481 TCCAAGTGGA ACATGCCTGA CACCGCGAGG GTCTTCGACA AGCTCATGAC CGGGCTTGGC
541 TACGAGAAGT ACATGGCGCA GGGCGGAGAC TGGGGAAGCA TCGCCGCTCG CTGCCTTGGA
601 TCGCTGCACA AGGACCATTG CAAAGCCGTC CACCTCAACT TCCTCCCCGT CTTCCCACCC
661 GTCCCGATGT GGCTTATCAA CCCGCACACG CTCCTTGCCT GGGCACCGCG CTTCCTCGTG
721 CCGGAGAAGC AGGCTGCGCG TATGAAGCGC GGGTTGGCGT ACCTTGAGAA GGGCTCCGCC
781 TACTACGTCA TGCAGCAGTT GACGCCTCGC ACGCCTGCGT ACGGCCTGAC CGACAGTCCC
841 GTCGGCTTGC TGGCCTGGAT CGGCGAGAAG TTCGAGCCGA CCATTCAGGA GGCGAGCAAG
901 CAAGCCCAGC CGACCCTGAC TCGCGACGAG CTCTACTTCA CCTGCTCGCT CTACTGGTTC
961 ACCCGCTCAA TCGGCACCTC CTTCCTTCCC TACTCGCTCA ACCCGCACTT CACCACCTTC
1021 CTGACCGACA GCAAGTACCA CCTGCCCAAC TTTGCCCTCT CGCTTTACCC AGGCGAGATC
1081 TACTGCCCCG CCGAGCGGGA CGCCAAGCGC ACCGGCAACC TCAAGTGGAT CAAGGACGCG
1141 CCTGAGGGAG GACACTTTGC TGCGCTCGAA AAGCCGGATG TGTTTGTCGA GCACCTCAGG
1201 GAGGCGTTTG GCGTCATGTG GGAGAAGTAG

SEQUENCE ID NO 10

***Rhodotorula araucariae NCYC 3183* (#25)**

Nucleotide sequence

ORIGIN

1 ATGAGCGAGC ACAGCTTCGA GGCCCCGCCA CAGCCGTTTA CGGTCGACTT TGCTCCCCAC

61 ATCGAGGATC TCCACCGCCG TCTCGACAAT GCGCGCTGGC CGACGCAAGA GATTGTCCCC

121 GTCGACGTGT CCGAGACGGA GCATCACAAC GCGTTCGGAC TCGGGATGGG CCCGCAGCTC

181 AACCTTATGA AGGAGCTCGC CAACGGCTGG CGCGCGTTCG ACCAGTCGGC GCTCCAGGAC

241 CACCTCAACA GCTTCAACAA CTGGAAGGTC GAGATCGAGG GATTGTCGAT CCACTTCCTC

301 CACCATCGCT CGACGCGCGC CGGCGCTCTC CCGCTCATCC TGTGCCATGG CTGGCCCGGC

361 GGGTACCACG AGTTCCTCCA CGTCGTCCAG CTCCTCACCG AACCAGAGGG GGCGGATGCG

421 CAGGCGTTTC ACCTCGTCGT CCCCTCGATG CCCGGGTACG CCTTCTCGTC TCCGCCGCCG

481 ACGGCCAAGT GGGGCATGGA AGACACTGCA AGGGTTTTTG ACAAGCTCAT GACCGGTTTG

541 GGGTACAACA AGTATGTCGC GCAGGGCGGT GACTGGGGGT CCATCACGGC GCGATGCCTC

601 GGCGCGCTGC ACAAGGACCA CTGCATTGCT GTCCACCTCA ACTTCTGCCC CGTCCCGCCG

661 CCGTTCCCAT TCGACCAGTT CAACCCGCGC ACGCTGCTCA ACTGGATGCC GCGCTTCGTG

721 ATCTCGGACC AGCAGCGTGC GAAGCTCGAG CGTGGGCTGG CGTACCTCGA GAAGGGGTCT

781 GCTTACTATG TCATGCAGCA GCTCACACCG CGTACCCCGG CCTACGCTCT CAATGACAGC

841 CCGATTGGCC TGCTCGCCTG GATTGGCGAA AAGATGATCC CAGGCATCAA CGAGGCGAGC

901 GCGCAGCCGA ACCCGACGCT CAATCGCGAT GCGTTGTACA CCACGCTCTC GCTGTACTGG

961 TTCACCAACT CCATCGGCAC CTCTTTCCTC CCCTACTCGC TTAACCCGCA CTTCAGCACG

1021 TTCCTCACCT CGCCCCGCTA TCGCCTGCCG AACTTTGCGC TGTCTTCCTT CCCGGGCGAG

1081 CTGTTCTCGC CGACGCCGCG CGATGCTGCG AGGACGGGCG TGATGCGCTG GTACAAGGAG

1141 GCGGACGATG GCGGGCACTT TGCGGCGCTC GAGAAGCCCG ATGTGTTCAG CCAGCATGTC

1201 AGGGAGGCAG TCAAGGCCAT GCTGTCGTCG TGA

SEQUENCE ID NO 11

***Cryptococcus curvatus NCYC 3158* (Car 054)**

Nucleotide sequence

ORIGIN

| | | | | | | |
|---|---|---|---|---|---|---|
| 1 | ATGGCGACAC | ACACATTCGC | TTCGCCTCCC | ACCCGCTTCA | CCGTCGACAT | TCCGCAGTCG |
| 61 | GAAGTTGACC | AACTTCACTC | GCGACTCGAT | AAGACTCGCT | GGCCAGGGAC | AGAGATCGTT |
| 121 | CCAGAGGATG | GGGCGGACGA | CCCGACGGCG | TTTGGACTCG | GAGCAGGACC | AACGCTGCCG |
| 181 | CTCATGAAGG | AACTGGCGAA | GGGTTGGCGC | GACTTCGACT | GGAAGAAGGC | GCAGGACCAC |
| 241 | CTCAACACCT | TCGAGCACTA | CACGGTGGAG | ATCGAGGACC | TCTCGATCCA | CTTCCTCCAC |
| 301 | CATCGCTCGA | CTCGTCCGAA | TGCTGTTCCC | CTCATCCTCT | GCCACGGCTG | GCCAGGACAC |
| 361 | TTCGGCGAGT | TTCTACACGT | TATACCACTC | TTGACGGAGC | CGTCGGACCC | GTCCGCTCAG |
| 421 | GCGTTTCACG | TCGTCGCGCC | TTCGATGCCT | GGTTATGCTT | GGTCTTCGCC | TCCTCCTACC |
| 481 | TCCAAGTGGA | ATATGCCTGA | CACCGCAAGG | GTGTCCGACA | AGCTTATGAA | CGGACTCGGC |
| 541 | TACGAGAAGT | ACATGGCGCA | GGGCGGAGAC | TGGGGCAGCA | TCGCCGCTCG | CTGCCTCGGA |
| 601 | GCGCTGCACA | AAGATCACTG | CAAAGCCGTC | CACCTCAACT | TCCTCCCCGT | CTTCCCGCCC |
| 661 | GTCCCTATGT | GGCTCATCAA | CCCACACACA | CTCCTCGCAT | GGGCTCCGCG | CTTCCTTGTG |
| 721 | CCGGAGAAGC | AGGCTGCGCG | CATGAAGCGC | GGGTTGGCGT | ACCTCGAAAA | GGGCTCCGCC |
| 781 | TACTACGTCA | TGCAGCAATT | GACGCCTCGC | ACGTCTGCGT | ACGGCCTTAC | TGACAGTCCC |
| 841 | GTCGGCTTGC | TGGCCTGGAT | CGGCGAGAAG | TTTGAGCCGA | CCATTCAGGA | AGCGAGCAAG |
| 901 | CAAGCCCAGC | CGACCCTCAC | TCGCGACGAG | CTCTACTTCA | CCTGCTCCTT | GTACTGGTTC |
| 961 | ACCCGCTCAA | TCGGCACCTC | CTTCCTGCCC | TACTCGCTCA | ACCCGCACTT | CACGACCTTC |
| 1021 | CTGACCGACA | GCAAGTATTA | CCTGCCCAAT | TTTGCCCTCT | CCCTGTACCC | GGGCGAGATC |
| 1081 | TACTGCCCTG | CCGAGCGGGA | CGCCAAGCGC | ACTGGCAACC | TCAAGTGGAT | CAAGGACGCG |
| 1141 | CCTGAGGGAG | GACACTTTGC | GGCGCTCGAG | AAGCCCGACG | TGTTTGTCGA | CCATCTCAGG |
| 1201 | GAGGCATTTG | GCGTTATGTG | GGAGAAGTAG | | | |

SEQUENCE ID NO 12

***Rhodosporidium paludigenum*** NCYC 3179 **( #692)**

Nucleotide sequence

```
ORIGIN
1       ATGGCTGCCC ATTCCTTTAC TGCACCTCCT GCACCCTACA ACATCGACTT TGCGCCCCAG
61      GTAAATGACC TGCACCGCCG TCTCGATGCT GCCCGCTGGC CGGAACACGA CGTGGTGCCC
121     GACGATGTGG ATCACGGAGA GCACGGCGCA TTCGGACTCG GCGCTGGTCC CAGCCTCGCC
181     CTCATGAAGG AGCTCGCGCA GGAATGGAGG GGCCAGGACC AGAAGCAGCT GCAGGACCAC
241     CTCAACTCCT ACAAGAACTA TCGCGTCGAG ATCGAGGGTC TCAACATCCA CTTCCTGCAC
301     TACCCGTCGT CTCGCGCCGA TGCGTTCCCG CTCATCCTGT GCCACGGCTG GCCTGGCGGC
361     TACCACGAGT TCCTGCACGT CCTAGAGCGC CTCACGGAGC CCGAGGATCA GGGGTCGCGG
421     GCCTTCCATG TCGTCGTGCC TTCCATGCCG GGTTACGCCT TCTCCTCGCC GCCCAAGACG
481     GCAAAATGGG GCATGGAGGA CACGGCTCGC GTGTTCGACA AGCTCATGAC GGGGCTAGGT
541     TACGCCAAGT ATGCGGCCCA AGGCGGTGAC TGGGGGTCTA TCACGGCGCG CTGCCTAGGT
601     TCGCTGCACA AGGAGAACTG CGTCGCTGTC CACCTCAACT TCTGCCCGGT TCCCCCGCCG
661     TTCCCGCTCA ACATGTTCAA CCCGCGCACA CTTCTGGACT GGATGCCTCG CTTTGTCCTG
721     CCTGATCAAC GGCGGGCCAA GATTGAGCGC GGCGTGGCCT ATATCGAGCG CGGCTCTGCC
781     TACTACGCCA TGCAAAACTT GACGCCGCGC ACGCCTGCGT ACGGCTTGAA CGATAGTCCG
841     ATTGGTTTGC TCGCGTGGAT TGGCGAGAAG ATGATTCCGG GCATTGACAA GGCTGTCAAG
901     CATCCGAACG CAACCCTCAA TCGCGAAGCT CTTTTCACGA CACTCTCGAT CTACTGGTTC
961     ACGGGCTCGA TTGGCTCCTC CTTCCTGCCA TACGCTCTCA ACCCGCACTT CTCTACCTTC
1021    CTCGTCTCGC CGCGGCACCA ACTGCCGAAC TTTGCTCTGT CCAACTTTCC CGACGAGCTG
1081    TTCACGCCCG AAGAACGCGA TGCTCGCCGA ACCGGAAACT TGCGGTGGTA CAAGGATGCA
1141    GAGGATGGAG GGCACTTCGC GGCGCTGGAG AAGCCCGAGG TCTTCGCCGA GCACGTAAGG
1201    GAGGCGATGG GGGTCTTGCT GTCGAACCAG GCCTGA
```

SEQUENCE ID NO 13

***Debaromyces hansenii NCYC 3167* (#113)**

Nucleotide sequence

```
ORIGIN
1       ATGATGCAAG GTCAATACAA GATTCAACTT CGCAACGGAG TAAGAATATT TTCTACCTTC
61      AGCAATTATG ATAACCACGA TGTATTTTGT AGTGGTGGTG AGCGTAAATG GACTCGAGTT
121     ATATTCCTCC TCCATGGGTT TCCCGATAAT AACAGTTCAT ATAATAGCGT TTGGACAGTG
181     ATATTAGATT CTGTCCCAGA AGATGAAAAG GTATTGTTGC TAGCGCCATC AATGAGAGGT
241     TACGAACCGT CTAGTCAAGG TGAACAATGT GATTATAAGA TGTCAGATCT TGCGGGCGAT
301     GTAAGCTCAT GGATTAAAAA TATTTCACCA GATGGTATTC CAGTACACCT TGTTGGTCAT
361     GATTGGGGAG CGATAGTGGC TTTTAAGACC GCTAGTATGT ACCCTGAGAT GATAACATCA
421     ATGGCTTGTT TGGCCATTCC ATATATCACC AATATTCGAT TATGGGAGTT TGCATGGTAT
481     TTTCCAGACC AGATTTATCA TTCCAGCTAT ATGTTCACCA TGCAGTTTGG ATGGCTCTAT
541     AAAAAGAGGC TTTCTGATAC GAGTAGCAAC AGTTATTTGG ACGGATTATG GCGCTGCTGG
601     TCTCCGACAT GGAATTATAC CGTAGATGAA ATCGATAGTG TTAAGAGAAC ACTTCAAGAA
661     CCTGGAGTTA TCGATGCATC AACTGCATAC TACAGATGTA TTGCTAACCC CCTAAACATT
721     TCTAATAGGC GCTGGAACGT AGACTTTAAA AAAGTTCCAA CGTTGTTGCT CGGCGGAGAG
781     CGGGATGGGT GCATGAATGA AAAGTTGTAT CGTTTAGAAG CCAGTAAGCT TGCAAATAAT
841     CCATATGTTA AGGTCAAGAC AATTTCAAAT GTCGGGCATT TCTTGCATCG TGAAGACCCT
901     ACAAAAGTTG GTGAATTGAT AACTGACTGG TTCAGTGAAT ATCCTTAG
```

SEQUENCE ID NO 14

*Rhodotorula minuta var. minuta* UOFS Y-0835 **(#712)**

Partial Nucleotide sequence

ORIGIN

```
1     CACTGGCGTT TTCCATCCAT TTCTTTCCAC TACCGCTCGA CTCGCCCCGA ACGCTATTCC
61    CGCTCATTCT TCTGCCACGG CTGGCCAGGC CACTTTCGGT TGAGTTTCTT GAACGTCATA
121   CCGCTCTTAA CGGAGCCGTC GGACCCGTCC GCCCAGGCGT TCCACGTCGT CGCGCCTTCG
181   ATGCCCGGTT ATGCTTGGTA TTCGCCTCCT CCGTCCTCCA AGTGGAGCAT GCCTGATACC
241   GCGAGGGTCT TCGACAAGCT CATGACCGGG CTTGGCTACG AGAAGTACAT GGCGCAGGGC
301   GGAGACTGGG GCAGCATCGC TGCTCGCTGC CTTGGATCGC TGTACAAAGA CCACTGCAAA
361   GCCGTCCACC TCAACTTCCT CCCCGTCTTC CCACCCGTCC CGATGTGGCT TATCAACCCG
421   CACACGCTCC TTGCCTGGGC ACCGCGCTTC CTCGTGCCGG AGAAGCAGGC TGCGCGTATG
481   AAGCGCGGGT TGGCGTACCT TGAGAAGGGC TCCGCCTACT ACGTCATGCA GCAGTTGACG
541   CCTCGCACGC CTGCGTACGG CCTGACCGAC AGTCCCGTCG GCTTGCTGGC CTGGATCGGC
601   GAGAAGTTCG AGCCGACCAT TCAGGAGGCG AGCAAGCAAG CCCAGCCGAC CCTGACTCGC
661   GACGAGCTCT ACTTCACCTG CTCGCTCTAC TGGTTCGCCC GCTCAATCGG CACCTCCTTC
721   CTTCCCTACT CGCTCAACCC GCACTTCACC ACCTTCCTGA CCGACAGCAG GTACCACCTG
781   CCCAACTTTG CCCTGTCCCT CTACCCGGGC GAGATCTACT GCCCGGCAGA ACGGGACGCC
841   AAGCGTACCG GCAACCTCAA GTGgATCAAG GACGCGCCCG AGGGAGGACA CTTTGCTGCG
901   CTCGAGAAGC CCGATGTGTT TGTCGAGCAT CTCAGGGAGG CGTTTGGTGT CATGTGGGAG
961   AAGTAG
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BOTES et al.** *Tetrahedron Asymmetry,* 1999, vol. 10, 3327-3336
- **VISSER et al.** *Biocatalysis and Biotransformation,* 2003, vol. 21 (1), 33-40
- **WEIJERS et al.** *Journal of Molecular Catalysis B: Enzymatic,* 1999, vol. 6, 199-124
- **KARLIN ; ALTSCHUL.** *Proc. Natl, Acad. Sci, USA,* 1993, vol. 90, 5873-5877
- **ALTSCHUL et al.** *J. Mol. Biol.,* 1990, vol. 215, 403-410
- **ALTSCHUL et al.** *Nucleic Acids Res.,* 1997, vol. 25, 3389-3402
- Current Protocols in Molecular Biology. John Wiley & Sons, 1991, 6.3.1-6.3.6
- **SAMBROOK et al.** Molecular Cloning: A Laboratory Manual. Cold Spring Harbor Laboratory, 1989
- **AUSUBEL et al.** Current Protocols in Molecular Biology. Green Publishing Associates and Wiley Interscience, 1989
- **MADZAK et al.** *Journal of Biotechnology,* 2004, 109
- **MADZAK, C. ; GAILLARDIN, C. ; BECKERICH, J-M.** Heterologous protein expression and secretion in the non-conventional yeast Yarrowia lipolytica: a review. *Journal of Biotechnology,* 2004, vol. 109, 63-81
- **MISCHITZ, M. ; KROUTIL, W. ; WANDEL, U. ; FABER, K.** *Tetrahedron Asymmetry,* 1995, vol. 6, 1261-1272
- **NICAUD J-M ; MADZAK C ; VAN DEN BROEK P ; GYSLER C ; DUBOC P ; NIEDERBERGER P ; GAILLARDIN C.** Protein expression and secretion in the yeast Yarrowia lipolytica. *FEMS Yeast Research,* 2002, vol. 2, 371-279
- **ORRU, R.V.A. ; MAYER, S. ; KROUTIL, W. ; FABER, K.** Chemoenzymatic deracemization of (±)-2,2-disubstituted oxiranes. *Tetrahedron,* 1998, vol. 54, 859-874
- **PEDRAGOSA-MOREAU, S. ; ARCHELAS, A. ; FURSTOSS, R.** Microbiological Transformations 32. Use of epoxide hydrolase mediated biohydrolysis as a way to enantiopure epoxides and vicinal diols - Application to substituted styrene oxide derivatives. *Tetrahedron,* 1996, vol. 52, 4593-4606
- **STEINREIBER, A. ; OSPRIAN, I. ; MAYER, S.F. ; ORRU, R.V. ; FABER, K.** Enantioselective hydrolysis of functionalized 2,2-disubstituted oxiranes with bacterial epoxide hydrolases. *Eur. J. Org. Chem.,* 2000, vol. 22, 3703-3711
- **XUAN J-W ; FOURNIER P ; GAILLARDIN C.** Cloning of the LYS5 gene encoding saccharopine dehydrogenase from the yeast Yarrowia lipolytica by target integration. *Current Genetics,* 1988, vol. 14, 15-21